(19) Europäisches Patentamt
European Patent Office
Office européen des brevets

(11) EP 2 963 022 A1

(12) EUROPEAN PATENT APPLICATION
published in accordance with Art. 153(4) EPC

(43) Date of publication:
**06.01.2016 Bulletin 2016/01**

(21) Application number: **14757773.8**

(22) Date of filing: **19.02.2014**

(51) Int Cl.:
***C07D 235/18*** *(2006.01)* ***A01N 43/50*** *(2006.01)*
***A01N 43/78*** *(2006.01)* ***A01N 43/90*** *(2006.01)*
***A01P 7/02*** *(2006.01)* ***A01P 7/04*** *(2006.01)*
***A61K 31/4184*** *(2006.01)* ***A61K 31/423*** *(2006.01)*
***A61K 31/437*** *(2006.01)* ***A61P 33/00*** *(2006.01)*
***A61P 33/14*** *(2006.01)* ***C07D 263/56*** *(2006.01)*
***C07D 277/66*** *(2006.01)* ***C07D 471/04*** *(2006.01)*
***C07D 498/04*** *(2006.01)* ***C07D 513/04*** *(2006.01)*

(86) International application number:
**PCT/JP2014/054597**

(87) International publication number:
**WO 2014/132972 (04.09.2014 Gazette 2014/36)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(30) Priority: **28.02.2013 JP 2013038437**

(71) Applicant: **Sumitomo Chemical Company, Limited**
**Tokyo 104-8260 (JP)**

(72) Inventors:
- **TANABE, Takamasa**
**Takarazuka-shi**
**Hyogo 665-8555 (JP)**
- **ITO, Mai**
**Takarazuka-shi**
**Hyogo 665-8555 (JP)**
- **SHIMIZU, Chie**
**Tokyo 104-8260 (JP)**
- **NOKURA, Yoshihiko**
**Takarazuka-shi**
**Hyogo 665-8555 (JP)**

(74) Representative: **Vossius & Partner**
**Patentanwälte Rechtsanwälte mbB**
**Siebertstrasse 3**
**81675 München (DE)**

(54) **FUSED HETEROCYCLIC COMPOUND AND PEST CONTROL APPLICATIONS THEREOF**

(57) A fused heterocyclic compound represented by formula (1) :

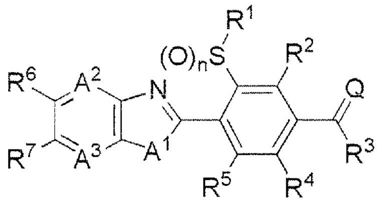

(1)

wherein $A^1$ represents $NR^8$ or the like; $A^2$ and $A^3$ represent N or the like, Q represents O or S; $R^1$ represents a C1 to C6 chain hydrocarbon group or the like; $R^2$, $R^4$ and $R^5$ are the same or different and represent a hydrogen atom or the like; $R^3$ represents group J1, group J2 or group J3 of the following formulae:

EP 2 963 022 A1

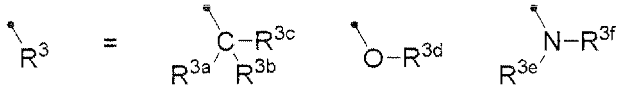

(J1) (J2) (J3)

wherein $R^{3a}$, $R^{3b}$, $R^{3c}$ and $R^{3e}$ are the same or different and represent a C1 to C6 alkyl group optionally having one or more halogen atoms or the like, and $R^{3d}$ and $R^{3f}$ represent a C1 to C6 chain hydrocarbon group or the like; $R^6$, $R^7$ and $R^8$ are the same or different and represent a C1 to C6 chain hydrocarbon group or the like; and n represents 0, 1 or 2, or an N-oxide thereof has an excellent control effect on pests.

## Description

TECHNICAL FIELD

The present invention relates to a certain type of fused heterocyclic compound and a use thereof for pest control.

BACKGROUND ART

It is known in WO2012/086848 that a certain type of fused heterocyclic compound is effective in pest control.

SUMMARY OF THE INVENTION

The present invention provides a compound having an excellent control effect on pests.
The present invention is as described below.

[1] A fused heterocyclic compound represented by formula (1) :

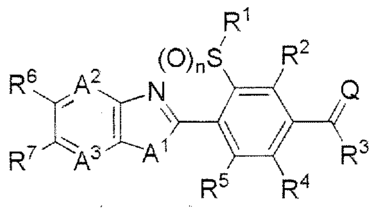 (1)

wherein

$A^1$ represents $NR^8$, S or O;
$A^2$ represents N or $CR^9$;
$A^3$ represents N or $CR^{10}$;
Q represents O or S;
$R^1$ represents a C1 to C6 chain hydrocarbon group optionally having one or more atoms or groups selected from group $\alpha$ or a C3 to C6 cycloalkyl group optionally having one or more halogen atoms or one or more C1 to C3 alkyl groups;
$R^2$, $R^4$ and $R^5$ are the same or different and represent a C1 to C6 chain hydrocarbon group optionally having one or more atoms or groups selected from group $\alpha$, $NR^{11}R^{12}$, $S(O)_mR^{12}$, $OR^{11}$, a cyano group, a nitro group, a halogen atom or a hydrogen atom;
$R^3$ represents a group represented by group J1, group J2 or group J3 of the following formulae:

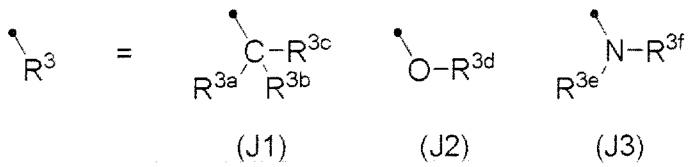

wherein
$R^{3a}$, $R^{3b}$ and $R^{3c}$ are the same or different and represent a C1 to C6 alkyl group optionally having one or more halogen atoms, a halogen atom or a hydrogen atom,
$R^{3d}$ represents a C1 to C6 chain hydrocarbon group optionally having one or more atoms or groups selected from group $\beta$, a C3 to C9 cycloalkyl group optionally having one or more halogen atoms or one or more C1 to C3 alkyl groups, a phenyl group optionally having one or more atoms or groups selected from group $\gamma$, a 5- or 6-membered heterocyclic group optionally having one or more atoms or groups selected from group $\gamma$ or a hydrogen atom, and
$R^{3e}$ represents a C1 to C6 chain hydrocarbon group optionally having one or more halogen atoms or a hydrogen atom and
$R^{3f}$ represents a C1 to C6 chain hydrocarbon group optionally having one or more atoms or groups selected from group $\beta$, a C3 to C9 cycloalkyl group optionally having one or more halogen atoms or one or more C1 to

C3 alkyl groups, a phenyl group optionally having one or more atoms or groups selected from group $\gamma$, a 5- or 6-membered heterocyclic group optionally having one or more atoms or groups selected from group $\gamma$, $NR^{L1}R^{12}$, $OR^{11}$, $C(O)R^{11}$, $CO_2R^{11}$ or a hydrogen atom, or
$R^{3e}$ and $R^{3f}$, together with the nitrogen atom to which they are bound, may form a 5- or 6-membered heterocyclic ring optionally having one or more atoms or groups selected from group $\gamma$;
$R^6$ and $R^7$ are the same or different and represent a C1 to C6 chain hydrocarbon group optionally having one or more atoms or groups selected from group $\alpha$, $OR^{11}$, $S(O)_mR^{11}$, $SF_5$, a halogen atom or a hydrogen atom (wherein $R^6$ and $R^7$ do not represent a hydrogen atom at the same time);
$R^8$ represents a C1 to C6 chain hydrocarbon group optionally having one or more atoms or groups selected from group $\beta$, a C3 to C6 cycloalkyl group optionally having one or more halogen atoms or one or more C1 to C3 alkyl groups, $C(O)R^{11}$, $CO_2R^{11}$ or a hydrogen atom;
$R^9$ and $R^{10}$ are the same or different and represent a C1 to C6 alkyl group optionally having one or more halogen atoms, $NR^{11}R^{12}$, $S(O)_mR^{11}$, $OR^{11}$, $C(O)R^{11}$, $CO_2R^{11}$, a cyano group, a halogen atom or a hydrogen atom;
$R^{11}$ and $R^{12}$ are the same or different and represent a C1 to C6 chain hydrocarbon group optionally having one or more atoms or groups selected from group $\alpha$ or a hydrogen atom (wherein, in $S(O)_mR^{11}$, when m is 1 or 2, $R^{11}$ does not represent a hydrogen atom) ;
n represents 0, 1 or 2; and
m represents 0, 1 or 2;
Group $\alpha$: a group consisting of C1 to C6 alkoxy groups optionally having one or more halogen atoms, C1 to C6 alkylsulfanyl groups optionally having one or more halogen atoms, C1 to C6 alkylsulfinyl groups optionally having one or more halogen atoms, C1 to C6 alkylsulfonyl groups optionally having one or more halogen atoms, C2 to C6 alkylcarbonyl groups optionally having one or more halogen atoms, C2 to C6 alkoxycarbonyl groups optionally having one or more halogen atoms, C3 to C6 cycloalkyl groups optionally having one or more halogen atoms or one or more C1 to C3 alkyl groups, cyano groups, hydroxy groups, and halogen atoms,
Group $\beta$: a group consisting of C1 to C6 alkoxy groups optionally having one or more halogen atoms, C1 to C6 alkylsulfanyl groups optionally having one or more halogen atoms, C1 to C6 alkylsulfinyl groups optionally having one or more halogen atoms, C1 to C6 alkylsulfonyl groups optionally having one or more halogen atoms, C2 to C6 alkylcarbonyl groups optionally having one or more halogen atoms, C2 to C6 alkoxycarbonyl groups optionally having one or more halogen atoms, C3 to C6 cycloalkyl groups optionally having one or more halogen atoms or one or more C1 to C3 alkyl groups, phenyl groups optionally having one or more atoms or groups selected from group $\gamma$, 5- or 6-membered heterocyclic groups optionally having one or more atoms or groups selected from group $\gamma$, cyano groups, hydroxy groups, and halogen atoms,
Group $\gamma$: a group consisting of C1 to C3 alkyl groups optionally having one or more halogen atoms, C1 to C3 alkoxy groups optionally having one or more halogen atoms, C1 to C3 alkylamino groups optionally having one or more halogen atoms, C2 to C6 dialkylamino groups optionally having one or more halogen atoms, C1 to C3 alkylsulfanyl groups optionally having one or more halogen atoms, C1 to C3 alkylsulfinyl groups optionally having one or more halogen atoms, C1 to C3 alkylsulfonyl groups optionally having one or more halogen atoms, C2 to C4 alkylcarbonyl groups optionally having one or more halogen atoms, C2 to C4 alkoxycarbonyl groups optionally having one or more halogen atoms, nitro groups, amino groups, cyano groups, and halogen atoms, or an N-oxide thereof (hereinafter, the fused heterocyclic compound represented by the formula (1) and the N-oxide thereof are referred to as the compound of the present invention) (in the case of the N-oxide, n represents 2, and m represents 2).

[2] The compound according to [1], wherein, in the formula (1),

$R^1$ is a C1 to C6 alkyl group optionally having one or more halogen atoms or one or more cyclopropyl groups (wherein the cyclopropyl group optionally has one or more halogen atoms or one or more C1 to C3 alkyl groups), a C2 to C6 alkenyl group optionally having one or more halogen atoms, a C2 to C6 alkynyl group optionally having one or more halogen atoms or a C3 to C6 cycloalkyl group optionally having one or more halogen atoms or one or more C1 to C3 alkyl groups;
$R^2$, $R^4$ and $R^5$ are the same or different and are a C1 to C3 alkyl group optionally having one or more halogen atoms, a halogen atom or a hydrogen atom;
$R^6$ is a C1 to C6 alkyl group optionally having one or more halogen atoms, $OR^{11a}$, $S(O)_mR^{11a}$ or a halogen atom;
$R^7$ is a C1 to C6 alkyl group optionally having one or more halogen atoms, $OR^{11a}$, $S(O)_mR^{11a}$, a halogen atom or a hydrogen atom;
$R^8$ is a C1 to C6 alkyl group optionally having one or more halogen atoms or one or more cyclopropyl groups (wherein the cyclopropyl group optionally has one or more halogen atoms or one or more C1 to C3 alkyl groups), a C2 to C6 alkenyl group optionally having one or more halogen atoms, a C2 to C6 alkynyl group optionally

having one or more halogen atoms, a C1 to C3 alkyl group having a phenyl group optionally having one or more atoms or groups selected from group γ, a C1 to C3 alkyl group having a 5- or 6-membered heterocyclic group optionally having one or more atoms or groups selected from group γ, a C3 to C6 cycloalkyl group optionally having one or more halogen atoms or one or more C1 to C3 alkyl groups, $C(O)R^{11a}$, $CO_2R^{11a}$ or a hydrogen atom;
$R^9$ and $R^{10}$ are the same or different and are a C1 to C6 alkyl group optionally having one or more halogen atoms, $OR^{11a}$, $S(O)_mR^{11a}$, a halogen atom or a hydrogen atom; and
$R^{11a}$ is a C1 to C6 alkyl group optionally having one or more halogen atoms or a hydrogen atom (wherein, in $S(O)_mR^{11a}$, when m is 1 or 2, $R^{11a}$ does not represent a hydrogen atom).

[3] The compound according to [1], wherein, in the formula (1),

$R^1$ is a C1 to C6 alkyl group optionally having one or more halogen atoms or one or more cyclopropyl groups (wherein the cyclopropyl group optionally has one or more halogen atoms or one or more C1 to C3 alkyl groups);
all of $R^2$, $R^4$ and $R^5$ are a hydrogen atom;
$R^6$ is a C1 to C6 perfluoroalkyl group, $OR^{11b}$, $S(O)_mR^{11b}$ or a halogen atom;
$R^7$ is a C1 to C6 perfluoroalkyl group, $OR^{11b}$, $S(O)_mR^{11b}$, a halogen atom or a hydrogen atom;
$R^8$ is a C1 to C6 alkyl group optionally having one or more halogen atoms, a C2 to C6 alkenyl group optionally having one or more halogen atoms, a C2 to C6 alkynyl group optionally having one or more halogen atoms, a C1 to C3 alkyl group having a thiazolyl group optionally having one or more atoms or groups selected from group γ or a C1 to C3 alkyl group having a pyridyl group optionally having one or more atoms or groups selected from group γ;
$R^9$ is a hydrogen atom;
$R^{10}$ is a C1 to C6 alkyl group optionally having one or more halogen atoms, $OR^{11c}$, $S(O)_mR^{11c}$, a halogen atom or a hydrogen atom;
$R^{11b}$ is a C1 to C6 perfluoroalkyl group; and
$R^{11c}$ is a C1 to C6 alkyl group optionally having one or more halogen atoms.

[4] The compound according to any of [1] to [3], wherein $R^3$ is group J1.
[5] The compound according to any of [1] to [3], wherein $R^3$ is group J2.
[6] The compound according to any of [1] to [3], wherein $R^3$ is group J3.
[7] The compound according to any of [1] to [6], wherein $A^1$ is $NR^8$.
[8] The compound according to any of [1] to [6], wherein $A^1$ is S.
[9] The compound according to any of [1] to [6], wherein $A^1$ is O.
[10] A pest control agent which comprises the compound as defined in any of [1] to [9], and an inert carrier.
[11] A method for controlling pests comprising applying an effective amount of the compound as defined in any of [1] to [9] to a pest or a pest-infested area.

MODE FOR CARRYING OUT THE INVENTION

The groups used in the description of the present specification will be described below with examples.
The halogen atom in this invention refers to a fluorine atom, a chlorine atom, a bromine atom, and an iodine atom.
Examples of the C1 to C6 alkyl group in this invention include a methyl group, an ethyl group, a propyl group, an isopropyl group, a butyl group, an isobutyl group, a sec-butyl group, a tert-butyl group, a pentyl group, a neopentyl group, and a hexyl group.
Examples of the C2 to C6 alkenyl group in this invention include a vinyl group, a 1-propenyl group, a 2-propenyl group, a 1-methylvinyl group, a 2-methyl-1-propenyl group, a 1-butenyl group, a 2-butenyl group, a 3-butenyl group, a 1-pentenyl group, and a 1-hexenyl group.
Examples of the C2 to C6 alkynyl group in this invention include an ethynyl group, a propargyl group, a 2-butynyl group, a 3-butynyl group, a 1-pentynyl group, and a 1-hexynyl group.
The C1 to C6 chain hydrocarbon group optionally having one or more atoms or groups selected from group α in this invention refers to a straight-chain or branched-chain hydrocarbon group having a carbon atom number of 1 to 6, in which hydrogen atoms bound to the carbon atom are optionally substituted by one or more atoms or groups selected from group α, and herein, when the C1 to C6 chain hydrocarbon group has two or more atoms or groups selected from group α, the atoms or groups selected from group α may be the same or different from each other.
Examples of the C1 to C6 chain hydrocarbon group optionally having one or more atoms or groups selected from group α include C1 to C6 alkyl groups optionally having one or more atoms or groups selected from group α such as a methyl group, an ethyl group, a propyl group, an isopropyl group, a butyl group, an isobutyl group, a sec-butyl group, a tert-butyl group, a pentyl group, a neopentyl group, a hexyl group, a methoxymethyl group, an ethoxymethyl group, a

propoxymethyl group, an isopropoxymethyl group, a butoxymethyl group, a sec-butoxymethyl group, a tert-butoxymethyl group, a 2-methoxyethyl group, a 2-ethoxyethyl group, a 2-propoxyethyl group, a 2-isopropoxyethyl group, a 2-butoxyethyl group, a 2-sec-butoxyethyl group, a 2-tert-butoxyethyl group, a trifluoromethyl group, a trichloromethyl group, a 2-fluoroethyl group, a 2,2-difluoroethyl group, a 2,2,2,-trifluoroethyl group, a pentafluoroethyl group, a 2- (methylsulfanyl) ethyl group, a 2- (ethylsulfanyl) ethyl group, a 2-(methylsulfinyl)ethyl group, a 2-(methylsulfonyl)ethyl group, a 2-hydroxyethyl group, a cyclopropylmethyl group, a 1-methylcyclopropylmethyl group and a 2,2-difluorocyclopropylmethyl group;

C2 to C6 alkenyl groups optionally having one or more atoms or groups selected from group α such as a vinyl group, a 1-propenyl group, a 2-propenyl group, a 1-methylvinyl group, a 2-methyl-1-propenyl group, a 1-butenyl group, a 2-butenyl group, a 3-butenyl group, a 1-pentenyl group, a 1-hexenyl group, a 1,1-difluoroallyl group and a pentafluoroallyl group; and

C2 to C6 alkynyl groups optionally having one or more atoms or groups selected from group α such as an ethynyl group, a propargyl group, a 2-butynyl group, a 3-butynyl group, a 1-pentynyl group, a 1-hexynyl group and a 4,4,4-trifluoro-2-butynyl group.

The C1 to C6 chain hydrocarbon group optionally having one or more atoms or groups selected from group β in this invention refers to a straight-chain or branched-chain hydrocarbon group having a carbon atom number of 1 to 6, in which hydrogen atoms bound to the carbon atom are optionally substituted by one or more atoms or groups selected from group β, and herein, when the C1 to C6 chain hydrocarbon group has two or more atoms or groups selected from group β, the atoms or groups selected from group β may be the same or different from each other.

Examples of the C1 to C6 chain hydrocarbon group optionally having one or more atoms or groups selected from group β include C1 to C6 alkyl groups optionally having one or more atoms or groups selected from group β such as a methyl group, an ethyl group, a propyl group, an isopropyl group, a butyl group, an isobutyl group, a sec-butyl group, a tert-butyl group, a pentyl group, a neopentyl group, a hexyl group, a methoxymethyl group, an ethoxymethyl group, a propoxymethyl group, an isopropoxymethyl group, a butoxymethyl group, a sec-butoxymethyl group, a tert-butoxymethyl group, a 2-methoxyethyl group, a 2-ethoxyethyl group, a 2-propoxyethyl group, a 2-isopropoxyethyl group, a 2-butoxyethyl group, a 2-sec-butoxyethyl group, a 2-tert-butoxyethyl group, a trifluoromethyl group, a trichloromethyl group, a 2-fluoroethyl group, a 2,2-difluoroethyl group, a 2,2,2,-trifluoroethyl group, a pentafluoroethyl group, a 2- (methylsulfanyl) ethyl group, a 2- (ethylsulfanyl) ethyl group, a 2-(methylsulfinyl)ethyl group, a 2-(methylsulfonyl)ethyl group, a 2-hydroxyethyl group, a cyclopropylmethyl group, a 1-methylcyclopropylmethyl group, a 2,2-difluorocyclopropylmethyl group, a phenylmethyl group, a 4-chlorophenylmethyl group, a 4-trifluoromethylphenylmethyl group, a tetrahydrofuran-2-ylmethyl group, a tetrahydropyran-2-ylmethyl group, a tetrahydropyran-3-ylmethyl group, a thiazol-5-ylmethyl group, a 2-chlorothiazol-5-ylmethyl group, a pyridin-3-ylmethyl group, a 6-chloropyridin-3-ylmethyl group and a 6-trifluoromethylpyridin-3-ylmethyl group;

C2 to C6 alkenyl groups optionally having one or more atoms or groups selected from group β such as a vinyl group, a 1-propenyl group, a 2-propenyl group, a 1-methylvinyl group, a 2-methyl-1-propenyl group, a 1-butenyl group, a 2-butenyl group, a 3-butenyl group, a 1-pentenyl group, a 1-hexenyl group, a 1,1-difluoroallyl group and a pentafluoroallyl group; and

C2 to C6 alkynyl groups optionally having one or more atoms or groups selected from group β such as an ethynyl group, a propargyl group, a 2-butynyl group, a 3-butynyl group, a 1-pentynyl group, a 1-hexynyl group and a 4,4,4-trifluoro-2-butynyl group.

Examples of the C1 to C6 chain hydrocarbon group optionally having one or more halogen atoms in this invention include C1 to C6 alkyl groups optionally having one or more halogen atoms in this invention such as a methyl group, an ethyl group, a propyl group, an isopropyl group, a butyl group, an isobutyl group, a sec-butyl group, a tert-butyl group, a pentyl group, a neopentyl group, a hexyl group, a fluoromethyl group, a chloromethyl group, a bromomethyl group, an iodomethyl group, a difluoromethyl group, a dichloromethyl group, a trifluoromethyl group, a chlorodifluoromethyl group, a bromodifluoromethyl group, a trichloromethyl group, a 2-fluoroethyl group, a 2-chloroethyl group, a 2-bromoethyl group, a 2,2-difluoroethyl group, a 2,2,2-trifluoroethyl group, a pentafluoroethyl group, a heptafluoropropyl group, and a heptafluoroisopropyl group;

C2 to C6 alkenyl groups optionally having one or more halogen atoms such as a vinyl group, a 1-propenyl group, a 2-propenyl group, a 1-methylvinyl group, a 2-methyl-1-propenyl group, a 1-butenyl group, a 2-butenyl group, a 3-butenyl group, a 1-pentenyl group, a 1-hexenyl group, a 1,1-difluoroallyl group and a pentafluoroallyl group; and

C2 to C6 alkynyl groups optionally having one or more halogen atoms such as an ethynyl group, a propargyl group, a 2-butynyl group, a 3-butynyl group, a 1-pentynyl group, a 1-hexynyl group and a 4,4,4-trifluoro-2-butynyl group.

Examples of the C1 to C6 alkyl group optionally having one or more halogen atoms or one or more cyclopropyl groups (wherein the cyclopropyl group optionally has one or more halogen atoms or one or more C1 to C3 alkyl groups) in this invention include a methyl group, an ethyl group, a propyl group, an isopropyl group, a butyl group, an isobutyl group, a sec-butyl group, a tert-butyl group, a pentyl group, a neopentyl group, a hexyl group, a fluoromethyl group, a chloromethyl group, a bromomethyl group, an iodomethyl group, a difluoromethyl group, a dichloromethyl group, a trifluoromethyl group, a chlorodifluoromethyl group, a bromodifluoromethyl group, a trichloromethyl group, a 2-fluoroethyl

group, a 2-chloroethyl group, a 2-bromoethyl group, a 2,2-difluoroethyl group, a 2,2,2-trifluoroethyl group, a pentafluoroethyl group, a heptafluoropropyl group, a heptafluoroisopropyl group, a cyclopropylmethyl group, a 2-cyclopropylethyl group, and a 1-cyclopropylethyl group.

Examples of the C1 to C6 alkyl group optionally having one or more halogen atoms in this invention include a methyl group, an ethyl group, a propyl group, an isopropyl group, a butyl group, an isobutyl group, a sec-butyl group, a tert-butyl group, a pentyl group, a neopentyl group, a hexyl group, a fluoromethyl group, a chloromethyl group, a bromomethyl group, an iodomethyl group, a difluoromethyl group, a dichloromethyl group, a trifluoromethyl group, a chlorodifluoromethyl group, a bromodifluoromethyl group, a trichloromethyl group, a 2-fluoroethyl group, a 2-chloroethyl group, a 2-bromoethyl group, a 2,2-difluoroethyl group, a 2,2,2-trifluoroethyl group, a pentafluoroethyl group, a heptafluoropropyl group, and a heptafluoroisopropyl group.

Examples of the C1 to C3 alkyl group optionally having one or more halogen atoms in this invention include a methyl group, an ethyl group, a propyl group, an isopropyl group, a fluoromethyl group, a chloromethyl group, a bromomethyl group, an iodomethyl group, a difluoromethyl group, a dichloromethyl group, a trifluoromethyl group, a chlorodifluoromethyl group, a bromodifluoromethyl group, a trichloromethyl group, a 2-fluoroethyl group, a 2-chloroethyl group, a 2-bromoethyl group, a 2,2-difluoroethyl group, a 2,2,2-trifluoroethyl group, a pentafluoroethyl group, a heptafluoropropyl group, and a heptafluoroisopropyl group.

The C1 to C6 perfluoroalkyl group in the compound of the present invention refers to a group in which all hydrogen atoms of the C1 to C6 alkyl group are substituted by a fluorine atom, and examples includes a trifluoromethyl group, a pentafluoroethyl group, a heptafluoropropyl group, and a heptafluoroisopropyl group.

The C1 to C3 alkyl group having a phenyl group optionally having one or more atoms or groups selected from group γ in this invention refers to a C1 to C3 alkyl group having a phenyl group, and herein, hydrogen atoms of the phenyl group are optionally substituted by one or more atoms or groups selected from group γ, and examples include a phenylmethyl group, a 4-chlorophenylmethyl group, and a 4-trifluoromethylphenylmethyl group. Herein, when the phenyl group has two or more atoms or groups selected from group γ, the atoms or groups selected from group γ may be the same or different from each other.

The C1 to C3 alkyl group having a 5- or 6-membered heterocyclic group optionally having one or more atoms or groups selected from group γ in this invention refers to a C1 to C3 alkyl group having a 5- or 6-membered heterocyclic group, and hydrogen atoms of the 5- or 6-membered heterocyclic group are optionally substituted by one or more atoms or groups selected from group γ. Examples include a tetrahydrofuran-2-ylmethyl group, a tetrahydropyran-2-ylmethyl group, a tetrahydropyran-3-ylmethyl group, a thiazol-5-ylmethyl group, a 2-chlorothiazol-5-ylmethyl group, a pyridin-3-ylmethyl group, a 6-chloropyridin-3-ylmethyl group, and a 6-trifluoromethylpyridin-3-ylmethyl group. At that time, when the 5- or 6-membered heterocyclic group has two or more atoms or groups selected from group γ, the atoms or groups selected from group γ may be the same or different from each other.

Examples of the C1 to C3 alkyl group having a thiazolyl group optionally having one or more atoms or groups selected from the group γ in this invention include a (thiazol-5-yl)methyl group, a (2-chlorothiazol-5-yl)methyl group, and a 1-(2-chlorothiazol-5-yl)ethyl group.

Examples of the C1 to C3 alkyl group having a pyridyl group optionally having one or more atoms or groups selected from the group γ in this invention include a (pyridin-5-yl) methyl group, a (2-chloropyridin-5-yl)methyl group, a 1-(2-chloropyridin-5-yl)ethyl group, and a (2-trifluoromethylpyridin-5-yl)methyl group.

Examples of the C2 to C6 alkenyl group optionally having one or more halogen atoms in this invention include a vinyl group, a 1-propenyl group, a 2-propenyl group, a 1-methylvinyl group, a 2-methyl-1-propenyl group, a 1-butenyl group, a 2-butenyl group, a 3-butenyl group, a 1-pentenyl group, a 1-hexenyl group, a 1,1-difluoroallyl group, and a pentafluoroallyl group.

Examples of the C2 to C6 alkynyl group optionally having one or more halogen atoms in this invention include an ethynyl group, a propargyl group, a 2-butynyl group, a 3-butynyl group, a 1-pentynyl group, a 1-hexynyl group, and a 4,4,4-trifluoro-2-butynyl group.

Examples of the C3 to C6 cycloalkyl group in this invention include a cyclopropyl group, a cyclobutyl group, a cyclopentyl group, and a cyclohexyl group.

Examples of the C3 to C6 cycloalkyl groups optionally having one or more halogen atoms or one or more C1 to C3 alkyl groups in this invention include a cyclopropyl group, a 1-methylcyclopropyl group, a 2-methylcyclopropyl group, a 1-fluorocyclopropyl group, a 2,2-difluorocyclopropyl group, a 2, 2-dichlorocyclopropyl group, a 2,2-dibromocyclopropyl group, a cyclobutyl group, a cyclopentyl group, and a cyclohexyl group.

Examples of the C3 to C9 cycloalkyl group in this invention include a cyclopropyl group, a cyclobutyl group, a cyclopentyl group, a cyclohexyl group, a cycloheptyl group, a cyclooctyl group, and a cyclononyl group.

Examples of the C3 to C9 cycloalkyl groups optionally having one or more halogen atoms or one or more C1 to C3 alkyl groups in this invention include a cyclopropyl group, a 1-methylcyclopropyl group, a 2-methylcyclopropyl group, a 1-fluorocyclopropyl group, a 2,2-difluorocyclopropyl group, a 2,2-dichlorocyclopropyl group, a 2,2-dibromocyclopropyl group, a cyclobutyl group, a cyclopentyl group, a cyclohexyl group, a cycloheptyl group, a cyclooctyl group,

and a cyclononyl group.

The phenyl group optionally having one or more atoms or groups selected from group γ in this invention refers to a phenyl group in which hydrogen atoms of the phenyl group are optionally substituted by atoms or groups selected from group γ, and herein, when the phenyl group has two or more atoms or groups selected from group γ, the atoms or groups selected from group γ may be the same or different from each other.

Examples of the phenyl group optionally having one or more atoms or groups selected from group γ include a phenyl group, a 2-fluorophenyl group, a 3-fluorophenyl group, a 4-fluorophenyl group, a 2,3-difluorophenyl group, a 2,4-difluorophenyl group, a 2,5-difluorophenyl group, a 2,6-difluorophenyl group, a 3,4-difluorophenyl group, a 3, 5-difluorophenyl group, a 2, 3, 4, 5, 6-pentafluorophenyl group, a 2-chlorophenyl group, a 3-chlorophenyl group, a 4-chlorophenyl group, a 2-bromophenyl group, a 3-bromophenyl group, a 4-bromophenyl group, a 2-iodophenyl group, a 3-iodophenyl group, a 4-iodophenyl group, a 2-trifluoromethylphenyl group, a 3-trifluoromethylphenyl group, a 4-trifluoromethylphenyl group, a 2-trifluoromethoxyphenyl group, a 3-trifluoromethoxyphenyl group, a 4-trifluoromethoxyphenyl group, a 2-trifluoromethylsulfanylphenyl group, a 3-trifluoromethylsulfanylphenyl group, a 4-trifluoromethylsulfanylphenyl group, a 4-nitrophenyl group, a 4-cyanophenyl group, a 4-methylaminophenyl group, a 4-dimethylaminophenyl group, a 4-methylsulfinylphenyl group, a 4-methylsulfonylphenyl group, a 4-acetylphenyl group, and a 4-methoxycarbonylphenyl group.

The heterocyclic group in this invention refers to a heterocyclic compound residue containing one or more nitrogen atoms, oxygen atoms or sulfur atoms, other than carbon atoms, as ring-constituting atoms, in the ring structure. Examples of the heterocyclic ring of the heterocyclic group include 5-membered non-aromatic heterocyclic rings such as a pyrrolidine ring, a tetrahydrofuran ring and a tetrahydrothiophene ring, 5-membered aromatic heterocyclic rings such as a pyrrole ring, a pyrazole ring, an imidazole ring, a furan ring, a thiophene ring, an oxazole ring and a thiazole ring, 6-membered non-aromatic heterocyclic rings such as a piperidine ring, a tetrahydropyran ring, a tetrahydrothiopyran ring, a piperazine ring and a morpholine ring, and 6-membered aromatic heterocyclic rings such as a pyridine ring, a pyrimidine ring, a pyridazine ring and a pyrazine ring.

In addition, in this invention, the 5-membered heterocyclic group refers to a 5-membered non-aromatic heterocyclic group and a 5-membered aromatic heterocyclic group, and the 6-membered heterocyclic group refers to a 6-membered non-aromatic heterocyclic group and a 6-membered aromatic heterocyclic group.

Examples of the 5- or 6-membered heterocyclic group optionally having one or more atoms or groups selected from group γ include 5- or 6-membered non-aromatic heterocyclic groups optionally having one or more atoms or groups selected from group γ such as a pyrrolidin-1-yl group, a 3,3,4,4-tetrafluoropyrrolidin-1-yl group, a tetrahydrofuran-2-yl group, a piperidin-1-yl group, a morpholin-4-yl group and a thiomorpholin-4-yl group; and 5- or 6-membered aromatic heterocyclic groups optionally having one or more atoms or groups selected from group γ such as a 2-pyrrolyl group, a 2-furyl group, a 3-furyl group, a 5-pyrazolyl group, a 4-pyrazolyl group, a 1-pyrrolyl group, a 1-methyl-2-pyrrolyl group, a 2-methylsulfanyl-1-pyrrolyl group, a 2-methylsulfinyl-1-pyrrolyl group, a 2-methylsulfonyl-1-pyrrolyl group, a 2-methylamino-1-pyrrolyl group, a 2-dimethylamino-1-pyrrolyl group, a 5-bromo-2-furyl group, a 5-nitro-2-furyl group, a 5-cyano-2-furyl group, a 5-methoxy-2-furyl group, a 5-acetyl-2-furyl group, a 5-methoxycarbonyl-2-furyl group, a 2-methyl-3-furyl group, a 2,5-dimethyl-3-furyl group, a 2,4-dimethyl-3-furyl group, a 5-methyl-2-thienyl group, a 3-methyl-2-thienyl group, a 1-methyl-3-trifluoromethyl-5-pyrazolyl group, a 5-chloro-1,3-dimethyl-4-pyrazolyl group, a pyrazol-1-yl group, a 3-chloro-pyrazol-1-yl group, a 3-bromopyrazol-1-yl group, a 4-chloropyrazol-1-yl group, a 4-bromopyrazol-1-yl group, an imidazol-1-yl group, a 1,2,4-triazol-1-yl group, a 3-chloro-1,2,4-triazol-1-yl group, a 1,2,3,4-tetrazol-1-yl group, a 1,2,3,5-tetrazol-1-yl group, a 2-thienyl group, a 3-thienyl group, a 3-trifluoromethyl-1,2,4-triazol-1-yl group, a 4-trifluoromethylpyrazol-1-yl group, a pyrazinyl group, a 4-pyrimidinyl group, a 5-pyrimidinyl group, a 2-pyridyl group, a 3-pyridyl group, a 4-pyridyl group, a 3-fluoro-2-pyridyl group, a 4-fluoro-2-pyridyl group, a 5-fluoro-2-pyridyl group, a 6-fluoro-2-pyridyl group, a 2-pyrimidinyl group, a 3-chloro-5-trifluoromethylpyridin-2-yl group, a 5-trifluoromethylpyridin-2-yl group and a 5-trifluoromethoxypyridin-2-yl group.

The phrase "$R^{3e}$ and $R^{3f}$, together with the nitrogen atom to which they are bound, may form a 5- or 6-membered heterocyclic ring optionally having one or more atoms or groups selected from group γ" in this invention refers to that the group J3 represents a 5- or 6-membered heterocyclic group, and the 5- or 6-membered heterocyclic group optionally has one or more atoms or groups selected from group γ. Examples of the group J3 include following groups.

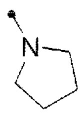 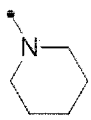 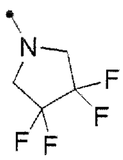 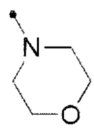 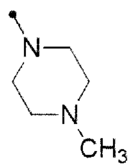

Examples of the C1 to C6 alkoxy groups optionally having one or more halogen atoms in this invention include a methoxy group, a trifluoromethoxy group, an ethoxy group, a 2,2,2-trifluoroethoxy group, a propoxy group, an isopropoxy

group, a butoxy group, an isobutoxy group, a sec-butoxy group, a tert-butoxy group, a pentyloxy group, and a hexyloxy group.

Examples of the C1 to C3 alkoxy groups optionally having one or more halogen atoms in this invention include a methoxy group, a trifluoromethoxy group, an ethoxy group, a 2, 2,2-trifluoroethoxy group, a propoxy group, and an isopropoxy group.

Examples of the C1 to C6 alkylsulfanyl groups optionally having one or more halogen atoms in this invention include a methylsulfanyl group, an ethylsulfanyl group, a propylsulfanyl group, an isopropylsulfanyl group, a butylsulfanyl group, a pentylsulfanyl group, a hexylsulfanyl group, a trifluoromethylsulfanyl group, a 2,2,2-trifluoroethylsulfanyl group, and a pentafluoroethylsulfanyl group.

Examples of the C1 to C3 alkylsulfanyl groups optionally having one or more halogen atoms in this invention include a methylsulfanyl group, a trifluoromethylsulfanyl group, an ethylsulfanyl group, a propylsulfanyl group, and an isopropylsulfanyl group.

Examples of the C1 to C6 alkylsulfinyl groups optionally having one or more halogen atoms in this invention include a methylsulfinyl group, an ethylsulfinyl group, a propylsulfinyl group, an isopropylsulfinyl group, a butylsulfinyl group, a pentylsulfinyl group, a hexylsulfinyl group, a trifluoromethylsulfinyl group, a 2,2,2-trifluoroethylsulfinyl group, and a pentafluoroethylsulfinyl group.

Examples of the C1 to C3 alkylsulfinyl groups optionally having one or more halogen atoms in this invention include a methylsulfinyl group, a trifluoromethylsulfinyl group, an ethylsulfinyl group, a propylsulfinyl group, and an isopropylsulfinyl group.

Examples of the C1 to C6 alkylsulfonyl groups optionally having one or more halogen atoms in this invention include a methylsulfonyl group, an ethylsulfonyl group, a propylsulfonyl group, an isopropylsulfonyl group, a butylsulfonyl group, a pentylsulfonyl group, a hexylsulfonyl group, a trifluoromethylsulfonyl group, a 2,2,2-trifluoroethylsulfonyl group, and a pentafluoroethylsulfonyl group.

Examples of the C1 to C3 alkylsulfonyl groups optionally having one or more halogen atoms in this invention include a methylsulfonyl group, a trifluoromethylsulfonyl group, an ethylsulfonyl group, a propylsulfonyl group, and an isopropylsulfonyl group.

Examples of the C2 to C6 alkylcarbonyl groups optionally having one or more halogen atoms in this invention include an acetyl group, a propionyl group, a butyryl group, a pentanoyl group, a hexanoyl group, and a trifluoroacetyl group.

Examples of the C2 to C4 alkylcarbonyl groups optionally having one or more halogen atoms in this invention include an acetyl group, a propionyl group, a butyryl group, and a trifluoroacetyl group.

Examples of the C2 to C6 alkoxycarbonyl groups optionally having one or more halogen atoms in this invention include a methoxycarbonyl group, an ethoxycarbonyl group, a propoxycarbonyl group, a butoxycarbonyl group, a pentyloxycarbonyl group, a tert-butoxycarbonyl group, and a 2,2,2-trifluoroethoxycarbonyl group.

Examples of the C2 to C4 alkoxycarbonyl groups optionally having one or more halogen atoms in this invention include a methoxycarbonyl group, an ethoxycarbonyl group, a propoxycarbonyl group, and a 2,2,2-trifluoroethoxycarbonyl group.

Examples of the C1 to C3 alkylamino groups optionally having one or more halogen atoms in this invention include a methylamino group, an ethylamino group, a 2,2,2-trifluoroethylamino group, a propylamino group, and an isopropylamino group.

Examples of the C2 to C6 dialkylamino groups optionally having one or more halogen atoms in this invention include a dimethylamino group, a diethylamino group, a bis(2,2,2-trifluoroethyl)amino group, and a dipropylamino group.

The N-oxide in this invention is a compound in which the nitrogen atom constituting the ring on the heterocyclic group is oxidized. Examples of the heterocyclic group that may form an N-oxide include a pyridine ring.

In the compound of the present invention, examples of the N-oxide include the compounds represented by the formula (1A).

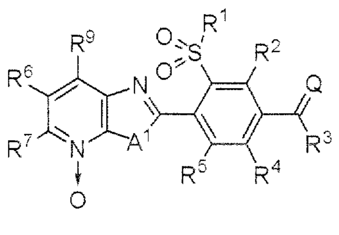

(1A)

Examples of the compound of the present invention include the following compounds.

In the formula (1), compounds wherein $A^1$ is $NR^8$;

In the formula (1), compounds wherein $A^1$ is $NR^8$, and $R^8$ is a methyl group;
In the formula (1), compounds wherein $A^1$ is S;
In the formula (1), compounds wherein $A^1$ is O;
In the formula (1), compounds wherein $A^2$ is N;
In the formula (1), compounds wherein $A^2$ is $CR^9$;
In the formula (1), compounds wherein $A^2$ is CH;
In the formula (1), compounds wherein $A^3$ is N;
In the formula (1), compounds wherein $A^3$ is $CR^{10}$;
In the formula (1), compounds wherein $A^3$ is CH;
In the formula (1), compounds wherein $A^1$ is $NR^8$, $A^2$ is $CR^9$, and $A^3$ is N;
In the formula (1), compounds wherein $A^1$ is $NR^8$, $A^2$ is $CR^9$, and $A^3$ is $CR^{10}$;
In the formula (1), compounds wherein $A^1$ is $NR^8$, $A^2$ is CH, and $A^3$ is N;
In the formula (1), compounds wherein $A^1$ is $NR^8$, $A^2$ is CH, and $A^3$ is $CR^{10}$;
In the formula (1), compounds wherein $A^1$ is $NR^8$, $A^2$ is CH, and $A^3$ is CH;
In the formula (1), compounds wherein $A^1$ is $NR^8$, $R^8$ is a methyl group, $A^2$ is $CR^9$, and $A^3$ is N;
In the formula (1), compounds wherein $A^1$ is $NR^8$, $R^8$ is a methyl group, $A^2$ is $CR^9$, and $A^3$ is $CR^{10}$;
In the formula (1), compounds wherein $A^1$ is $NR^8$, $R^8$ is a methyl group, $A^2$ is CH, and $A^3$ is N;
In the formula (1), compounds wherein $A^1$ is $NR^8$, $R^8$ is a methyl group, $A^2$ is CH, and $A^3$ is $CR^{10}$;
In the formula (1), compounds wherein $A^1$ is $NR^8$, $R^8$ is a methyl group, $A^2$ is CH, and $A^3$ is CH;
In the formula (1), compounds wherein $A^1$ is S, $A^2$ is $CR^9$, and $A^3$ is N;
In the formula (1), compounds wherein $A^1$ is S, $A^2$ is $CR^9$, and $A^3$ is $CR^{10}$;
In the formula (1), compounds wherein $A^1$ is S, $A^2$ is CH, and $A^3$ is N;
In the formula (1), compounds wherein $A^1$ is S, $A^2$ is CH, and $A^3$ is $CR^{10}$;
In the formula (1), compounds wherein $A^1$ is S, $A^2$ is CH, and $A^3$ is CH;
In the formula (1), compounds wherein $A^1$ is O, $A^2$ is $CR^9$, and $A^3$ is N;
In the formula (1), compounds wherein $A^1$ is O, $A^2$ is $CR^9$, and $A^3$ is $CR^{10}$;
In the formula (1), compounds wherein $A^1$ is O, $A^2$ is CH, and $A^3$ is N;
In the formula (1), compounds wherein $A^1$ is O, $A^2$ is CH, and $A^3$ is $CR^{10}$;
In the formula (1), compounds wherein $A^1$ is O, $A^2$ is CH, and $A^3$ is CH;
In the formula (1), compounds wherein $R^1$ is a C1 to C6 chain hydrocarbon group optionally having one or more atoms or groups selected from group $\alpha$;
In the formula (1), compounds wherein $R^1$ is a C1 to C6 alkyl group optionally having one or more atoms or groups selected from group $\alpha$;
In the formula (1), compounds wherein $R^1$ is a C2 to C6 alkenyl group $\beta$ optionally having one or more atoms or groups selected from group $\alpha$;
In the formula (1), compounds wherein $R^1$ is a C2 to C6 alkynyl group optionally having one or more atoms or groups selected from group $\alpha$;
In the formula (1), compounds wherein $R^1$ is a C1 to C6 alkyl group optionally having one or more halogen atoms or one or more cyclopropyl groups (wherein the cyclopropyl group optionally has one or more halogen atoms or one or more C1 to C3 alkyl groups);
In the formula (1), compounds wherein $R^1$ is a C1 to C6 chain hydrocarbon group optionally having one or more halogen atoms; In the formula (1), compounds wherein $R^1$ is a C1 to C6 alkyl group optionally having one or more halogen atoms;
In the formula (1), compounds wherein $R^1$ is a C2 to C6 alkenyl group optionally having one or more halogen atoms;
In the formula (1), compounds wherein $R^1$ is a C2 to C6 alkynyl group optionally having one or more halogen atoms;
In the formula (1), compounds wherein $R^1$ is a C3 to C6 cycloalkyl group optionally having one or more halogen atoms or one or more C1 to C3 alkyl groups;
In the formula (1), compounds wherein $R^1$ is a C1 to C6 alkyl group optionally having one or more halogen atoms or one or more cyclopropyl groups (wherein the cyclopropyl group optionally has one or more halogen atoms or one or more C1 to C3 alkyl groups), a C2 to C6 alkenyl group optionally having one or more halogen atoms, a C2 to C6 alkynyl group optionally having one or more halogen atoms, or a C3 to C6 cycloalkyl group optionally having one or more halogen atoms or one or more C1 to C3 alkyl groups;
In the formula (1), compounds wherein $R^2$ is a C1 to C6 alkyl group;
In the formula (1), compounds wherein $R^1$ is a C1 to C3 alkyl group;
In the formula (1), compounds wherein $R^1$ is a C1 to C6 alkyl group optionally having a cyclopropyl group;
In the formula (1), compounds wherein $R^1$ is a C1 to C6 alkyl group having a cyclopropyl group;
In the formula (1), compounds wherein $R^1$ is a methyl group, an ethyl group, a propyl group, a cyclopropyl group or a cyclopropylmethyl group;
In the formula (1), compounds wherein $R^1$ is a methyl group;

In the formula (1), compounds wherein $R^1$ is an ethyl group;
In the formula (1), compounds wherein $R^1$ is a propyl group;
In the formula (1), compounds wherein $R^1$ is a cyclopropyl group; In the formula (1), compounds wherein $R^1$ is a cyclopropylmethyl group;
In the formula (1), compounds wherein $R^2$, $R^4$ and $R^5$ are the same or different and are a C1 to C6 chain hydrocarbon group optionally having one or more atoms or groups selected from group $\alpha$ or a hydrogen atom;
In the formula (1), compounds wherein $R^2$, $R^4$ and $R^5$ are the same or different and are a halogen atom or a hydrogen atom;
In the formula (1), compounds wherein all of $R^2$, $R^4$ and $R^5$ are a hydrogen atom;
In the formula (1), compounds wherein $R^3$ is group J1;
In the formula (1), compounds wherein $R^3$ is group J2;
In the formula (1), compounds wherein $R^3$ is group J3;
In the formula (1), compounds wherein $R^3$ is group J1 or group J2;
In the formula (1), compounds wherein $R^3$ is group J2 or group J3;
In the formula (1), compounds wherein $R^3$ is group J1 or group J3;
In the formula (1), compounds wherein $R^5$ is group J1, and $R^{3a}$, $R^{3b}$ and. $R^{3c}$ are the same or different and are a C1 to C6 alkyl group optionally having one or more halogen atoms or a hydrogen atom;
In the formula (1), compounds wherein $R^3$ is group J1, and $R^{3a}$, $R^{3b}$ and $R^{3c}$ are the same or different and are a C1 to C3 alkyl group optionally having one or more halogen atoms or a hydrogen atom;
In the formula (1), compounds wherein $R^3$ is group J1, and $R^{3a}$, $R^{3b}$ and $R^{3c}$ are the same or different and are a C1 to C6 alkyl group or a hydrogen atom;
In the formula (1), compounds wherein $R^3$ is group J1, and all of $R^{3a}$, $R^{3b}$ and $R^{3c}$ are a hydrogen atom;
In the formula (1), compounds wherein $R^3$ is group J1, and all of $R^{3a}$, $R^{3b}$ and $R^{3c}$ are a fluorine atom;
In the formula (1), compounds wherein $R^3$ is group J1, $R^{3a}$ is a methyl group, and both $R^{3b}$ and $R^{3c}$ are a hydrogen atom;
In the formula (1), compounds wherein $R^3$ is group J2, and $R^{3d}$ is a C1 to C6 chain hydrocarbon group optionally having one or more atoms or groups selected from group $\beta$, a C3 to C9 cycloalkyl group optionally having one or more halogen atoms or one or more C1 to C3 alkyl groups, a phenyl group optionally having one or more atoms or groups selected from group $\gamma$ or a 5- or 6-membered heterocyclic group optionally having one or more atoms or groups selected from group $\gamma$;
In the formula (1), compounds wherein $R^3$ is group J2, and $R^{3d}$ is a C1 to C6 chain hydrocarbon group optionally having one or more halogen atoms, a C3 to C9 cycloalkyl group optionally having one or more halogen atoms or one or more C1 to C3 alkyl groups, a phenyl group optionally having one or more atoms or groups selected from group $\gamma$ or a 5- or 6-membered heterocyclic group optionally having one or more atoms or groups selected from group $\gamma$;
In the formula (1), compounds wherein $R^3$ is group J2, and $R^{3d}$ is a C1 to C6 chain hydrocarbon group optionally having one or more halogen atoms or a C3 to C9 cycloalkyl group optionally having one or more halogen atoms or one or more C1 to C3 alkyl groups;
In the formula (1), compounds wherein $R^3$ is group J2, and $R^{3d}$ is a C1 to C6 alkyl group optionally having one or more halogen atoms or a C3 to C9 cycloalkyl group optionally having one or more halogen atoms or one or more C1 to C3 alkyl groups;
In the formula (1), compounds wherein $R^3$ is group J2, and $R^{3d}$ is a C1 to C6 alkyl group optionally having one or more halogen atoms;
In the formula (1), compounds wherein $R^3$ is group J2, and $R^{3d}$ is a C3 to C9 cycloalkyl group optionally having one or more halogen atoms or one or more C1 to C3 alkyl groups;
In the formula (1), compounds wherein $R^3$ is group J2, and $R^{3d}$ is a methyl group, an ethyl group, a propyl group, an isopropyl group, a butyl group, an isobutyl group or a cyclopropylmethyl group;
In the formula (1), compounds wherein $R^3$ is group J2, and $R^{3d}$ is a hydrogen atom;
In the formula (1), compounds wherein $R^3$ is group J3, $R^{3e}$ is a C1 to C6 chain hydrocarbon group optionally having one or more halogen atoms or a hydrogen atom, $R^{3f}$ is a C1 to C6 chain hydrocarbon group optionally having one or more atoms or groups selected from group $\beta$, a C3 to C9 cycloalkyl group optionally having one or more halogen atoms or one or more C1 to C3 alkyl groups, a phenyl group optionally having one or more atoms or groups selected from group $\gamma$, a 5- or 6-membered heterocyclic group optionally having one or more atoms or groups selected from group $\gamma$, $NR^{11}R^{12}$, $OR^{11}$, $C(O)R^{11}$, $CO_2R^{11}$ or a hydrogen atom; In the formula (1), compounds wherein $R^3$ is group J3, and $R^{3e}$ and $R^{3f}$, together with the nitrogen atom to which they are bound, form a 5- or 6-membered heterocyclic ring optionally having one or more atoms or groups selected from group $\gamma$;
In the formula (1), compounds wherein $R^3$ is group J3, $R^{3e}$ is a C1 to C6 chain hydrocarbon group optionally having one or more halogen atoms or a hydrogen atom, and $R^{3f}$ is a C1 to C6 chain hydrocarbon group optionally having one or more halogen atoms, a C3 to C9 cycloalkyl group optionally having one or more halogen atoms or one or more C1 to C3 alkyl groups, $NR^{11}R^{12}$, $OR^{11}$, $C(O)R^{11}$, $CO_2R^{11}$ or a hydrogen atom;
In the formula (1), compounds wherein $R^3$ is group J3, $R^{3e}$ is a C1 to C6 chain hydrocarbon group optionally having one

or more halogen atoms or a hydrogen atom, and $R^{3f}$ is a C1 to C6 chain hydrocarbon group optionally having one or more halogen atoms, $NR^{11}R^{12}$, $OR^{11}$, $C(O)R^{11}$, $CO_2R^{11}$ or a hydrogen atom;

In the formula (1), compounds wherein $R^3$ is group J3, and $R^{3e}$ and $R^{3f}$ are the same or different and are a C1 to C6 chain hydrocarbon group optionally having one or more halogen atoms or a hydrogen atom;

In the formula (1), compounds wherein $R^3$ is group J3, and $R^{3e}$ and $R^{3f}$ are the same or different and are a C1 to C6 alkyl group optionally having one or more halogen atoms or a hydrogen atom;

In the formula (1), compounds wherein $R^3$ is group J3, and $R^{3e}$ and $R^{3f}$ are the same or different and are a C1 to C6 alkyl group or a hydrogen atom;

In the formula (1), compounds wherein $R^3$ is group J3, and $R^{3e}$ and $R^{3f}$ are the same or different and are a methyl group, an ethyl group, a propyl group, an isopropyl group or a hydrogen atom;

In the formula (1), compounds wherein $R^3$ is group J3, and both $R^{3e}$ and $R^{3f}$ are a hydrogen atom;

In the formula (1), compounds wherein $R^3$ is a methyl group, an ethyl group, a propyl group, an isopropyl group, a methoxy group, an ethoxy group, a propoxy group, an isopropoxy group, an amino group, a methylamino group, an ethylamino group, a dimethylamino group or a diethylamino group;

In the formula (1), compounds wherein $R^1$ is an ethyl group, all of $R^2$, $R^4$, and $R^5$ are a hydrogen atom, and $R^3$ is group J1;

In the formula (1), compounds wherein $R^1$ is an ethyl group, all of $R^2$, $R^4$, and $R^5$ are a hydrogen atom, and $R^3$ is group J2;

In the formula (1), compounds wherein $R^1$ is an ethyl group, all of $R^2$, $R^4$, and $R^5$ are a hydrogen atom, and $R^3$ is group J3;

In the formula (1), compounds wherein $R^6$ and $R^7$ are the same or different and are a C1 to C6 chain hydrocarbon group optionally having one or more atoms or groups selected from group $\alpha$, $OR^{11}$, $S(O)_mR^{11}$ or a hydrogen atom;

In the formula (1), compounds wherein $R^6$ and $R^7$ are the same or different and are a C1 to C6 perfluoroalkyl group, $OR^{11b}$, $S(O)_mR^{11b}$, a halogen atom or a hydrogen atom;

In the formula (1), compounds wherein $R^6$ and $R^7$ are the same or different and are a trifluoromethyl group, a pentafluoroethyl group, a heptafluoropropyl group, a heptafluoroisopropyl group, a trifluoromethoxy group, a trifluoromethylsulfanyl group, a trifluoromethylsulfinyl group, a trifluoromethylsulfonyl group, a bromine atom, an iodine atom or a hydrogen atom;

In the formula (1), compounds wherein $R^6$ is a C1 to C6 alkyl group optionally having one or more halogen atoms, $OR^{11}$, $S(O)_mR^{11}$ or a halogen atom, and $R^7$ is a C1 to C6 alkyl group optionally having one or more halogen atoms, $OR^{11}$, $S(O)_mR^{11}$, a halogen atom or a hydrogen atom;

In the formula (1), compounds wherein $R^6$ is a C1 to C6 perfluoroalkyl group, $OR^{11b}$, $S(O)_mR^{11b}$ or a halogen atom, and $R^7$ is a C1 to C6 perfluoroalkyl group, $OR^{11b}$, $S(O)R^{11b}$, a halogen atom or a hydrogen atom (wherein $R^{10b}$ represents a C1 to C6 perfluoroalkyl group);

In the formula (1), compounds wherein $R^6$ is a C1 to C6 perfluoroalkyl group, $OR^{11b}$, $S(O)_mR^{11b}$ or a halogen atom, and $R^7$ is a hydrogen atom;

In the formula (1), compounds wherein $R^6$ is a trifluoromethyl group, a pentafluoroethyl group, a heptafluoropropyl group, a heptafluoroisopropyl group, a rrifluoromethoxy group, a trifluoromethylsulfanyl group, a trifluoromethylsulfinyl group, a trifluoromethylsulfonyl group, a bromine atom or an iodine atom, and $R^7$ is a hydrogen atom;

In the formula (1), compounds wherein $R^6$ is a trifluoromethyl group, a pentafluoroethyl group, a trifluoromethylsulfanyl group, a trifluoromethylsulfinyl group or a trifluoromethylsulfonyl group, and $R^7$ is a hydrogen atom;

In the formula (1), compounds wherein $R^6$ is a trifluoromethyl group, and $R^7$ is a hydrogen atom;

In the formula (1), compounds wherein $R^6$ is a pentafluoroethyl group, and $R^7$ is a hydrogen atom;

In the formula (1), compounds wherein $R^6$ is a trifluoromethylsulfanyl group, and $R^7$ is a hydrogen atom;

In the formula (1), compounds wherein $R^6$ is a trifluoromethylsulfinyl group, and $R^7$ is a hydrogen atom;

In the formula (1), compounds wherein $R^6$ is a trifluoromethylsulfonyl group, and $R^7$ is a hydrogen atom;

In the formula (1), compounds wherein $R^8$ is a C1 to C6 alkyl group optionally having one or more atoms or groups selected from group $\beta$, a C3 to C6 cycloalkyl group optionally having one or more halogen atoms or one or more C1 to C3 alkyl groups, $C(O)R^{11}$, $CO_2R^{11}$ or a hydrogen atom;

In the formula (1), compounds wherein $R^8$ is a C1 to C6 alkyl group optionally having one or more halogen atoms and one or more cyclopropyl groups (wherein the cyclopropyl group optionally has one or more halogen atoms or one or more C1 to C3 alkyl groups), a C2 to C6 alkenyl group optionally having one or more halogen atoms, a C2 to C6 alkynyl group optionally having one or more halogen atoms, a C1 to C3 alkyl group having a phenyl group optionally having one or more atoms or groups selected from group $\gamma$, a C1 to C3 alkyl group having a 5- or 6-membered heterocyclic group optionally having one or more atoms or groups selected from group $\gamma$, a C3 to C6 cycloalkyl group optionally having one or more halogen atoms or one or more C1 to C3 alkyl groups, $C(O)R^{11a}$, $CO_2R^{11a}$ or a hydrogen atom; In the formula (1), compounds wherein $R^6$ is a C1 to C6 alkyl group optionally having one or more halogen atoms, a C2 to C6 alkenyl group optionally having one or more halogen atoms, a C2 to C6 alkynyl group optionally having one or more halogen atoms, a C1 to C3 alkyl group having a thiazolyl group optionally having one or more atoms or groups selected from group $\gamma$ or a C1 to C3 alkyl group having a pyridyl group optionally having one or more atoms or groups selected from group $\gamma$;

In the formula (1), compounds wherein $R^8$ is a C1 to C6 alkyl group optionally having one or more atoms or groups

selected from group β or a hydrogen atom;
In the formula (1), compounds wherein $R^8$ is a C1 to C6 alkyl group optionally having one or more atoms or groups selected from group β, $C(O)R^{11}$, $CO_2R^{11}$ or a hydrogen atom;
In the formula (1), compounds wherein $R^8$ is a C1 to C6 alkyl group optionally having one or more atoms or groups selected from group β;
In the formula (1), compounds wherein $R^8$ is a methyl group, an ethyl group, a propyl group, an isopropyl group, a cyclopropyl group, or a hydrogen atom;
In the formula (1), compounds wherein $R^8$ is a methyl group or a hydrogen atom;
In the formula (1), compounds wherein $R^8$ is a methyl group;
In the formula (1), compounds wherein $R^8$ is a hydrogen atom;
In the formula (1), compounds wherein $R^9$ and $R^{10}$ are the same or different and are a C1 to C6 alkyl group optionally having one or more halogen atoms, a halogen atom or a hydrogen atom; In the formula (1), compounds wherein $R^9$ and $R^{10}$ are the same or different and are $NR^{11}R^{12}$, $S(O)_mR^{11}$, $OR^{11}$, $C(O)R^{11}$, $CO_2R^{11}$, a cyano group, a halogen atom or a hydrogen atom;
In the formula (1), compounds wherein $R^9$ is a hydrogen atom, $R^{10}$ is a C1 to C6 alkyl group optionally having one or more halogen atoms, $OR^{11c}$, $S(O)_mR^{11c}$, a halogen atom or a hydrogen atom;
In the formula (1), compounds wherein $R^9$ and $R^{10}$ are the same or different and are a C1 to C6 alkyl group optionally having one or more halogen atoms, $OR^{11a}$, $S(O)_mR^{11a}$, a halogen atom or a hydrogen atom;
In the formula (1), compounds wherein $R^9$ and $R^{10}$ are the same or different and are a halogen atom or a hydrogen atom;
In the formula (1), compounds wherein $R^9$ is a hydrogen atom;
In the formula (1), compounds wherein $R^{10}$ is a hydrogen atom;
In the formula (1), compounds wherein $R^{10}$ is a halogen atom;
In the formula (1), compounds wherein $R^9$ is a hydrogen atom, and $R^{10}$ is a halogen atom or a hydrogen atom;
In the formula (1), compounds wherein both $R^9$ and $R^{10}$ are a hydrogen atom;
In the formula (1), compounds wherein Q is 0;
In the formula (1), compounds wherein Q is S;
In the formula (1), compounds wherein n is 0;
In the formula (1), compounds wherein n is 1;
In the formula (1), compounds wherein n is 2;
In the formula (1), compounds wherein

$R^1$ is a C1 to C6 alkyl group optionally having one or more halogen atoms or one or more cyclopropyl groups (wherein the cyclopropyl group optionally has one or more halogen atoms or one or more C1 to C3 alkyl groups), a C2 to C6 alkenyl group optionally having one or more halogen atoms, a C2 to C6 alkynyl group optionally having one or more halogen atoms or a C3 to C6 cycloalkyl group optionally having one or more halogen atoms or one or more C1 to C3 alkyl groups,
$R^2$, $R^4$ and $R^5$ are the same or different and are a C1 to C3 alkyl group optionally having one or more halogen atoms, a halogen atom or a hydrogen atom,
$R^6$ is a C1 to C6 alkyl group optionally having one or more halogen atoms, $OR^{11a}$, $S(O)_mR^{11a}$ or a halogen atom,
$R^7$ is a C1 to C6 alkyl group optionally having one or more halogen atoms, $OR^{11a}$, $S(O)_mR^{11a}$, a halogen atom or a hydrogen atom,
$R^8$ is a C1 to C6 alkyl group optionally having one or more halogen atoms or one or more cyclopropyl groups (wherein the cyclopropyl group optionally has one or more halogen atoms or one or more C1 to C3 alkyl groups), a C2 to C6 alkenyl group optionally having one or more halogen atoms, a C2 to C6 alkynyl group optionally having one or more halogen atoms, a C1 to C3 alkyl group having a phenyl group optionally having one or more atoms or groups selected from group γ, a C1 to C3 alkyl group having a 5- or 6-membered heterocyclic group optionally having one or more atoms or groups selected from group γ, a C3 to C6 cycloalkyl group optionally having one or more halogen atoms or one or more C1 to C3 alkyl groups, $C(O)R^{11a}$, $CO_2R^{11a}$ or a hydrogen atom,
$R^9$ and $R^{10}$ are the same or different and are a C1 to C6 alkyl group optionally having one or more halogen atoms, $OR^{11a}$, $S(O)_mR^{11a}$, a halogen atom or a hydrogen atom, and
$R^{11a}$ is a C1 to C6 alkyl group optionally having one or more halogen atoms or a hydrogen atom (wherein, in $S(O)_mR^{11a}$, when m is 1 or 2, $R^{11a}$ does not represent a hydrogen atom); In the formula (1), compounds wherein
$R^1$ is a C1 to C6 alkyl group optionally having one or more halogen atoms or one or more cyclopropyl groups (wherein the cyclopropyl group optionally has one or more halogen atoms or one or more C1 to C3 alkyl groups),
all of $R^2$, $R^4$ and $R^5$ are a hydrogen atom,
$R^6$ is a C1 to C6 perfluoroalkyl group, $OR^{11b}$, $S(O)_mR^{11b}$ or a halogen atom,
$R^7$ is a C1 to C6 perfluoroalkyl group, $OR^{11b}$, $S(O)_mR^{11b}$, a halogen atom or a hydrogen atom,
$R^8$ is a C1 to C6 alkyl group optionally having one or more halogen atoms, a C2 to C6 alkenyl group optionally

having one or more halogen atoms, a C2 to C6 alkynyl group optionally having one or more halogen atoms, a C1 to C3 alkyl group having a thiazolyl group optionally having one or more atoms or groups selected from group γ or a C1 to C3 alkyl group having a pyridyl group optionally having one or more atoms or groups selected from group γ,
$R^9$ is a hydrogen atom,
$R^{10}$ is a C1 to C6 alkyl group optionally having one or more halogen atoms, $OR^{11c}$, $S(O)_mR^{11c}$, a halogen atom or a hydrogen atom,
$R^{11b}$ is a C1 to C6 perfluoroalkyl group, and
$R^{11c}$ is a C1 to C6 alkyl group optionally having one or more halogen atoms;

In the formula (1), compounds wherein

$A^1$ is $NR^8$, $R^8$ is a C1 to C6 chain hydrocarbon group optionally having one or more atoms or groups selected from group β, $A^2$ is CH, $A^3$ is N, and Q is O,
$R^1$ is a C1 to C6 chain hydrocarbon group optionally having one or more atoms or groups selected from group α,
$R^2$, $R^4$ and $R^5$ are a hydrogen atom,
$R^{3a}$, $R^{3b}$ and $R^{3c}$ are the same or different and are a C1 to C6 alkyl group optionally having one or more halogen atoms,
$R^{3d}$ is a C1 to C6 chain hydrocarbon group optionally having one or more atoms or groups selected from group β, a C3 to C9 cycloalkyl group optionally having one or more halogen atoms or one or more C1 to C3 alkyl groups, a 6-membered heterocyclic group optionally having one or more atoms or groups selected from group γ or a hydrogen atom,
$R^{3e}$ is a C1 to C6 chain hydrocarbon group optionally having one or more halogen atoms or a hydrogen atom and $R^{3f}$ is a C1 to C6 chain hydrocarbon group optionally having one or more atoms or groups selected from group β, a C3 to C9 cycloalkyl group optionally having one or more halogen atoms or one or more C1 to C3 alkyl groups, a phenyl group optionally having one or more atoms or groups selected from group γ, a 5- or 6-membered heterocyclic group optionally having one or more atoms or groups selected from group γ, $NR^{11}R^{12}$, $OR^{11}$ or a hydrogen atom, or $R^{3e}$ and $R^{3f}$, together with the nitrogen atom to which they are bound, may form a 5- or 6-membered heterocyclic ring optionally having one or more atoms or groups selected from group γ,
$R^6$ and $R^7$ are the same or different and are a C1 to C6 chain hydrocarbon group optionally having one or more atoms or groups selected from group α or a hydrogen atom (wherein $R^6$ and $R^7$ do not represent a hydrogen atom at the same time), and
$R^{11}$ and $R^{12}$ are the same or different and are a C1 to C6 chain hydrocarbon group optionally having one or more atoms or groups selected from group α;

In the formula (1), compounds wherein

$A^1$ is $NR^8$, $R^8$ is a C1 to C6 chain hydrocarbon group optionally having one or more atoms or groups selected from group β, $A^2$ is CH, $A^3$ is N, and Q is O,
$R^1$ is a C1 to C6 chain hydrocarbon group optionally having one or more atoms or groups selected from group α,
$R^2$, $R^4$ and $R^5$ are a hydrogen atom,
$R^3$ is group J1,
$R^{3a}$, $R^{3b}$ and $R^{3c}$ are the same or different and are a C1 to C6 alkyl group optionally having one or more halogen atoms, and
$R^6$ and $R^7$ are the same or different and are a C1 to C6 chain hydrocarbon group optionally having one or more atoms or groups selected from group α or a hydrogen atom (wherein $R^6$ and $R^7$ do not represent a hydrogen atom at the same time);

In the formula (1), compounds wherein

$A^1$ is $NR^6$, $R^8$ is a C1 to C6 chain hydrocarbon group optionally having one or more atoms or groups selected from group β, $A^2$ is CH, $A^3$ is N, and Q is O,
$R^1$ is a C1 to C6 chain hydrocarbon group optionally having one or more atoms or groups selected from group α,
$R^2$, $R^4$ and $R^5$ are a hydrogen atom,
$R^3$ is group. J2,
$R^{3d}$ is a C1 to C6 chain hydrocarbon group optionally having one or more atoms or groups selected from group β, a C3 to C9 cycloalkyl group optionally having one or more halogen atoms or one or more C1 to C3 alkyl groups, a 6-membered heterocyclic group optionally having one or more atoms or groups selected from group γ or a hydrogen atom, and
$R^6$ and $R^7$ are the same or different and are a C1 to C6 chain hydrocarbon group optionally having one or more

atoms or groups selected from group $\alpha$ or a hydrogen atom (wherein $R^6$ and $R^7$ do not represent a hydrogen atom at the same time);

In the formula (1), compounds wherein

$A^1$ is $NR^8$, $R^8$ is a C1 to C6 chain hydrocarbon group optionally having one or more atoms or groups selected from group $\beta$, $A^2$ is CH, $A^3$ is N, and Q is O,
$R^1$ is a C1 to C6 chain hydrocarbon group optionally having one or more atoms or groups selected from group $\alpha$,
$R^2$, $R^4$ and $R^5$ are a hydrogen atom,
$R^3$ is group J3,
$R^{3e}$ is a C1 to C6 chain hydrocarbon group optionally having one or more halogen atoms or a hydrogen atom and
$R^{3f}$ is a C1 to C6 chain hydrocarbon group optionally having one or more atoms or groups selected from group $\beta$, a C3 to C9 cycloalkyl group optionally having one or more halogen atoms or one or more C1 to C3 alkyl groups, a phenyl group optionally having one or more atoms or groups selected from group $\gamma$, a 5-or 6-membered heterocyclic group optionally having one or more atoms or groups selected from group $\gamma$, $NR^{11}R^{12}$, $OR^{11}$ or a hydrogen atom, or $R^{3e}$ and $R^{3f}$, together with the nitrogen atom to which they are bound, may form a 5- or 6-membered heterocyclic ring optionally having one or more atoms or groups selected from group $\gamma$,
$R^6$ and $R^7$ are the same or different and are a C1 to C6 chain hydrocarbon group optionally having one or more atoms or groups selected from group $\alpha$ or a hydrogen atom (wherein $R^6$ and $R^7$ do not represent a hydrogen atom at the same time), and
$R^{11}$ and $R^{12}$ are the same or different and are a C1 to C6 chain hydrocarbon group optionally having one or more atoms or groups selected from group $\alpha$;

In the formula (1), compounds wherein

$A^1$ is $NR^8$, $R^8$ is a C1 to C6 alkyl group optionally having one or more halogen atoms or one or more cyclopropyl groups (wherein the cyclopropyl group optionally has one or more halogen atoms or one or more C1 to C3 alkyl groups), $A^2$ is CH, $A^3$ is N, and Q is O,
$R^1$ is a C1 to C6 alkyl group optionally having one or more halogen atoms or one or more cyclopropyl groups (wherein the cyclopropyl group optionally has one or more halogen atoms or one or more C1 to C3 alkyl groups),
$R^2$, $R^4$ and $R^5$ are a hydrogen atom,
$R^{3a}$, $R^{3b}$ and $R^{3c}$ are the same or different and are a C1 to C6 alkyl group optionally having one or more halogen atoms,
$R^{3d}$ is a C1 to C6 chain hydrocarbon group optionally having one or more atoms or groups selected from group $\beta$, a C3 to C9 cycloalkyl group optionally having one or more halogen atoms or one or more C1 to C3 alkyl groups, a 6-membered heterocyclic group optionally having one or more atoms or groups selected from group $\gamma$ or a hydrogen atom,
$R^{3e}$ is a C1 to C6 chain hydrocarbon group optionally having one or more halogen atoms or a hydrogen atom and
$R^{3f}$ is a C1 to C6 chain hydrocarbon group optionally having one or more atoms or groups selected from group $\beta$, a C3 to C9 cycloalkyl group optionally having one or more halogen atoms or one or more C1 to C3 alkyl groups, a phenyl group optionally having one or more atoms or groups selected from group $\gamma$, a 5- or 6-membered heterocyclic group optionally having one or more atoms or groups selected from group $\gamma$, $NR^{11a}R^{12a}$, $OR^{11a}$ or a hydrogen atom, or $R^{3e}$ and $R^{3f}$, together with the nitrogen atom to which they are bound, may form a 5- or 6-membered heterocyclic ring optionally having one or more atoms or groups selected from group $\gamma$,
$R^6$ is a C1 to C6 alkyl group optionally having one or more halogen atoms,
$R^7$ is a hydrogen atom, and
$R^{11a}$ and $R^{12a}$ are a C1 to C6 alkyl group optionally having one or more halogen atoms or a hydrogen atom. (wherein, in $S(O)_mR^{11a}$, when m is 1 or 2, $R^{11a}$ does not represent a hydrogen atom);

In the formula (1), compounds wherein

$A^1$ is $NR^8$, $R^8$ is a C1 to C6 alkyl group optionally having one or more halogen atoms or one or more cyclopropyl groups (wherein the cyclopropyl group optionally has one or more halogen atoms or one or more C1 to C3 alkyl groups), $A^2$ is CH, $A^3$ is N, and Q is O,
$R^1$ is a C1 to C6 alkyl group optionally having one or more halogen atoms or one or more cyclopropyl groups (wherein the cyclopropyl group optionally has one or more halogen atoms or one or more C1 to C3 alkyl groups),
$R^2$, $R^4$ and $R^5$ are a hydrogen atom,
$R^{3a}$, $R^{3b}$ and $R^{3c}$ are the same or different and are a C1 to C6 alkyl group optionally having one or more halogen atoms,
$R^{3d}$ is a C1 to C6 chain hydrocarbon group optionally having one or more atoms or groups selected from group $\beta$,

a C3 to C9 cycloalkyl group optionally having one or more halogen atoms or one or more C1 to C3 alkyl groups, a 6-membered heterocyclic group optionally having one or more atoms or groups selected from group γ or a hydrogen atom,
$R^{3e}$ is a C1 to C6 chain hydrocarbon group optionally having one or more halogen atoms or a hydrogen atom and $R^{3f}$ is a C1 to C6 chain hydrocarbon group optionally having one or more atoms or groups selected from group β, a C3 to C9 cycloalkyl group optionally having one or more halogen atoms or one or more C1 to C3 alkyl groups, a phenyl group optionally having one or more atoms or groups selected from group γ, a 5-or 6-membered heterocyclic group optionally having one or more atoms or groups selected from group γ, $NR^{11a}R^{12a}$, $CR^{11a}$ or a hydrogen atom, or $R^{3e}$ and $R^{3f}$, together with the nitrogen atom to which they are bound, may form a 5- or 6-membered heterocyclic ring optionally having one or more atoms or groups selected from group γ,
$R^6$ is a C1 to C6 perfluoroalkyl group,
$R^7$ is a hydrogen atom,
$R^8$ is a C1 to C6 alkyl group optionally having one or more halogen atoms, and
$R^{11a}$ and $R^{12a}$ are a C1 to C6 alkyl group optionally having one or more halogen atoms or a hydrogen atom (wherein, in $S(O)_mR^{11a}$, when m is 1 or 2, Roll‘ does not represent a hydrogen atom);

In the formula (1), compounds wherein

$A^1$ is $NR^8$, $R^8$ is a C1 to C6 chain hydrocarbon group optionally having one or more atoms or groups selected from group β, $A^2$ is CH, $A^3$ is N, and Q is O,
$R^1$ is a C1 to C6 chain hydrocarbon group optionally having one or more atoms or groups selected from group α,
$R^2$, $R^4$ and $R^5$ are a hydrogen atom,
$R^{3d}$ is a C1 to C6 chain hydrocarbon group optionally having one or more atoms or groups selected from group β or a hydrogen atom,
$R^{3e}$ is a C1 to C6 chain hydrocarbon group optionally having one or more halogen atoms,
$R^{3f}$ is a C1 to C6 chain hydrocarbon group optionally having one or more atoms or groups selected from group β or $OR^{11}$,
$R^6$ and $R^7$ are the same or different and are a C1 to C6 chain hydrocarbon group optionally having one or more atoms or groups selected from group a or a hydrogen atom (wherein $R^6$ and $R^7$ do not represent a hydrogen atom at the same time), and
$R^{11}$ is a C1 to C6 chain hydrocarbon group optionally having one or more atoms or groups selected from group α;

In the formula (1), compounds wherein

$A^1$ is $NR^3$, $R^8$ is a C1 to C6 alkyl group optionally having one or more halogen atoms or one or more cyclopropyl groups, $A^2$ is CH, $A^3$ is N, and Q is O,
$R^1$ is a C1 to C6 alkyl group optionally having one or more halogen atoms or one or more cyclopropyl groups,
$R^2$, $R^4$ and $R^5$ are a hydrogen atom,
$R^{3d}$ is a C1 to C6 alkyl group optionally having one or more halogen atoms or a hydrogen atom,
$R^{3e}$ is a C1 to C6 alkyl group optionally having one or more halogen atoms,
$R^{3f}$ is a C1 to C6 alkyl group or $OR^{11}$,
$R^6$ is a C1 to C6 alkyl group optionally having one or more halogen atoms,
$R^7$ is a hydrogen atom, and
$R^{11}$ is a C1 to C6 alkyl group optionally having one or more halogen atoms;

In the formula (1), compounds wherein

$A^1$ is $NR^8$, $R^8$ is a C1 to C6 chain hydrocarbon group optionally having one or more atoms or groups selected from group β, $A^2$ is CH, $A^3$ is N, and Q is O,
$R^1$ is a C1 to C6 chain hydrocarbon group optionally having one or more atoms or groups selected from group α,
$R^2$, $R^4$ and $R^5$ are a hydrogen atom,
$R^3$ is group J2,
$R^{3d}$ is a C1 to C6 chain hydrocarbon group optionally having one or more atoms or groups selected from group β or a hydrogen atom, and
$R^6$ and $R^7$ are the same or different and are a C1 to C6 chain hydrocarbon group optionally having one or more atoms or groups selected from group α or a hydrogen atom (wherein $R^6$ and $R^7$ do not represent a hydrogen atom at the same time);

In the formula (1), compounds wherein

$A^1$ is $NR^8$, $R^8$ is a C1 to C6 alkyl group optionally having one or more halogen atoms or one or more cyclopropyl groups, $A^2$ is CH, $A^3$ is N, and Q is O,
$R^1$ is a C1 to C6 alkyl group optionally having one or more halogen atoms or one or more cyclopropyl groups,
$R^2$, $R^4$ and $R^5$ are a hydrogen atom,
$R^3$ is group J2,
$R^{3d}$ is a C1 to C6 alkyl group optionally having one or more halogen atoms or a hydrogen atom,
$R^6$ is a C1 to C6 alkyl group optionally having one or more halogen atoms, and
$R^7$ is a hydrogen atom;

In the formula (1), compounds wherein

$A^1$ is $NR^8$, $R^8$ is a C1 to C6 chain hydrocarbon group optionally having one or more atoms or groups selected from group β, $A^2$ is CH, $A^3$ is N, and Q is O,
$R^1$ is a C1 to C6 chain hydrocarbon group optionally having one or more atoms or groups selected from group α,
$R^2$, $R^4$ and $R^5$ are a hydrogen atom,
$R^3$ is group J3,
$R^{3e}$ is a C1 to C6 chain hydrocarbon group optionally having one or more halogen atoms,
$R^{3f}$ is a C1 to C6 chain hydrocarbon group optionally having one or more atoms or groups selected from group β or $OR^{11}$,
$R^6$ and $R^7$ are the same or different and are a C1 to C6 chain hydrocarbon group optionally having one or more atoms or groups selected from group α or a hydrogen atom (wherein $R^6$ and $R^7$ do not represent a hydrogen atom at the same time), and
$R^{11}$ is a C1 to C6 chain hydrocarbon group optionally having one or more atoms or groups selected from group α;

In the formula (1), compounds wherein

$A^1$ is $NR^8$, $R^8$ is a C1 to C6 alkyl group optionally having one or more halogen atoms or one or more cyclopropyl groups, $A^2$ is CH, $A^3$ is N, and Q is O,
$R^1$ is a C1 to C6 alkyl group optionally having one or more halogen atoms or one or more cyclopropyl groups,
$R^2$, $R^4$ and $R^5$ are a hydrogen atom,
$R^3$ is group J3,
$R^{3e}$ is a C1 to C6 alkyl group optionally having one or more halogen atoms,
$R^{3f}$ is a C1 to C6 chain hydrocarbon group optionally having one or more atoms or groups selected from group β or $OR^{11}$,
$R^6$ is a C1 to C6 alkyl group optionally having one or more halogen atoms,
$R^7$ is a hydrogen atom, and
$R^{11}$ is a C1 to C6 alkyl group optionally having one or more halogen atoms;

In the formula (1), compounds wherein

$A^1$ is $N(CH_3)$, $A^2$ is CH, $A^3$ is CH or N, and Q is O,
$R^1$ is a C1 to C6 alkyl group optionally having one or more halogen atoms or one or more cyclopropyl groups,
$R^2$, $R^4$ and $R^5$ are a hydrogen atom,
$R^3$ is group J2 or group J3,
$R^{3d}$ is a C1 to C6 alkyl group optionally having one or more halogen atoms or a hydrogen atom,
$R^{3e}$ is a C1 to C6 alkyl group optionally having one or more halogen atoms,
$R^{3f}$ is a C1 to C6 chain hydrocarbon group optionally having one or more atoms or groups selected from group β or $OR^{11}$,
$R^6$ is a C1 to C6 alkyl group optionally having one or more halogen atoms,
$R^7$ is a hydrogen atom, and
$R^{11}$ is a C1 to C6 alkyl group optionally having one or more halogen atoms;

In the formula (1), compounds wherein

$A^1$ is $N(CH_3)$, $A^2$ is CH, $A^3$ is CH or N, and Q is O,
$R^1$ is a C1 to C6 alkyl group optionally having one or more halogen atoms or one or more cyclopropyl groups,

$R^2$, $R^4$ and $R^5$ are a hydrogen atom,
$R^3$ is group J2,
$R^{3e}$ is a C1 to C6 alkyl group optionally having one or more halogen atoms,
$R^{3f}$ is a C1 to C6 chain hydrocarbon group optionally having one or more atoms or groups selected from group β or $OR^{11}$,
$R^6$ is a C1 to C6 alkyl group optionally having one or more halogen atoms,
$R^7$ is as hydrogen atom, and
$R^{11}$ is a C1 to C6 alkyl group optionally having one or more halogen atoms;

In the formula (1), compounds wherein

$A^1$ is $N(CH_3)$, $A^2$ is CH, $A^3$ is CH or N, and Q is O,
$R^1$ is a C1 to C6 alkyl group optionally having one or more halogen atoms or one or more cyclopropyl groups,
$R^2$, $R^4$ and $R^5$ are a hydrogen atom,
$R^3$ is group J3.,
$R^{3e}$ is a C1 to C6 alkyl group optionally having one or more halogen atoms,
$R^{3f}$ is a C1 to C6 chain hydrocarbon group optionally having one or more atoms or groups selected from group β or $OR^{11}$,
$R^6$ is a C1 to C6 alkyl group optionally having one or more halogen atoms,
$R^7$ is a hydrogen atom, and
$R^{11}$ is a C1 to C6 alkyl group optionally having one or more halogen atoms;

In the formula (1), compounds wherein

$A^1$ is $N(CH_3)$, $A^2$ is CH, $A^3$ is N, and Q is O,
$R^1$ is a C1 to C6 alkyl group optionally having one or more halogen atoms or one or more cyclopropyl groups,
$R^2$, $R^4$ and $R^5$ are a hydrogen atom,
$R^3$ is group J2 or group J3,
$R^{3d}$ is a C1 to C6 alkyl group optionally having one or more halogen atoms or a hydrogen atom,
$R^{3e}$ is a C1 to C6 alkyl group optionally having one or more halogen atoms,
$R^{3f}$ is a C1 to C6 chain hydrocarbon group optionally having one or more atoms or groups selected from group β or $OR^{11}$,
$R^6$ is a C1 to C6 alkyl group optionally having one or more halogen atoms,
$R^7$ is a hydrogen atom, and
$R^{11}$ is a C1 to C6 alkyl group optionally having one or more halogen atoms;

In the formula (1), compounds wherein

$A^1$ is $N(CH_3)$, $A^2$ is CH, $A^3$ is CH, and Q is O,
$R^1$ is a C1 to C6 alkyl group optionally having one or more halogen atoms or one or more cyclopropyl groups,
$R^2$, $R^4$ and $R^5$ are a hydrogen atom,
$R^3$ is group J2 or group J3,
$R^{3d}$ is a C1 to C6 alkyl group optionally having one or more halogen atoms or a hydrogen atom,
$R^{3e}$ is a C1 to C6 alkyl group optionally having one or more halogen atoms,
$R^{3f}$ is a C1 to C6 chain hydrocarbon group optionally having one or more atoms or groups selected from group β or $OR^{11}$,
$R^6$ is a C1 to C6 alkyl group optionally having one or more halogen atoms,
$R^7$ is a hydrogen atom, and
$R^{11}$ is a C1 to C6 alkyl group optionally having one or more halogen atoms;

Compounds represented by formula (1-1),

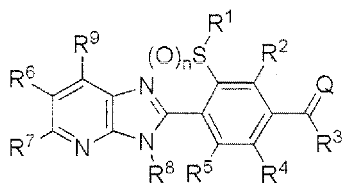

(1-1)

wherein symbols represent the same meaning as in the formula (1) ;
In the formula (1-1), compounds wherein $R^3$ is group J1;
In the formula (1-1), compounds wherein $R^3$ is group J2;
In the formula (1-1), compounds wherein $R^3$ is group J3;
In the formula (1-1), compounds wherein

Q is O,
$R^1$ is an ethyl group,
$R^2$, $R^4$ and $R^5$ are a hydrogen atom,
$R^3$ is group J2 or group J3,
$R^{3d}$ is a C1 to C6 alkyl group optionally having one or more halogen atoms or a hydrogen atom,
$R^{3e}$ is a C1 to C6 alkyl group optionally having one or more halogen atoms,
$R^{3f}$ is a C1 to C6 chain hydrocarbon group optionally having one or more atoms or groups selected from group β or $OR^{11}$,
$R^6$ is a trifluoromethyl group,
$R^7$ is a hydrogen atom, and
$R^{11}$ is a C1 to C6 alkyl group optionally having one or more halogen atoms;

In the formula (1-1), compounds wherein

Q is O,
$R^1$ is an ethyl group,
$R^2$, $R^4$ and $R^5$ are a hydrogen atom,
$R^3$ is group J2,
$R^{3d}$ is a C1 to C6 alkyl group optionally having one or more halogen atoms or a hydrogen atom,
$R^6$ is a trifluoromethyl group,
$R^7$ is a hydrogen atom, and
$R^{11}$ is a C1 to C6 alkyl group optionally having one or more halogen atoms;

In the formula (1-1), compounds wherein

Q is O,
$R^1$ is an ethyl group,
$R^2$, $R^4$ and $R^5$ are a hydrogen atom,
$R^3$ is group J3,
$R^{3e}$ is a C1 to C6 alkyl group optionally having one or more halogen atoms,
$R^{3f}$ is a C1 to C6 chain hydrocarbon group optionally having one or more atoms or groups selected from group β or $OR^{11}$,
$R^6$ is a trifluoromethyl group,
$R^7$ is a hydrogen atom, and
$R^{11}$ is a C1 to C6 alkyl group optionally having one or more halogen atoms;

Compounds represented by formula (1-2),

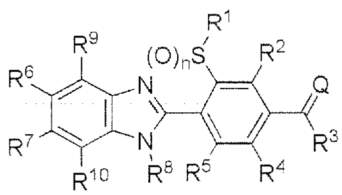
(1-2)

wherein symbols represent the same meaning as in the formula (1) ;
In the formula (1-2), compounds wherein $R^3$ is group J1;
In the formula (1-2), compounds wherein $R^3$ is group J2;
In the formula (1-2), compounds wherein $R^3$ is group J3;
In the formula (1-2), compounds wherein

Q is O,
$R^1$ is an ethyl group,
$R^2$, $R^4$ and $R^5$ are a hydrogen atom,
$R^3$ is group J2 or group J3,
$R^{3c}$ is a C1 to C6 alkyl group optionally having one or more halogen atoms or a hydrogen atom,
$R^{3e}$ is a C1 to C6 alkyl group optionally having one or more halogen atoms,
$R^{3f}$ is a C1 to C6 chain hydrocarbon group optionally having one or more atoms or groups selected from group β or $OR^{11}$,
$R^6$ is a trifluoromethyl group,
$R^1$ is a hydrogen atom, and
$R^{11}$ is a C1 to C6 alkyl group optionally having one or more halogen atoms;

In the formula (1-2), compounds wherein

Q is O,
$R^1$ is an ethyl group,
$R^2$, $R^4$ and $R^b$ are a hydrogen atom,
$R^3$ is group J2,
$R^{3d}$ is a C1 to C6 alkyl group optionally having one or more halogen atoms or a hydrogen atom,
$R^6$ is a trifluoromethyl group,
$R^7$ is a hydrogen atom, and
$R^{11}$ is a C1 to C6 alkyl group optionally having one or more halogen atoms;

In the formula (1-2), compounds wherein

Q is O,
$R^1$ is an ethyl group,
$R^2$, $R^4$ and $R^5$ are a hydrogen atom,
$R^3$ is group J3,
$R^{3e}$ is a C1 to C6 alkyl group optionally having one or more halogen atoms,
$R^{3f}$ is a C1 to C6 chain hydrocarbon group optionally having one or more atoms or groups selected from group β or $OR^{11}$,
$R^6$ is a trifluoromethyl group,
$R^7$ is a hydrogen atom, and
$R^{11}$ is a C1 to C6 alkyl group optionally having one or more halogen atoms;

Compounds represented by formula (1-3),

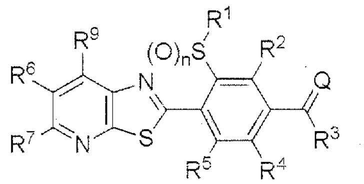
(1-3)

wherein symbols represent the same meaning as in the formula (1);
In the formula (1-3), compounds wherein $R^3$ is group J1;
In the formula (1-3), compounds wherein $R^3$ is group J2;
In the formula (1-3), compounds wherein $R^3$ is group J3; Compounds represented by formula (1-4),

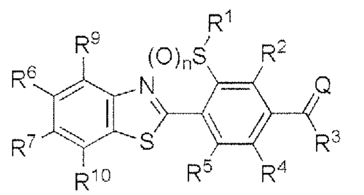
(1-4)

wherein symbols represent the same meaning as in the formula (1) :
In the formula (1-4), compounds wherein $R^3$ is group J1;
In the formula (1-4), compounds wherein $R^3$ is group J2;
In the formula (1-4), compounds wherein $R^3$ is group J3; Compounds represented by formula (1-5),

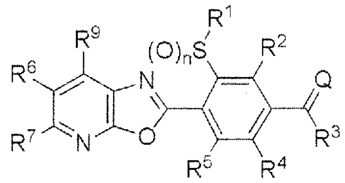
(1-5)

wherein symbols represent the same meaning as in the formula (1);
In the formula (1-5), compounds wherein $R^3$ is group J1;
In the formula (1-5), compounds wherein $R^3$ is group J2;
In the formula (1-5), compounds wherein $R^3$ is group J3; Compounds represented by formula (1-6),

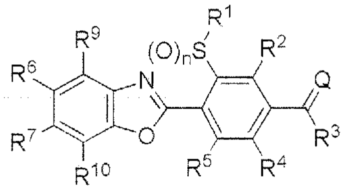
(1-6)

wherein symbols represent the same meaning as in the formula (1);
In the formula (1-6), compounds wherein $R^3$ is group J1;
In the formula (1-6), compounds wherein $R^3$ is group J2;
In the formula (1-6), compounds wherein $R^3$ is group J3; Compounds represented by formula (1A),

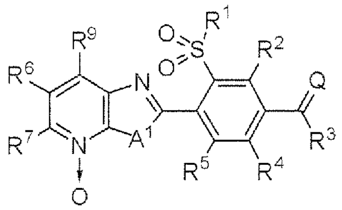
(1A)

wherein symbols represent the same meaning as in the formula (1) ;
In the formula (1A), compounds wherein $A^1$ is $NR^8$;
In the formula (1A), compounds wherein $A^1$ is S;
In the formula (1A), compounds wherein $A^1$ is 0;
In the formula (1A), compounds wherein $R^3$ is group J1;
In the formula (1A), compounds wherein $R^3$ is group J1, and $A^1$ is $NR^8$;
In the formula (1A), compounds wherein $R^3$ is group J1, and $A^1$ is S;
In the formula (1A), compounds wherein $R^3$ is group J1, and $A^1$ is 0;
In the formula (1A), compounds wherein $R^3$ is group J2;
In the formula (1A), compounds wherein $R^3$ is group J2, and $A^1$ is $NR^8$;
In the formula (1A), compounds wherein $R^3$ is group J2, and $A^1$ is S;
In the formula (1A), compounds wherein $R^3$ is group J2, and $A^1$ is 0;
In the formula (1A), compounds wherein $R^3$ is group J3;
In the formula (1A), compounds wherein $R^3$ is group J3, and $A^1$ is $NR^8$;
In the formula (1A), compounds wherein $R^3$ is group J3, and $A^1$ is S;
In the formula (1A), compounds wherein $R^3$ is group J3, and $A^1$ is O.

Next, the method for producing the compound of the present invention will be described.

The compound of the present invention and the intermediate compound can be produced, for example, according to the following (Production Method 1) to (Production Method 20).

(Production Method 1)

The compound of the present invention wherein n is 1 or 2 in the formula (1) can be produced by oxidizing the compound of the present invention (1-n0) wherein n is 0.

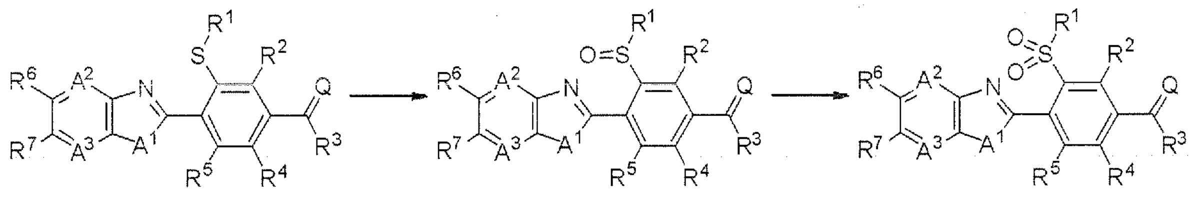

(1-n0) (1-n1) (1-n2)

In the formula, symbols represent the same meaning as in the formula (1).

The compound of the present invention (1-n1) in which n is 1 in the formula (1) can be produced by reacting the compound of the present invention (1-n0) in which n is 0 with an oxidizing agent.

The reaction is usually carried out in a solvent. Examples of the solvent include aliphatic halogenated hydrocarbons such as dichloromethane and chloroform, alcohols such as methanol and ethanol, acetic acid, water, and mixtures thereof.

Examples of the oxidizing agent include sodium periodate and m-chloroperbenzoic acid.

In the reaction, the oxidizing agent is usually used in a ratio of 1 to 3 mol, based on 1 mol of the compound of the present invention (1-n0). Preferably, the oxidizing agent is used in a ratio of 1 to 1.2 mol, based on 1 mol of the compound of the present invention (1-n0).

The reaction temperature is usually within the range of -50 to 50°C, and the reaction time is usually within the range of 0.1 to 12 hours.

After completion of the reaction, the reaction mixture is subjected to post-treatment operations, for example, the reaction mixture is extracted with an organic solvent, and the organic layer is washed with an aqueous solution of a reducing agent (for example, sodium sulfite, sodium thiosulfate) and an aqueous solution of a base (for example, sodium bicarbonate) as necessary, and subjected to drying and concentration, whereby the compound of the present invention (1-n1) can be isolated. The isolated compound of the present invention (1-n1) also can be further purified by chromatography, recrystallization, or the like.

The compound of the present invention (1-n2) in which n is 2 in the formula (1) can be produced by reacting the compound of the present invention (1-n1) in which n is 1 with an oxidizing agent.

The reaction is usually carried out in a solvent. Examples of the solvent include aliphatic halogenated hydrocarbons such as dichloromethane and chloroform, alcohols such as methanol and ethanol, acetic acid, water, and mixtures thereof.

Examples of the oxidizing agent include m-chloroperbenzoic acid, aqueous hydrogen peroxide and oxone (registered trademark).

The reaction also can be carried out in the presence of a catalyst, as necessary. Examples of the catalyst include tungstic acid, sodium tungstate and potassium tungstate.

In the reaction, the oxidizing agent is usually used in a ratio of 1 to 4 mol, and the catalyst is usually used in a ration of 0.01 to 0.5 mol, based on 1 mol of the compound of the present invention (1-n1). Preferably, the oxidizing agent is used in a ratio of 1 to 2 mol, and the catalyst is used in a ratio of 0.05 to 0.2 mol, based on 1 mol of the compound of the present invention (1-n1).

The reaction temperature is usually within the range of -50 to 100°C. The reaction time is usually within the range of 0.1 to 12 hours.

After completion of the reaction, the reaction mixture is subjected to post-treatment operations, for example, the reaction mixture is extracted with an organic solvent, and the organic layer is washed with an aqueous solution of a reducing agent (for example, sodium sulfite, sodium thiosulfate) and an aqueous solution of a base (for example, sodium bicarbonate) as necessary, and subjected to drying and concentration, whereby the compound of the present invention (1-n2) can be isolated. The compound of the present invention (1-n2) also can be further purified by chromatography, recrystallization, or the like.

Here, during the synthesis of the compound of the present invention (1-n2) wherein n is 2, an N-oxide of the compound of the present invention is produced in some cases.

In addition, the compound of the present invention (1-n2) wherein n is 2 in the formula (1) can be produced by a one step reaction (one pot), by reacting the compound of the present invention (1-n0) wherein n is 0 with an oxidizing agent.

The reaction is usually carried out in a solvent. Examples of the solvent include aliphatic halogenated hydrocarbons such as dichloromethane and chloroform, alcohols such as methanol and ethanol, acetic acid, water, and mixtures thereof.

Examples of the oxidizing agent include m-chloroperbenzoic acid, aqueous hydrogen peroxide and oxone (registered trademark).

The reaction also can be carried out in the presence of a catalyst, as necessary. Examples of the catalyst include tungstic acid, sodium tungstate and potassium tungstate.

In the reaction, the oxidizing agent is usually used in a ratio of 2 to 5 mol, and the catalyst is usually used in a ratio of 0.01 to 0.5 mol, based on 1 mol of the compound of the present invention (1-n0). Preferably, the oxidizing agent is used in a ratio of 2 to 3 mol, and the catalyst is used in a ratio of 0.05 to 0.2 mol, based on 1 mol of the compound of the present invention (1-n0).

The reaction temperature is usually within the range of 0 to 120°C, and the reaction time is usually within the range of 0.1 to 12 hours.

After completion of the reaction, the reaction mixture is subjected to post-treatment operations, for example, the reaction mixture is extracted with an organic solvent, and the organic layer is washed with an aqueous solution of a reducing agent (for example, sodium sulfite, sodium thiosulfate) and an aqueous solution of a base (for example, sodium bicarbonate) as necessary, and subjected to drying and concentration, whereby the compound of the present invention (1-n2) can be isolated. The isolated compound of the present invention (1-n2) also can be further purified by chromatography, recrystallization, or the like.

Here, during the synthesis of the compound of the present invention (1-n2) wherein n is 2, an N-oxide of the compound of the present invention is produced in some cases.

(Production Method 2)

Examples of an N-oxide of the compound of the present invention wherein $A^2$ is $CR^9$, and $A^3$ is N, include the compound of the present invention (1A) and the like. The compound of the present invention (1A) can be produced by reacting the compound of the present invention ($1\text{-n2-}A^2\text{=}CR^9\text{-}A^3\text{=}N$) in which n is 2, $A^2$ is $CR^9$, and $A^3$ is N, with an oxidizing agent.

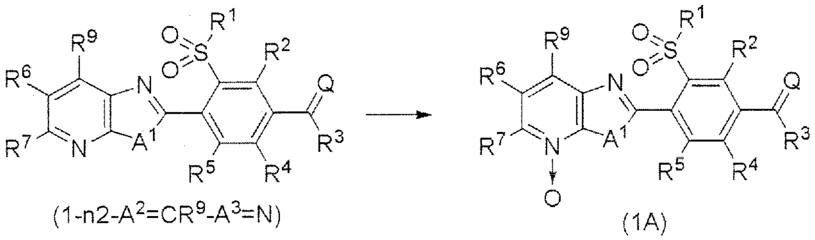

($1\text{-n2-}A^2\text{=}CR^9\text{-}A^3\text{=}N$) (1A)

In the formula, symbols represent the same meaning as in the formula (1).

The reaction is usually carried out in a solvent. Examples of the solvent include aliphatic halogenated hydrocarbons such as dichloromethane and chloroform, alcohols such as methanol and ethanol, acetic acid, water, and mixtures thereof.

Examples of the oxidizing agent include m-chloroperbenzoic acid, aqueous hydrogen peroxide and oxone (registered trademark).

The reaction is carried out in the presence of a catalyst, as necessary. Examples of the catalyst include tungstic acid, sodium tungstate and potassium tungstate.

In the reaction, the oxidizing agent is usually used in a ratio of 1 to 10 mol, and the catalyst is usually used in a ratio of 0.01 to 0.5 mol, based on 1 mol of the compound of the present invention ($1\text{-n2-}A^2\text{=}CR^9\text{-}A^3\text{=}N$). Preferably, the oxidizing agent is used in a ratio of 2 to 5 mol, and the catalyst is used in a ratio of 0.05 to 0.2 mol, based on 1 mol of the compound of the present invention ($1\text{-n2-}A^2\text{=}CR^9\text{-}A^3\text{=}N$).

The reaction temperature is usually within the range of 20 to 120°C, and the reaction time is usually within the range of 0.1 to 48 hours.

After completion of the reaction, the reaction mixture is subjected to post-treatment operations, for example, the reaction mixture is extracted with an organic solvent, and the organic layer is washed with an aqueous solution of a reducing agent (for example, sodium sulfite, sodium thiosulfate) and an aqueous solution of a base (for example, sodium bicarbonate) as necessary, and subjected to drying and concentration, whereby the compound of the present invention (1A) can be isolated. The isolated compound of the present invention (1A) also can be further purified by chromatography, recrystallization, or the like.

(Production Method 3-1)

The compound of the present invention can be produced by reacting intermediate compound (M1) with intermediate compound (M2) to produce intermediate compound (M3), and then intramolecularly condensing the obtained intermediate compound (M3), or produced by a one step reaction (one pot), by reacting the intermediate compound (M1) with the intermediate compound (M2).

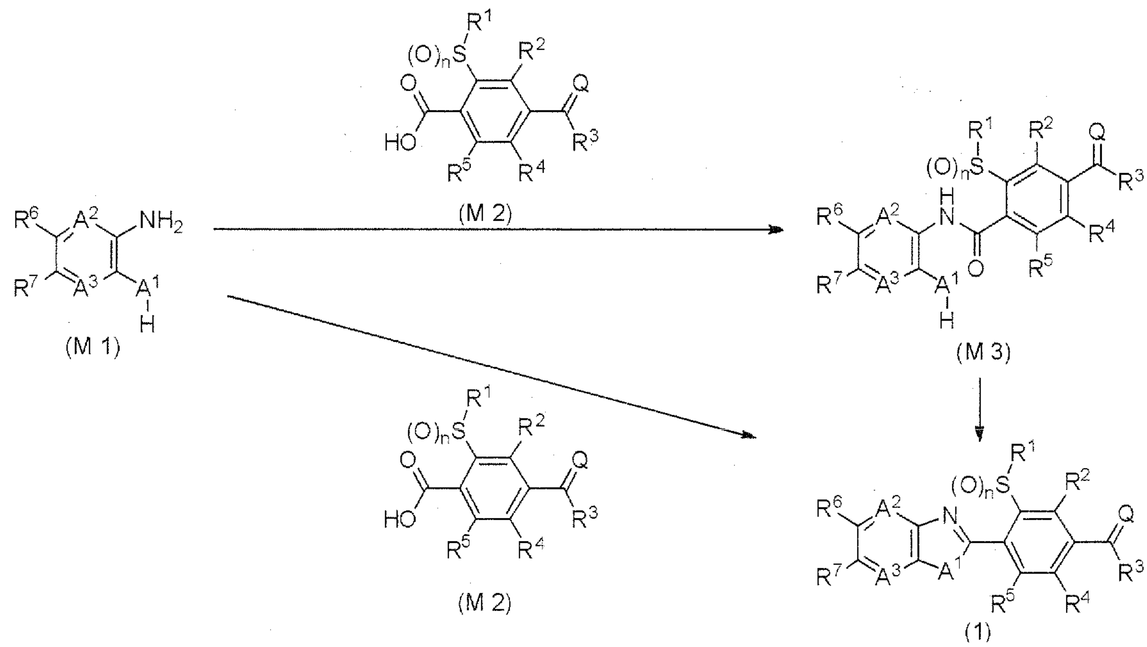

(M 1) (M 2) (M 3) (M 2) (1)

In the formula, symbols represent the same meaning as in the formula (1).

The intermediate compound (M3) can be produced by reacting the intermediate compound (M1) with the intermediate compound (M2), in the presence of a condensing agent.

The reaction is usually carried out in a solvent. Examples of the solvent include ethers such as 1,4-dioxane, diethyl ether, tetrahydrofuran (hereinafter, referred to as THF) and tert-butyl methyl ether, halogenated hydrocarbons such as dichloromethane, chloroform, carbon tetrachloride, 1,2-dichloroethane and chlorobenzene, aromatic hydrocarbons such as toluene, benzene and xylene, esters such as ethyl acetate and butyl acetate, nitriles such as acetonitrile, aprotic polar solvents such as N,N-dimethylformamide (hereinafter, referred to as DMF), N-methyl pyrrolidone (hereinafter, referred to as NMP), 1,3-dimethyl-2-imidazolidinone and dimethyl sulfoxide (hereinafter, referred to as DMSO), nitrogen-containing aromatic compounds such as pyridine and quinolone, and mixtures thereof.

Examples of the condensing agent include carbodiimides such as 1-ethyl-3-(3-dimethylaminopropyl)carbodiimide hydrochloride (hereinafter, referred to as EDAC) and 1,3-dicyclohexylcarbodiimide.

The reaction also can be carried out in the presence of a catalyst, as necessary. Examples of the catalyst include 1-hydroxybenzotriazole (hereinafter, referred to as HOBt).

In the reaction, the intermediate compound (M2) is usually used in a ratio of 0.5 to 2 mol, the condensing agent is usually used in a ratio of 1 to 5 mol, and the catalyst is usually used in a ratio of 0.01 to 1 mol, based on 1 mol of the intermediate compound (M1).

The reaction temperature is usually within the range of 0 to 120°C, and the reaction time is usually within the range of 0.1 to 24 hours.

After completion of the reaction, the intermediate compound (M3) can be isolated by adding the reaction mixture to water, then extracting the mixture with an organic solvent, and concentrating the organic layer; collecting by filtration a solid generated by adding the reaction mixture to water; or collecting by filtration a solid generated in the reaction mixture. The isolated intermediate compound (M3) also can be further purified by recrystallization, chromatography, or the like.

The compound of the present invention (1) can be produced by intramolecular condensation of the intermediate compound (M3) .

The reaction is usually carried out in a solvent. Examples of the solvent include ethers such as 1,4-dioxane, diethyl ether, THF and tert-butyl methyl ether, halogenated hydrocarbons such as dichloromethane, chloroform, carbon tetrachloride, 1,2-dichloroethane and chlorobenzene, aromatic hydrocarbons such as toluene, benzene and xylene,

esters such as ethyl acetate and butyl acetate, nitriles such as acetonitrile, aprotic polar solvents such as DMF, NMP, 1,3-dimethyl-2-imidazolidinone and DMSO, nitrogen-containing aromatic compounds such as pyridine and quinoline, and mixtures thereof.

In the reaction, a condensing agent, an acid, a base or a chlorinating agent can be used, as necessary.

Examples of the condensing agent include acetic anhydride, trifluoroacetic anhydride, EDAC, a mixture of triphenylphosphine, a base and carbon tetrachloride or carbon tetrabromide, and a mixture of triphenylphosphine and an azodiester such as diethyl azodicarboxylate.

Examples of the acid include sulfonic acids such as p-toluenesulfonic acid, carboxylic acids such as acetic acid, and polyphosphoric acid.

Examples of the base include nitrogen-containing heterocyclic compounds such as pyridine, picoline, 2,6-lutidine, 1,8-diazabicyclo[5.4.0]undec-7-ene (hereinafter, referred to as DBU) and 1,5-diazabicyclo[4.3.0]non-5-ene, tertiary amines such as triethylamine and N,N-diisopropylethylamine, and inorganic bases such as tripotassium phosphate, potassium carbonate and sodium hydride.

Examples of the chlorinating agent include phosphorus oxychloride.

In the reaction, when a condensing agent is used, the condensing agent is usually used in a ratio of 1 to 5 mol, when an acid is used, the acid is usually used in a ratio of 0.1 to 5 mol, when a base is used, the base is usually used in a ratio of 1 to 5 mol, and when a chlorinating agent is used, the chlorinating agent is usually used in a ratio of 1 to 5 mol, based on 1 mol of the intermediate compound (M3).

The reaction temperature is usually within the range of 0 to 200°C, and the reaction time is usually within the range of 0.1 to 24 hours.

After completion of the reaction, the compound of the present invention (1) can be isolated by adding the reaction mixture to water, then extracting the mixture with an organic solvent, and concentrating the organic layer; collecting by filtration a solid generated by adding the reaction mixture to water; or collecting by filtration a solid generated in the reaction mixture. The isolated compound of the present invention (1) also can be further purified by recrystallization, chromatography, or the like.

The compound of the present invention (1) can be produced by a one step reaction (one pot) by reacting the intermediate compound (M1) with the intermediate compound (M2), in the presence of a condensing agent.

The reaction is usually carried out in a solvent. Examples of the solvent include ethers such as 1,4-dioxane, diethyl ether, THF and tert-butyl methyl ether, halogenated hydrocarbons such as dichloromethane, chloroform, carbon tetrachloride, 1, 2-dichloroethane and chlorobenzene, aromatic hydrocarbons such as toluene, benzene and xylene, esters such as ethyl acetate and butyl acetate, nitriles such as acetonitrile, aprotic polar solvents such as DMF, NMP, 1,3-dimethyl-2-imidazolidinone and DMSO, nitrogen-containing aromatic compounds such as pyridine and quinoline, and mixtures thereof.

Examples of the condensing agent include carbodiimides such as EDAC and 1, 3-dicyclohexylcarbodiimide, and boric acid.

The reaction also can be carried out in the presence of a catalyst, as necessary. Examples of the catalyst include HOBt.

In the reaction, the intermediate compound (M2) is usually used in a ratio of 0.5 to 2 mol, the condensing agent is usually used in a ratio of 1 to 5 mol, and the catalyst is usually used in a ratio of 0.01 to 1 mol, based on 1 mol of the intermediate compound (M1).

The reaction temperature is usually within the range of 0 to 200°C, and the reaction time is usually within the range of 0.1 to 24 hours.

After completion of the reaction, the compound of the present invention (1) can be isolated by adding the reaction mixture to water, then extracting the mixture with an organic solvent, and concentrating the organic layer; collecting by filtration a solid generated by adding the reaction mixture to water; or collecting by filtration a solid generated in the reaction mixture. The isolated compound of the present invention (1) also can be further purified by recrystallization, chromatography, or the like.

(Production Method 3-2)

The compound of the present invention (1) can be produced by reacting the intermediate compound (M1) with intermediate compound (M4) to produce the intermediate compound (M3), and then intramolecularly condensing the obtained intermediate compound (M3).

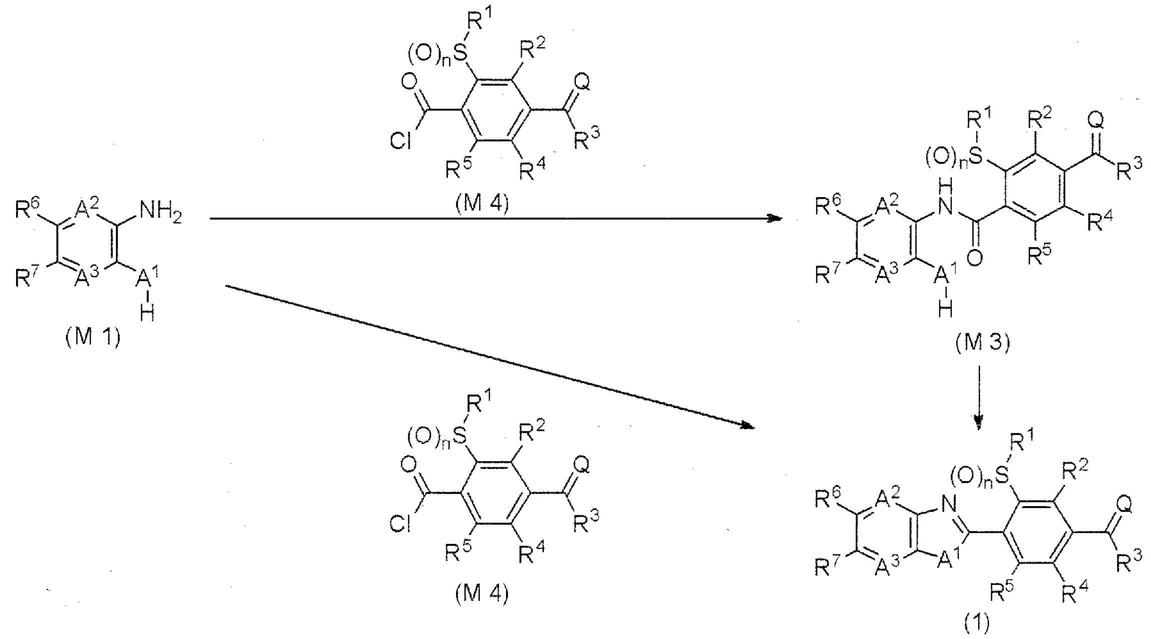

In the formula, symbols represent the same meaning as in the formula (1).

The intermediate compound (M3) can be produced by reacting the intermediate compound (M1) with the intermediate compound (M4).

When the reaction is carried out in a solvent, examples of the solvent include ethers such as THF, ethylene glycol dimethyl ether, tert-butyl methyl ether and 1,4-dioxane, aliphatic hydrocarbons such as hexane, heptane and octane, aromatic hydrocarbons such as toluene and xylene, halogenated hydrocarbons such as chlorobenzene, esters such as ethyl acetate and butyl acetate, nitriles such as acetonitrile, aprotic polar solvents such as DMF, NMP and DMSO, and mixtures thereof.

The reaction also can be carried out in the presence of a base, as necessary. The base includes alkali metal carbonates such as sodium carbonate and potassium carbonate, tertiary amines such as triethylamine and N,N-diisopropylethylamine, and nitrogen-containing aromatic compounds such as pyridine and 4-dimethylaminopyridine, and the like.

In the reaction, the intermediate compound (M4) is usually used in a ratio of 1 to 3 mol, and the base is usually used in a ratio of 1 to 10 mol, based on 1 mol of the intermediate compound (M1).

The reaction temperature is usually within the range of -20 to 100°C. The reaction time is usually within the range of 0.1 to 24 hours.

After completion of the reaction, the reaction mixture is subjected to post-treatment operations, for example, water is added to the reaction mixture, then the mixture is extracted with an organic solvent, and the organic layer is subjected to drying and concentration, whereby the intermediate compound (M3) can be isolated. The isolated intermediate compound (M3) also can be further purified by chromatography, recrystallization, or the like.

The compound of the present invention (1) can be produced by intramolecular condensation of the intermediate compound (M3), according to the method described in Production Method 3-1.

The compound of the present invention (1) can be produced by a one step reaction (one pot) by reacting the intermediate compound (M1) with the intermediate compound (M4).

When the reaction is carried out in a solvent, examples of the solvent include ethers such as THF, ethylene glycol dimethyl ether, tert-butyl methyl ether and 1,4-dioxane, aliphatic hydrocarbons such as hexane, heptane and octane, aromatic hydrocarbons such as toluene and xylene, halogenated hydrocarbons such as chlorobenzene, esters such as ethyl acetate and butyl acetate, nitriles such as acetonitrile, aprotic polar solvents such as DMF, NMP and DMSO, and mixtures thereof.

The reaction also can be carried out in the presence of a base, as necessary.. The base includes alkali metal carbonates such as sodium carbonate and potassium carbonate, tertiary amines such as triethylamine and N,N-diisopropylethylamine, and nitrogen-containing aromatic compounds such as pyridine and 4-dimethylaminopyridine, and the like.

In the reaction, the intermediate compound (M4) is usually used in a ratio of 1 to 3 mol, and the base is usually used in a ratio of 1 to 10 mol, based on 1 mol of the intermediate compound (M1).

The reaction temperature is usually within the range of 20 to 200°C, and the reaction time is usually within the

range of 0.1 to 24 hours.

After completion of the reaction, the reaction mixture is subjected to post-treatment operations, for example, water is added to the reaction mixture, then the mixture is extracted with an organic solvent, and the organic layer is subjected to drying and concentration, whereby the compound of the present invention (1) can be isolated. The isolated compound of the present invention (1) also can be further purified by chromatography, recrystallization, or the like.

(Production Method 4-1)

The compound of the present invention (1-$A^1$=S-$A^3$=N) wherein $A^1$ is S, and $A^3$ is N, in the formula (1), can be produced by reacting intermediate compound (M5) with the intermediate compound (M2) to produce intermediate compound (M6), and then reacting the obtained intermediate compound (M6) with a sulfurizing agent.

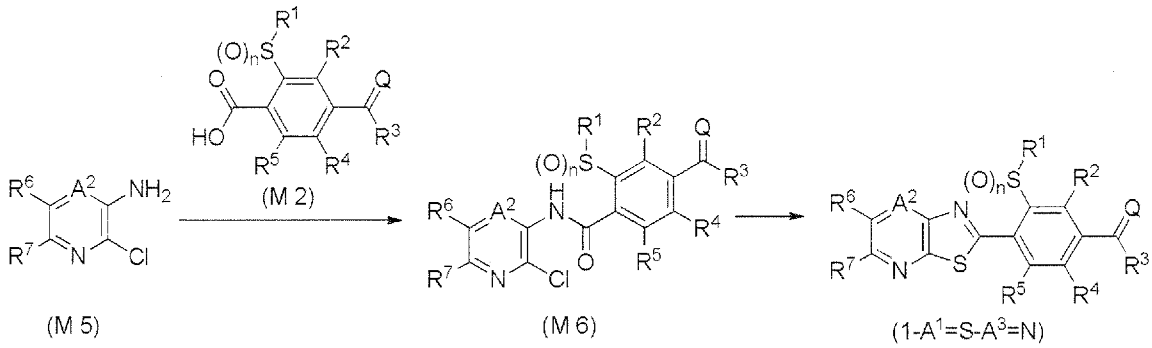

In the formula, symbols represent the same meaning as in the formula (1).

The intermediate compound (M6) can be produced by reacting the intermediate compound (M5) with the intermediate compound (M2), in the presence of a dehydration condensing agent.

The reaction is usually carried out in a solvent. Examples of the solvent include ethers such as THF, ethylene glycol dimethyl ether, tert-butyl methyl ether and 1, 4-dioxane, aliphatic hydrocarbons such as hexane, heptane and octane, aromatic hydrocarbons such as toluene and xylene, halogenated hydrocarbons such as chlorobenzene, esters such as ethyl acetate and butyl acetate, nitriles such as acetonitrile, aprotic polar solvents such as DMF, NMP and DMSO, nitrogen-containing aromatic compounds such as pyridine and quinoline, and mixtures thereof.

Examples of the dehydration condensing agent used in the reaction include carbodiimides such as EDAC and 1,3-dicyclohexylcarbodiimide, and BOP reagent.

In the reaction, the intermediate compound (M2) is usually used in a ratio of 1 to 3 mol, and the dehydration condensing agent is usually used in a ratio of 1 to 5 mol, based on 1 mol of the intermediate compound (M5).

The reaction temperature is usually within the range of 0 to 200°C, and the reaction time is usually within the range of 0.1 to 24 hours.

After completion of the reaction, the reaction mixture is subjected to post-treatment operations, for example, water is added to the reaction mixture, then the mixture is extracted with an organic solvent, and the organic layer is subjected to drying and concentration, whereby the intermediate compound (M6) can be isolated. The isolated intermediate compound (M6) also can be further purified by chromatography, recrystallization, or the like.

The compound of the present invention (1-$A^1$=S-$A^3$=N) can be produced by reacting the intermediate compound (M6) with a sulfurizing agent.

The reaction is usually carried out in a solvent. Examples of the solvent include ethers such as 1,4-dioxane, diethyl ether, tetrahydrofuran, tert-butyl methyl ether and diglyme, halogenated hydrocarbons such as dichloromethane, chloroform, carbon tetrachloride, 1,2-dichloroethane and chlorobenzene, aromatic hydrocarbons such as toluene, benzene and xylene, nitriles such as acetonitrile, nitrogen-containing aromatic compounds such as pyridine, picoline, lutidine and quinoline, and mixtures thereof.

Examples of the sulfurizing agent include diphosphorus pentasulfide and Lawesson's reagent (2,4-bis-(4-methoxyphenyl)-1,3-dithia-2,4-diphosphetane-2,4 -disulfide).

In the reaction, the sulfurizing agent is usually used in a ratio of 1 to 3 mol, based on 1 mol of the intermediate compound (M6).

The reaction temperature is usually within the range of 0 to 200°C, and the reaction time is usually within the range of 1 to 24 hours.

After completion of the reaction, the compound of the present invention (1-$A^1$=S-$A^3$=N) can be isolated by adding the reaction mixture to water, then extracting the mixture with an organic solvent, and concentrating the organic

layer; collecting by filtration a solid generated by adding the reaction mixture to water; or collecting by filtration a solid generated in the reaction mixture. The isolated compound of the present invention (1-$A^1$=S-$A^3$=N) also can be further purified by recrystallization, chromatography, or the like.

(Production Method 4-2)

The compound of the present invention (1-$A^1$=S-$A^3$=N) wherein $A^1$ is S, and $A^3$ is N, in the formula (1), can be produced by reacting intermediate compound (M5) with the intermediate compound (M4) to produce intermediate compound (M6), and then reacting the obtained intermediate compound (M6) with a sulfurizing agent.

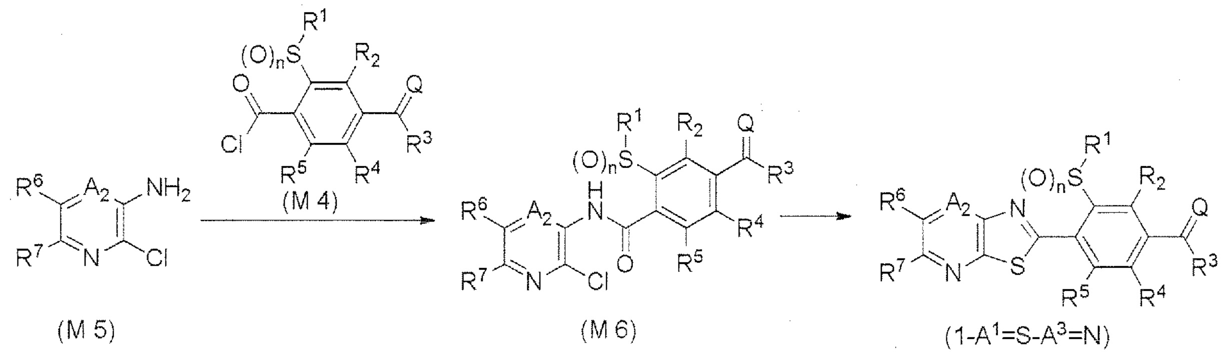

The intermediate compound (M6) can be produced by reacting the intermediate compound (M5) with the intermediate compound (M4).

When the reaction is carried out in a solvent, examples of the solvent include ethers such as THF, ethylene glycol dimethyl ether, tert-butyl methyl ether and 1,4-dioxane, aliphatic hydrocarbons such as hexane, heptane and octane, aromatic hydrocarbons such as toluene and xylene, halogenated hydrocarbons such as chlorobenzene, esters such as ethyl acetate and butyl acetate, nitriles such as acetonitrile, aprotic polar solvents such as DMF, NMP and DMSO, nitrogen-containing aromatic compounds such as pyridine and quinoline, and mixtures thereof.

The reaction also can be carried out in the presence of a base, as necessary. The base includes alkali metal carbonates such as sodium carbonate and potassium carbonate, tertiary amines such as triethylamine and N,N-diisopropylethylamine, and nitrogen-containing aromatic compounds such as pyridine and 4-dimethylaminopyridine, and the like.

In the reaction, the intermediate compound (M4) is usually used in a ratio of 1 to 3 mol, and the base is usually used in a ratio of 1 to 5 mol, based on 1 mol of the intermediate compound (M5).

The reaction temperature is usually within the range of 0 to 200°C, and the reaction time is usually.within the range of 0.1 to 24 hours.

After completion of the reaction, the reaction mixture is subjected to post-treatment operations, for example, water is added to the reaction mixture, then the mixture is extracted with an organic solvent, and the organic layer is subjected to drying and concentration, whereby the intermediate compound (M6) can be isolated. The isolated intermediate compound (M6) also can be further purified by chromatography, recrystallization, or the like.

The compound of the present invention (1-$A^1$=S-$A^3$=N) can be produced by reacting the intermediate compound (M6) with a sulfurizing agent, according to the method described in Production Method 4-1.

(Production Method 5)

The compound of the present invention can be produced by reacting the intermediate compound (M1) with intermediate compound (M7), in the presence of an oxidizing agent.

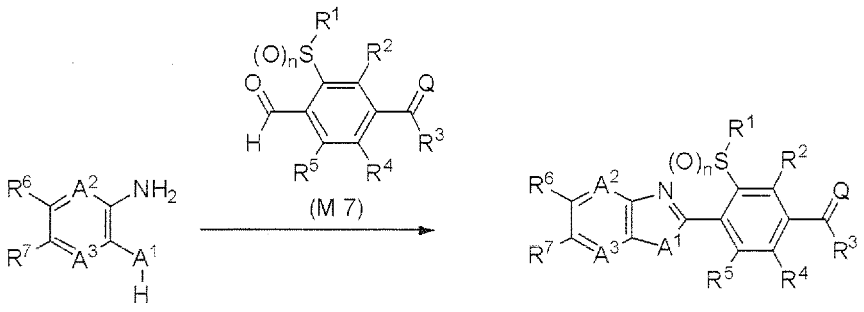


(M 1) (1)

In the formula, symbols represent the same meaning as in the formula (1).

The reaction is usually carried out in a solvent. Examples of the solvent include alcohols such as methanol and ethanol, ethers such as 1,4-dioxane, diethyl ether, THF and tert-butyl methyl ether, halogenated hydrocarbons such as dichloromethane, chloroform, carbon tetrachloride, 1,2-dichloroethane and chlorobenzene, aromatic hydrocarbons such as toluene, benzene and xylene, esters such as ethyl acetate and butyl acetate, nitriles such as acetonitrile, aprotic polar solvents such as DMF, NMP, 1,3-dimethyl-2-imidazolidinone and DMSO, nitrogen-containing aromatic compounds such as pyridine and quinoline, and mixtures thereof.

Examples of the oxidizing agent include oxygen, copper (II) chloride and DDQ.

The reaction also can be carried out in the presence of an acid, as necessary. Examples of the acid include sulfonic acids such as p-toluenesulfonic acid, carboxylic acids such as acetic acid, and polyphosphoric acid.

The reaction also can be carried out in the presence of a sulfite, as necessary. Examples of the sulfite include sodium bisulfite and sodium disulfite.

In the reaction, the oxidizing agent is usually used in a ratio of 1 to 5 mol, the intermediate compound (M7) is usually used in a ratio of 1 to 2 mol, the acid is usually used in a ratio of 0.1 to 2 mol, and the sulfite is usually used in a ratio of 1 to 5 mol, based on 1 mol of the intermediate compound (M1).

The reaction temperature is usually within the range of 0 to 200°C, and the reaction time is usually within the range of 0.1 to 24 hours.

After completion of the reaction, the compound of the present invention (1) can be isolated by adding the reaction mixture to water, then extracting the mixture with an organic solvent, and concentrating the organic layer; collecting by filtration a solid generated by adding the reaction mixture to water; or collecting by filtration a solid generated in the reaction mixture. The isolated compound of the present invention (1) also can be further purified by recrystallization, chromatography, or the like.

(Production Method 6).

The compound of the present invention (1-n0) wherein n is 0 in the formula (1) can be produced by reacting intermediate compound (M8) with compound (M9), in the presence of a base.

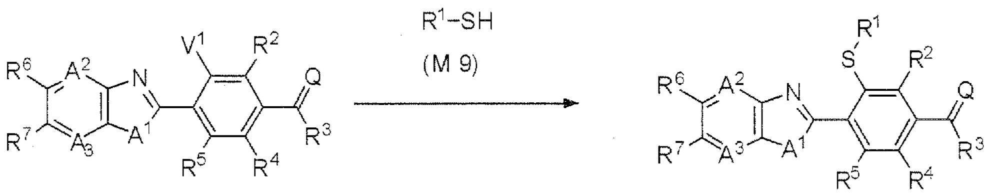


(M 8) (1-n0)

In the formula, $V^1$ represents a halogen atom, and other symbols represent the same meaning as in the formula (1).

The reaction is usually carried out in a solvent. Examples of the solvent include ethers such as THF, ethylene glycol dimethyl ether, tert-butyl methyl ether and 1, 4-dioxane, aromatic hydrocarbons such as toluene and xylene, nitriles such as acetonitrile, aprotic polar solvents such as DMF, NMP and DMSO, water, and mixtures thereof.

Examples of the base include alkali metal carbonates such as sodium carbonate and potassium carbonate, and alkali metal hydrides such as sodium hydride.

In the reaction, the compound (M9) is usually used in a ratio of 1 to 10 mol, and the base is usually used in a ratio of 1 to 10 mol, based on 1 mol of the intermediate compound (M8).

The reaction temperature is usually within the range of 0 to 150°C, and the reaction time is usually within the range of 0.5 to 24 hours.

After completion of the reaction, the reaction mixture is subjected to post-treatment operations, for example, the reaction mixture is extracted with an organic solvent, and the organic layer is subjected to drying and concentration, whereby the compound of the present invention (1-n0) wherein n is 0 can be isolated. The isolated compound of the present invention (1-n0) wherein n is 0 also can be further purified by chromatography, recrystallization, or the like.

In the reaction, $V^1$ is preferably a fluorine atom or a chlorine atom.

(Production Method 7)

The intermediate compound (M8) can be produced by reacting the intermediate compound (M1) with intermediate compound (M10) or intermediate compound (M11) to produce intermediate compound (M12), and then intramolecularly condensing the obtained intermediate compound (M12).

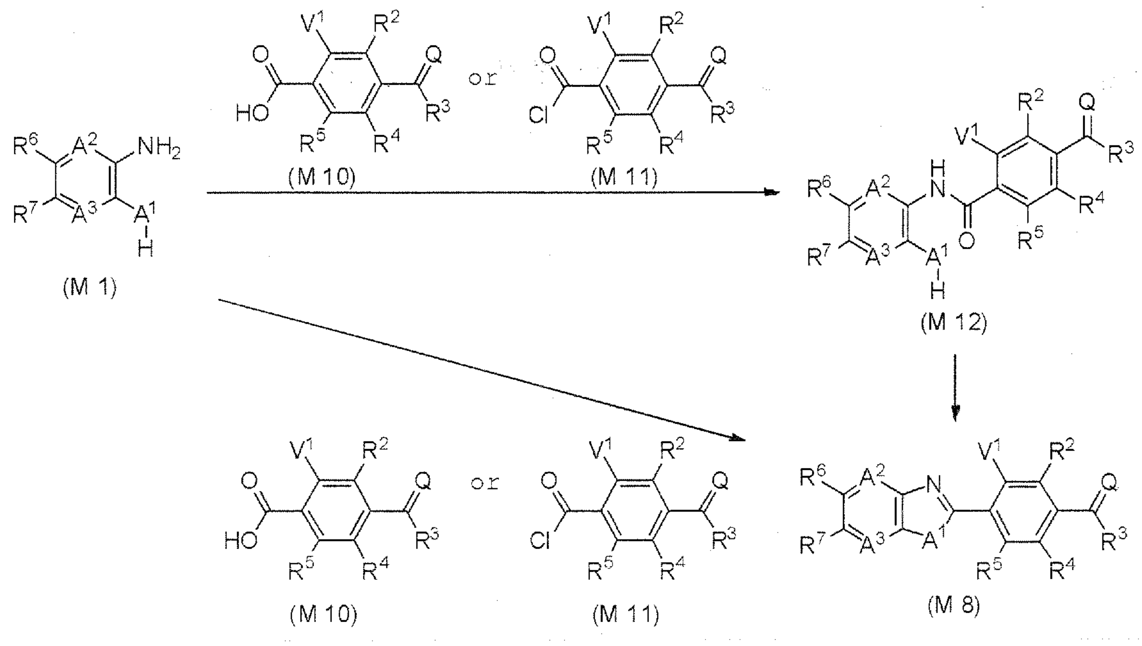
(M 1)
(M 10) or (M 11)
(M 12)
(M 10) or (M 11)
(M 8)

In the formula, symbols represent the same meaning as described above.

The intermediate compound (M12) can be produced, using the intermediate compound (M10) in place of the intermediate compound (M2), in accordance with the method of Production Method 3-1.

The intermediate compound (M12) can be produced, using the intermediate compound (M11) in place of the intermediate compound (M4), in accordance with the method of Production Method 3-2.

The intermediate compound (M8) can be produced, using the intermediate compound (M12) in place of the intermediate compound (M3), in accordance with the method of Production Method 3-1.

Also, the intermediate compound (M8) can be produced by a one step reaction (one pot), using the intermediate compound (M10) in place of the intermediate compound (M2), in accordance with the method of Production Method 3-1.

Also, the intermediate compound (M8) can be produced by a one step reaction (one pot), using the intermediate compound (M11) in place of the intermediate compound (M4), in accordance with the method of Production Method 3-2.

In the reaction, $V^1$ is preferably a fluorine atom or a chlorine atom.

(Production Method 8)

The compound of the present invention (1-n0) in which n is 0 in the formula (1) can be produced by reacting the intermediate compound (M8) with a sulfurizing agent to produce intermediate compound (M13) and reacting the intermediate compound (M13) with compound (M14), in the presence of a base.

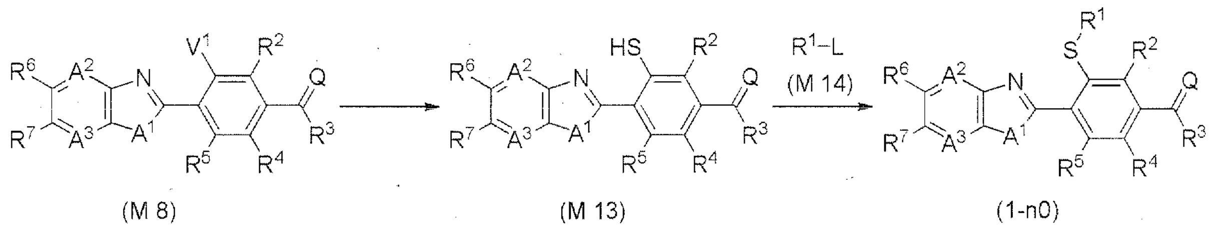


In the formula, L represents a leaving group such as a chlorine atom, a bromine atom, an iodine atom, a trifluoromethanesulfonyloxy group or a methanesulfonyloxy group, and other symbols represent the same meaning as in the formula (1).

(Production Method 8-1)

The intermediate compound (M13) can be produced by reacting the intermediate compound (M8) with a sulfurizing agent.

The reaction is usually carried out in a solvent. Examples of the solvent include ethers such as THF, ethylene glycol dimethyl ether, MTBE and 1,4-dioxane, aromatic hydrocarbons such as toluene and xylene, nitriles such as acetonitrile, aprotic polar solvents such as DMF, NMP and DMSO, water, and mixtures thereof.

Examples of the sulfurizing agent include sodium sulfide and sodium sulfide nonahydrate.

In the reaction, the sulfurizing agent is usually used in a ratio of 1 to 2 mol, based on 1 mol of the intermediate compound (M8).

The reaction temperature is usually within the range of 0 to 150°C, and the reaction time is usually within the range of 0.5 to 24 hours.

After completion of the reaction, the reaction mixture is subjected to post-treatment operations, for example, the reaction mixture is extracted with an organic solvent, and the organic layer is subjected to drying and concentration, whereby the intermediate compound (M13) can be isolated. The isolated intermediate compound (M13) also can be further purified by chromatography, recrystallization, or the like.

In the reaction, $V^1$ is preferably a fluorine atom or a chlorine atom.

(Production Method 8-2)

The compound of the present invention (1-n0) can be produced by reacting the intermediate compound (M13) with the compound (M14), in the presence of a base.

The reaction is usually carried out in a solvent. Examples of the solvent include ethers such as THF, ethylene glycol dimethyl ether, tert-butyl methyl ether and 1, 4-dioxane, aromatic hydrocarbons such as toluene and xylene, nitriles such as acetonitrile, aprotic polar solvents such as DMF, NMP and DMSO, and mixtures thereof.

Examples of the base include hydrides of alkali metals and alkaline earth metals such as sodium hydride, potassium hydride and calcium hydride, inorganic bases such as sodium carbonate and potassium carbonate, and organic bases such as triethylamine.

In the reaction, the compound (M14) is usually used in a ratio of 1 to 10 mol, and the base is usually used in a ratio of 1 to 10 mol, based on 1 mol of the intermediate compound (M13).

The reaction temperature is usually within the range of 0 to 100°C, and the reaction time is usually within the range of 0.1 to 24 hours.

After completion of the reaction, the reaction mixture is subjected to post-treatment operations, for example, the reaction mixture is extracted with an organic solvent, and the organic layer is subjected to drying and concentration, whereby the compound of the present invention (1-n0) wherein n is 0 can be isolated. The isolated compound of the present invention (1-n0) also can be further purified by chromatography, recrystallization, or the like.

(Production Method 9)

The compound of the present invention (1-$A^1$=$NR^{8a}$) wherein $A^1$ is $NR^{8a}$ in the formula (1) can be produced by reacting the compound of the present invention (1-$A^1$=NH) wherein $A^1$ is NH in the formula (1) with compound (M15), in the presence of a base.

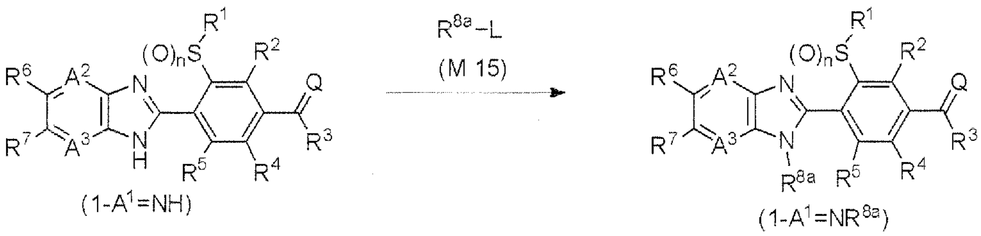


(1-$A^1$=NH) (1-$A^1$=$NR^{8a}$)

In the formula, $R^{8a}$ is a C1 to C6 chain hydrocarbon group optionally having one or more atoms or groups selected from group β, a C3 to C6 cycloalkyl group optionally having one or more halogen atoms or one or more C1 to C3 alkyl groups, $C(O)R^{11}$ or $CO_2R^{11}$, and other symbols represent the same meaning as described above.

The reaction is usually carried out in a solvent. Examples of the solvent include ethers such as THF, ethylene glycol dimethyl ether, tert-butyl methyl ether and 1,4-dioxane, aromatic hydrocarbons such as toluene and xylene, nitriles such as acetonitrile, aprotic polar solvents such as DMF, NMP and DMSO, and mixtures thereof.

Examples of the base include hydrides of alkali metals and alkaline earth metals such as sodium hydride, potassium hydride and calcium hydride, inorganic bases such as sodium carbonate and potassium carbonate, and organic bases such as triethylamine.

In the reaction, the compound (M15) is usually used in a ratio of 1 to 5 mol, and the base is usually used in a ratio of 1 to 3 mol, based on 1 mol of the compound of the present invention (1-$A^1$=NH).

The reaction temperature is usually within the range of 0 to 100°C, and the reaction time is usually within the range of 0.1 to 24 hours.

After completion of the reaction, the reaction mixture is subjected to post-treatment operations, for example, the reaction mixture is extracted with an organic solvent, and the organic layer is subjected to drying and concentration, whereby the compound of the present invention (1-$A^1$=$NR^{8a}$) can be isolated. The isolated compound of the present invention (1-$A^1$=$NR^{8a}$) also can be further purified by chromatography, recrystallization, or the like.

(Production Method 10)

The compound of the present invention (1-$R^6$=Rf) wherein $R^6$ is a C1 to C6 perfluoroalkyl group in the formula (1) can be produced by reacting the compound of the present invention (1-$R^6$=$V^1$) wherein $R^6$ is a halogen atom in the formula (1) with compound (M16) or compound (M17), in the presence of copper or a copper compound.

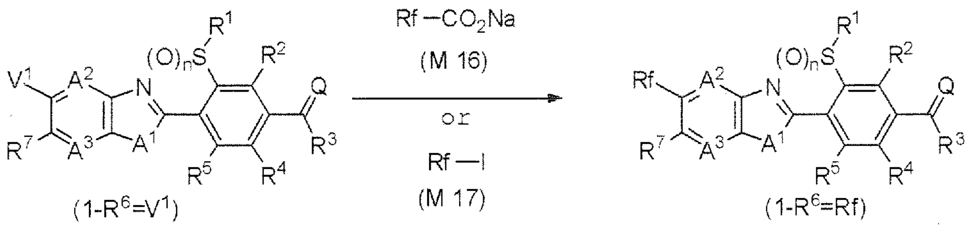


(1-$R^6$=$V^1$) (1-$R^6$=Rf)

In the formula, Rf represents a C1 to C6 perfluoroalkyl group, and other symbols represent the same meaning as described above.

The reaction is usually carried out in a solvent. Examples of the solvent include aromatic hydrocarbons such as toluene and xylene, aprotic polar solvents such as DMF, NMP and DMSO, and mixtures thereof.

Examples of the copper compound include copper (I) iodide. When the compound (M16) is used in the reaction, the compound (M16) is usually used in a ratio of 1 to 10 mol, and copper or the copper compound is usually used in a ratio of 0.5 to 10 mol, based on 1 mol of the compound of the present invention (1-$R^6$=$V^1$), and

the reaction temperature is usually within the range of 100 to 200°C, and the reaction time is usually within the range of 0.5 to 48 hours.

When the compound (M17) is used in the reaction, potassium fluoride may be added. The compound (M17) is usually used in a ratio of 1 to 10 mol, copper or the copper compound is usually used in a ratio of 0.1 to 10 mol, and potassium fluoride is usually used in a ratio of 0.1 to 5 mol, based on 1 mol of the compound of the present invention (1-$R^6$=$V^1$). The reaction temperature is usually within the range of 0 to 150°C, and the reaction time is usually within the range of 0.5 to 48 hours.

After completion of the reaction, the reaction mixture is subjected to post-treatment operations, for example, the reaction mixture is extracted with an organic solvent, and the organic layer is subjected to drying and concentration,

whereby the compound of the present invention (1-$R^6$=Rf) can be isolated. The isolated compound of the present invention (1-$R^6$=Rf) also can be further purified by chromatography, recrystallization, or the like.

In the reaction, $V^1$ is preferably a bromine atom or an iodine atom.

(Production Method 11)

The compound of the present invention (1-$R^6$=SH) wherein $R^6$ is SH in the formula (1) can be produced by reacting the compound of the present invention (1-$R^6$=$V^1$) with a sulfurizing agent. Also, intermediate compound (M18) that is the disulfide body thereof can be produced by oxidizing the compound of the present invention (1-$R^6$=SH).

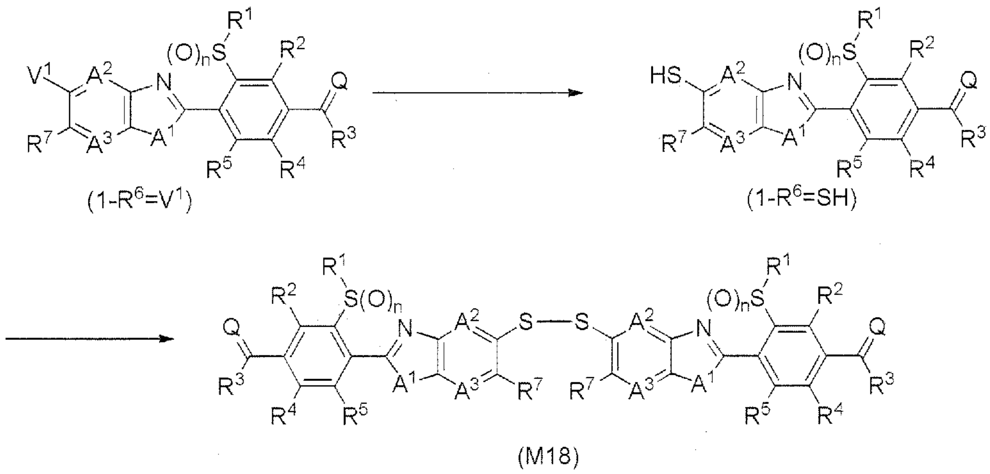

(1-$R^6$=$V^1$)

(1-$R^6$=SH)

(M18)

In the formula, symbols represent the same meaning as described above.

(Production Method 11-1)

The compound of the present invention (1-$R^6$=SH) can be produced by reacting the compound of the present invention (1-$R^6$=$V^1$) with a sulfurizing agent, in the presence of a catalyst.

The reaction is usually carried out in a solvent. Examples of the solvent include aromatic hydrocarbons such as toluene and xylene, aprotic polar solvents such as DMF, NMP and DMSO, and mixtures thereof.

Examples of the sulfurizing agent include sodium sulfide, sodium sulfide nonahydrate, and thiourea.

Examples of the catalyst include copper(I) chloride, copper(I) bromide, and copper(I) iodide.

The reaction can be also carried out by adding a ligand as necessary. Examples of the ligand include acetylacetone, salen, and phenanthroline.

The reaction also can be carried out in the presence of a base, as necessary. Examples of the base include inorganic bases such as potassium carbonate, cesium carbonate and tripotassium phosphate, and organic bases such as triethylamine.

In the reaction, the sulfurizing agent is usually used in a ratio of 1 to 10 mol, the catalyst is usually used in a ratio of 0.1 to 5 mol, the ligand is usually used in a ratio of 0.1 to 5 mol, and the base is usually used in a ratio of 1 to 2 mol, based on 1 mol of the compound of the present invention (1-$R^6$=$V^1$).

The reaction temperature is usually within the range of 50 to 200°C, and the reaction time is usually within the range of 0.5 to 24 hours.

After completion of the reaction, the reaction mixture is subjected to post-treatment operations, for example, the reaction mixture is extracted with an organic solvent, and the organic layer is subjected to drying and concentration, whereby the compound of the present invention (1-$R^6$=SH) can be isolated. The isolated compound of the present invention (1-$R^6$=SH) also can be further purified by chromatography, recrystallization, or the like.

In the reaction, $V^1$ is preferably a bromine atom or an iodine atom.

Here, the reaction from the compound of the present invention (1-$R^6$=SH) to the intermediate compound (M18) is likely to occur, and the intermediate compound (M18) is produced during synthesis of the compound of the present invention (1-$R^6$=SH) in some cases.

(Production Method 11-2)

The intermediate compound (M18) can be produced by reacting the compound of the present invention (1-$R^6$=SH) with an oxidizing agent.

The reaction is usually carried out in a solvent. Examples of the solvent include water, alcohols such as methanol and ethanol, ethers such as THF, ethylene glycol dimethyl ether, tert-butyl methyl ether and 1, 4-dioxane, aromatic hydrocarbons such as toluene and xylene, nitriles such as acetonitrile, aprotic polar solvents such as DMF, NMP and DMSO, carboxylic acids such as acetic acid, and mixtures thereof.

Examples of the oxidizing agent include oxygen, iodine, aqueous hydrogen peroxide, and potassium ferricyanide.

In the reaction, the oxidizing agent is usually used in a ratio of 0.5 to 10 mol, based on 1 mol of the compound of the present invention (1-$R^6$=SH).

The reaction temperature is usually within the range of 0 to 100°C, and the reaction time is usually within the range of 0.1 to 24 hours.

After completion of the reaction, the reaction mixture is subjected to post-treatment operations, for example, the reaction mixture is extracted with an organic solvent, and the organic layer is subjected to drying and concentration, whereby the intermediate compound (M18) can be isolated. The isolated intermediate compound (M18) also can be further purified by chromatography, recrystallization, or the like.

(Production Method 12)

The compound of the present invention (1-$R^6$=SH) can be produced by reacting the compound of the present invention (1-$R^6$=$V^1$) with a thiobenzoic acid to produce intermediate compound (M19), and then hydrolyzing the obtained intermediate compound (M19).

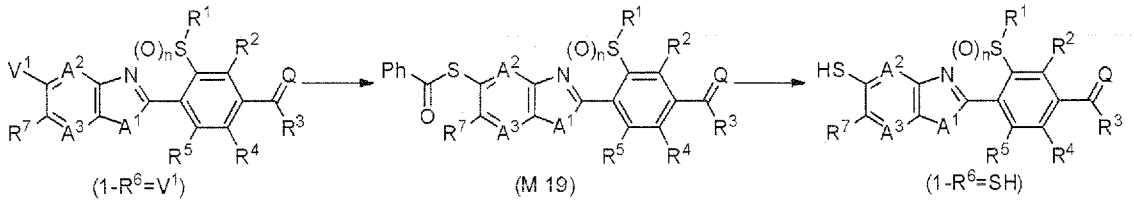

(1-$R^6$=$V^1$) (M 19) (1-$R^6$=SH)

In the formula, symbols represent the same meaning as described above.

(Production Method 12-1)

The intermediate compound (M19) can be produced by reacting the compound of the present invention (1-$R^6$=$V^1$) with a thiobenzoic acid, in the presence of a base and a catalyst.

The reaction is usually carried out in a solvent. Examples of the solvent include aromatic hydrocarbons such as toluene and xylene, aprotic polar solvents such as DMF, NMP and DMSO, and mixtures thereof.

Examples of the base include inorganic bases such as potassium carbonate, cesium carbonate and tripotassium phosphate, and organic bases such as triethylamine.

Examples of the catalyst include copper(I) chloride, copper(I) bromide, and copper(I) iodide.

The reaction can be also carried out by adding a ligand as necessary. Examples of the ligand include acetylacetone, salen, and phenanthroline.

In the reaction, the thiobenzoic acid is usually used in a ratio of 1 to 10 mol, the base is usually used in a ratio of 1 to 2 mol, the catalyst is usually used in a ratio of 0.1 to 5 mol, and the ligand is usually used in a ratio of 0.1 to 5 mol, based on 1 mol of the compound of the present invention (1-$R^6$=$V^1$).

The reaction temperature is usually within the range of 50 to 200°C, and the reaction time is usually within the range of 0.5 to 24 hours.

After completion of the reaction, the reaction mixture is subjected to post-treatment operations, for example, the reaction mixture is extracted with an organic solvent, and the organic layer is subjected to drying and concentration, whereby the intermediate compound (M19) can be isolated. The isolated intermediate compound (M19) also can be further purified by chromatography, recrystallization, or the like.

In the reaction, $V^1$ is preferably a bromine atom or an iodine atom.

(Production Method 12-2)

The compound of the present invention (1-$R^6$=SH) can be produced by hydrolyzing the intermediate compound (M19).

When the hydrolysis reaction is carried out with an acid, an aqueous solution of the acid is usually used as a solvent. Examples of the acid include mineral acids such as hydrochloric acid, nitric acid, phosphoric acid and sulfuric acid, and carboxylic acids such as acetic acid and trifluoroacetic acid.

In the hydrolysis reaction, the acid is usually used in a ratio of 1 mol or more, based on 1 mol of the intermediate compound (M19).

The reaction temperature is usually within the range of 0 to 100°C, and the reaction time is usually within the range of 0.1 to 24 hours.

After completion of the reaction, the reaction mixture is subjected to post-treatment operations, for example, the reaction mixture is extracted with an organic solvent, and the organic layer is subjected to drying and concentration, whereby the compound of the present invention (1-$R^6$=SH) can be isolated. The isolated compound of the present invention (1-$R^6$=SH) also can be further purified by chromatography, recrystallization, or the like.

When the hydrolysis reaction is carried out with a base, the reaction is usually carried out in a solvent. Examples of the solvent include ethers such as THF, ethylene glycol dimethyl ether, tert-butyl methyl ether and 1,4-dioxane, alcohols such as methanol and ethanol, water, and mixtures thereof. Examples of the base include alkali metal hydroxides such as sodium hydroxide and potassium hydroxide.

In the hydrolysis reaction, the base is usually used in a ratio of 1 to 10 mol, based on 1 mol of the intermediate compound (M19).

The reaction temperature is usually within the range of 0 to 120°C, and the reaction time is usually within the range of 0.1 to 24 hours.

After completion of the reaction, the reaction mixture is subjected to post-treatment operations, for example, the reaction liquid is acidified, then the reaction mixture is extracted with an organic solvent, and the organic layer is subjected to drying and concentration, whereby the compound of the present invention (1-$R^6$=SH) can be isolated. The isolated compound of the present invention (1-$R^6$=SH) also can be further purified by chromatography, recrystallization, or the like.

(Production Method 13)

The compound of the present invention (1-$R^6$=$SR^{11d}$) wherein $R^6$ is $S(O)_mR^{11d}$, and m is 0, can be produced by reacting the compound of the present invention (1-$R^6$=SH) or the intermediate compound (M18) that is a disulfide body thereof with compound (M20). The compound of the present invention (1-$R^6$=$S(O)_mR^{11d}$) wherein $R^6$ is -$S(O)_nR^{11d}$, and m is 1 or 2 in the formula (1)., can be produced by oxidizing the compound of the present invention (1-$R^6$=$SR^{11d}$).

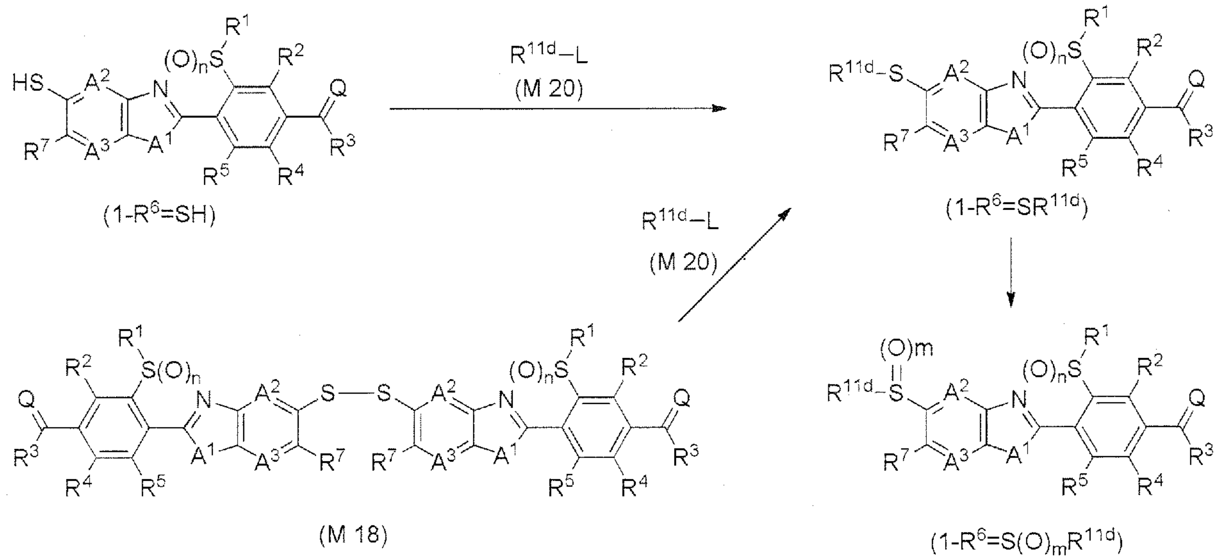


In the formula, $R^{11d}$ represents a C1 to C6 chain hydrocarbon group optionally having one or more atoms or groups selected from group $\alpha$, and other symbols represent the same meaning as described above.

(Production Method 13-1)

The compound of the present invention (1-$R^6$=$SR^{11d}$) wherein $R^6$ is $S(O)_mR^{11d}$, and m is 0, can be produced by reacting the compound of the present invention (1-$R^6$=SH) or the intermediate compound (M18) that is a disulfide body thereof with the compound (M20).

The reaction is usually carried out in a solvent. Examples of the solvent include ethers such as THF, ethylene glycol dimethyl ether, tert-butyl methyl ether and 1, 4-dioxane, aromatic hydrocarbons such as toluene and xylene, nitriles such as acetonitrile, aprotic polar solvents such as DMF, NMP and DMSO, and mixtures thereof.

When the compound of the present invention (1-$R^6$=SH) is used, the reaction is carried out in the presence of a base. Examples of the base include hydrides of alkali metals and alkaline earth metals such as sodium hydride, potassium hydride and calcium hydride, inorganic bases such as sodium carbonate and potassium carbonate, and organic bases such as triethylamine.

In this case, the compound (M20) is usually used in a ratio of 1 to 10 mol, and the base is usually used in a ratio of 1 to 10 mol, based on 1 mol of the compound of the present invention (1-$R^6$=SH).

The reaction temperature is usually within the range of 0 to 100°C, and the reaction time is usually within the range of 0.1 to 24 hours.

After completion of the reaction, the reaction mixture is subjected to post-treatment operations, for example, the reaction mixture is extracted with an organic solvent, and the organic layer is subjected to drying and concentration, whereby the compound of the present invention (1-$R^6$=$SR^{11d}$) can be isolated. The isolated compound of the present invention (1-$R^6$=$SR^{11d}$) also can be further purified by chromatography, recrystallization, or the like.

Also, when the intermediate compound (M18) that is a disulfide body is used, the reaction is carried out in the presence of a reducing agent.

Examples of the reducing agent include sodium hydroxymethanesulfinate (trade name: Rongalite).

When the intermediate compound (M18) that is a disulfide body is used, the compound (M20) is usually used in a ratio of 2 to 10 mol, and the reducing agent is usually used in a ratio of 1 to 5 mol, based on 1 mol of the intermediate compound (M18).

The reaction temperature is usually within the range of -100 to 150°C. The reaction time is usually within the range of 0.1 to 24 hours.

After completion of the reaction, the reaction mixture is subjected to post-treatment operations, for example, the reaction mixture is extracted with an organic solvent, and the organic layer is subjected to drying and concentration, whereby the compound of the present invention (1-$R^6$=$SR^{11d}$) can be isolated. The isolated compound of the present invention (1-$R^6$=$SR^{11d}$) also can be further purified by chromatography, recrystallization, or the like.

Among the compounds of the.present invention (1-$R^6$=$SR^{11d}$) wherein m is 0, the compound of the present invention (1-$R^6$=$SR^f$) wherein $R^{11d}$ is a C1 to C6 perfluoroalkyl group can be produced by reacting the intermediate compound (M18), C1 to C6 perfluoroalkyl iodide and a reducing agent.

The reaction is usually carried out in a solvent. Examples of the solvent include ethers such as THF, ethylene glycol dimethyl ether, tert-butyl methyl ether and 1, 4-dioxane, aromatic hydrocarbons such as toluene and xylene, nitriles such as acetonitrile, aprotic polar solvents such as DMF, NMP and DMSO, and mixtures thereof.

Examples of the C1 to C6 perfluoroalkyl iodide include trifluoromethane iodide, pentafluoroethane iodide, and heptafluoro-2-iodopropane.

Examples of the reducing agent include tetrakis(dimethylamino)ethylene, hydrazine, and hydrazine monohydride.

In the reaction, C1 to C6 perfluoroalkyl iodide is usually used in a ratio of 2 to 10 mol, and the reducing agent is used in a ratio of 1 to 5 mol, based on 1 mol of the intermediate compound (M18).

The reaction temperature is usually within the range of -80 to 50°C. The reaction time is usually within the range of 0.1 to 24 hours.

After completion of the reaction, the reaction mixture is subjected to post-treatment operations, for example, the reaction mixture is extracted with an organic solvent, and the organic layer is subjected to drying and concentration, whereby the compound of the present invention (1-$R^6$=$SR^f$) can be isolated. The isolated compound of the present invention (1-$R^6$=$SR^f$) also can be further purified by chromatography, recrystallization, or the like.

(Production Method 13-2)

The compound of the present invention (1-$R^6$=$(O)_mR^{11d}$) wherein m is 1 or 2 can be produced by reacting the compound of the present invention (1-$R^6$=$SR^{11d}$) wherein m is 0 with an oxidizing agent.

The reaction is usually carried out in a solvent. Examples of the solvent include aliphatic halogenated hydrocarbons such as dichloromethane and chloroform, alcohols such as methanol and ethanol, acetic acid, water, and mixtures thereof.

Examples of the oxidizing agent include m-chloroperbenzoic acid and aqueous hydrogen peroxide.

The reaction also can be carried out in the presence of a catalyst, as necessary. Examples of the catalyst include sodium tungstate.

In the reaction, the oxidizing agent is usually used in a ratio of 1 to 5 mol, and the catalyst is usually used in a ratio of 0.01 to 0.5 mol, based on 1 mol of the compound of the present invention (1-$R^6$=$SR^{11d}$).

In the compound of the present invention (1-$R^6$=(O)$R^{11d}$) wherein m is 1, the oxidizing agent is usually used in a ratio of 0.8 to 1.2 mol, and the catalyst is usually used in a ratio of 0.05 to 0.2 mol, based on 1 mol of the compound of the present invention (1-$R^6$=$SR^{11d}$), and in the compound of the present invention (1-$R^6$=$(O)_2R^{11d}$) wherein m is 2, the oxidizing agent is usually used in a ratio of 1.8 to 5 mol, and the catalyst is usually used in a ratio of 0.05 to 0.2 mol, based on 1 mol of the compound of the present invention (1-$R^6$=$SR^{11d}$).

The reaction temperature is usually within the range of -20 to 120°C. The reaction time is usually within the range of 0.1 to 12 hours.

After completion of the reaction, the reaction mixture is subjected to post-treatment operations, for example, the reaction mixture is extracted with an organic solvent, and the organic layer is washed with an aqueous solution of a reducing agent (for example, sodium sulfite, sodium thiosulfate) and an aqueous solution of a base (for example, sodium bicarbonate) as necessary, and subjected to drying and concentration, whereby the compound of the present invention (1-$R^6$=$(O)_mR^{11d}$) wherein m is 1 or 2 can be isolated. The isolated compound of the present invention (1-$R^6$=$(O)_mR^{11d}$) wherein m is 1 or 2 also can be further purified by chromatography, recrystallization, or the like.

(Production Method 14)

The compound of the present invention (1-J2-$R^{3c}$=H) wherein $R^3$ is group J2, and $R^{3d}$ is a hydrogen atom, in the formula (1), can be produced by hydrolyzing intermediate compound (M21).

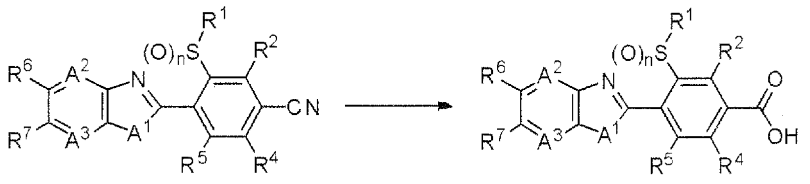

(M 21) (1-J2-$R^{3d}$=H)

In the formula, symbols represent the same meaning as in the formula (1).

The intermediate compound (M21) can be synthesized, using a compound wherein C(Q)$R^3$ is substituted with a cyano group, for example, in accordance with the method of Production Methods 1, 6 and/or 7.

The compound of the present invention (1-J2-$R^{3d}$=H) can be produced by hydrolyzing the intermediate compound (M21).

When the hydrolysis reaction is carried out with an acid, an aqueous solution of the acid is usually used as a solvent. Examples of the acid include mineral acids such as hydrochloric acid, nitric acid, phosphoric acid and sulfuric acid, and carboxylic acids such as acetic acid and trifluoroacetic acid.

In the hydrolysis reaction, the acid is usually used in a ratio of 1 mol or more, based on 1 mol of the intermediate compound (M21).

The reaction temperature is usually within the range of 0 to 150°C, and the reaction time is usually within the range of 0.1 to 24 hours.

After completion of the reaction, the reaction mixture is subjected to post-treatment operations, for example, the reaction mixture is extracted with an organic solvent, and the organic layer is subjected to drying and concentration, or water is added to the reaction mixture and the precipitated crystal is obtained by filtration, whereby the compound of the present invention (1-J2-$R^{3d}$=H) can be isolated. The isolated compound of the present invention (1-J2-$R^{3d}$=H) also can be further purified by chromatography, recrystallization, or the like.

When the hydrolysis reaction is carried out with a base, the reaction is usually carried out in a solvent. Examples of the solvent include ethers such as THF, ethylene glycol dimethyl ether, tert-butyl methyl ether and 1,4-dioxane, alcohols such as methanol and ethanol, water, and mixtures thereof.

Examples of the base include alkali metal hydroxides such as sodium hydroxide and potassium hydroxide.

In the hydrolysis reaction, the base is usually used in a ratio of 1 to 10 mol, based on 1 mol of the intermediate compound (M21) .

The reaction temperature is usually within the range of 0 to 150°C, and the reaction time is usually within the range of 0.1 to 24 hours.

After completion of the reaction, the reaction mixture is subjected to post-treatment operations, for example,

the reaction liquid is acidified, then the reaction mixture is extracted with an organic solvent, and the organic layer is subjected to drying and concentration, whereby the compound of the present invention (1-J2-$R^{3d}$=H) can be isolated. The isolated compound of the present invention (1-J2-$R^{3d}$=H) also can be further purified by chromatography, recrystallization, or the like.

(Production Method 15)

The compound of the present invention (1-J2-$R^{3da}$) wherein $R^3$ is group J2, and $R^{3d}$ is $R^{3da}$, in the formula (1), can be produced by reacting the compound of the present invention (1-J2-$R^{3d}$=H) with compound (M22) in the presence of a condensing agent. In addition, the compound of the present invention (1-J2-$R^{3da}$) can be produced by reacting the intermediate compound (1-J2-$R^{3d}$=H) in the presence of a chlorinating agent to produce intermediate compound (M23), and then reacting the intermediate compound (M23) with the compound (M22).

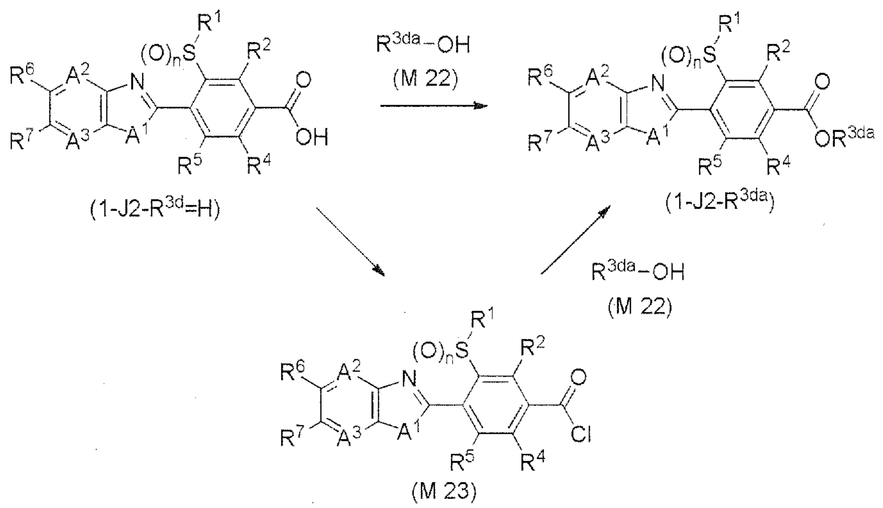


In the formula, $R^{3da}$ is a C1 to C6 chain hydrocarbon group optionally having one or more atoms or groups selected from group β, a C3 to C9 cycloalkyl group optionally having one or more halogen atoms or one or more C1 to C3 alkyl groups, a phenyl group optionally having one or more atoms or groups selected from group γ or a 5- or 6-membered heterocyclic group optionally having one or more atoms or groups selected from group γ, and other symbols represent the same meaning as described above.

(Production Method 15-1)

The compound of the present invention (1-J2-$R^{3da}$) can be produced by reacting the compound of the present invention (1-J2-$R^{3d}$=H) with the compound (M22) in the presence of a condensing agent.

The reaction is usually carried out in a solvent.

Examples of the solvent include ethers such as 1,4-dioxane, diethyl ether, THF and tert-butyl methyl ether, halogenated hydrocarbons such as dichloromethane, chloroform, carbon tetrachloride, 1,2-dichloroethane and chlorobenzene, aromatic hydrocarbons such as toluene, benzene and xylene, esters such as ethyl acetate and butyl acetate, nitriles such as acetonitrile, aprotic polar solvents such as DMF, NMP, 1,3-dimethyl-2-imidazolidinone and DMSO, nitrogen-containing aromatic compounds such as pyridine and quinoline, and mixtures thereof.

Examples of the condensing agent include carbodiimides such as EDAC and 1,3-dicyclohexylcarbodiimide.

The reaction also can be carried out in the presence of a catalyst, as necessary. Examples of the catalyst include HOBt.

In the reaction, the compound (M22) is usually used in a ratio of 0.5 to 2 mol, the condensing agent is usually used in a ratio of 1 to 5 mol, and the catalyst is usually used in a ratio of 0.01 to 1 mol, based on 1 mol of the compound of the present invention (1-J2-$R^{3d}$=H).

The reaction temperature is usually within the range of 0 to 120°C, and the reaction time is usually within the range of 0.1 to 24 hours.

After completion of the reaction, the compound of the present invention (1-J2-$R^{3da}$) can be isolated by adding the reaction mixture to water, then extracting the mixture with an organic.solvent, and concentrating the organic layer; collecting by filtration a solid generated by adding the reaction mixture to water; or collecting by filtration a solid generated

in the reaction mixture. The isolated compound of the present invention (1-J2-$R^{3da}$) also can be further purified by recrystallization, chromatography, or the like.

(Production Method 15-2)

The intermediate compound (M23) can be produced by reacting the compound of the present invention (1-J2-$R^{3d}$=H) with a chlorinating agent.
The reaction is usually carried out in a solvent. Examples of the solvent include ethers such as THF, ethylene glycol dimethyl ether, tert-butyl methyl ether and 1,4-dioxane, aromatic hydrocarbons such as toluene and xylene, aliphatic halogenated hydrocarbons such as dichloromethane and chloroform, and mixtures thereof.
Examples of the chlorinating agent include thionyl chloride, oxalyl dichloride and phosphorus oxychloride.
In the reaction, the chlorinating agent is usually used in a ratio of 1 to 5 mol, based on 1 mol of the compound of the present invention (1-J2-$R^{3d}$=H).
The reaction temperature is usually within the range of 0 to 100°C, and the reaction time is usually within the range of 0.1 to 24 hours.
After completion of the reaction, the intermediate compound (M23) can be isolated by distilling the solvent.
The compound of the present invention (1-J2-$R^{3da}$) can be produced by reacting the intermediate compound (M23) with the compound (M22).
The reaction is usually carried out in a solvent. Examples of the solvent include ethers such as THF, ethylene glycol dimethyl ether, tert-butyl methyl ether and 1, 4-dioxane, aliphatic hydrocarbons such as hexane, heptane and octane, aromatic hydrocarbons such as toluene and xylene, halogenated hydrocarbons such as chlorobenzene, esters such as ethyl acetate and butyl acetate, nitriles such as acetonitrile, aprotic polar solvents such as DMF, NMP and DMSO, and mixtures thereof.
The reaction also can be carried out in the presence of a base, as necessary. The base includes alkali metal carbonates such as sodium carbonate and potassium carbonate, tertiary amines such as triethylamine and N,N-diisopropylethylamine, and nitrogen-containing aromatic compounds such as pyridine and 4-dimethylaminopyridine, and the like.
In the reaction, the compound (M22) is usually used in a ratio of 1 to 3 mol, and the base is usually used in a ratio of 1 to 10 mol, based on 1 mol of the intermediate compound (M23).
The reaction temperature is usually within the range of -20 to 100°C. The reaction time is usually within the range of 0.1 to 24 hours.
After completion of the reaction, the reaction mixture is subjected to post-treatment operations, for example, water is added to the reaction mixture, then the mixture is extracted with an organic solvent, and the organic layer is subjected to drying and concentration, whereby the compound of the present invention (1-J2-$R^{3da}$) can be isolated. The isolated compound of the present invention (1-J2-$R^{3da}$) also can be further purified by chromatography, recrystallization, or the like.

(Production Method 16)

The compound of the present invention (1-J3) wherein $R^3$ is group J3 in the formula (1) can be produced by reacting the compound of the present invention (1-J2-$R^{3d}$=H) with compound (M24). Also, the compound of the present invention (1-J3) can be produced by reacting the intermediate compound (M23) with the compound (M24).

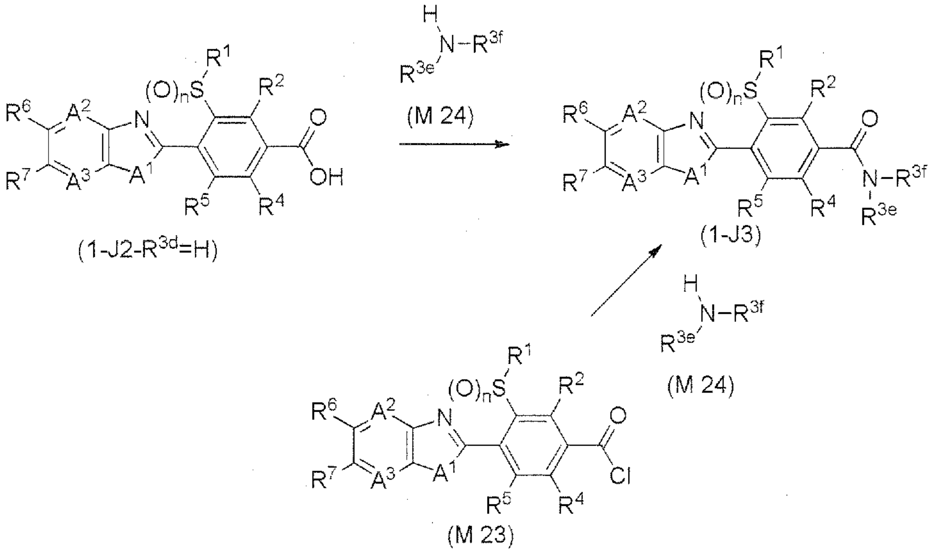


(1-J2-$R^{3d}$=H) (1-J3) (M 23)

In the formula, symbols represent the same meaning as in the formula (1).

The compound of the present invention (1-J3) wherein $R^3$ is group J3 can be produced, using the compound (M24) in place of the compound (M22), in accordance with the method of Production Method 15-1.

The compound of the present invention (1-J3) wherein $R^3$ is group J3 can be produced, using the compound (M24) in place of the compound (M22), in accordance with the method of Production Method 15-2.

(Production Method 17)

The compound of the present invention (1-J1) wherein $R^3$ is group J1 in the formula (1) can be produced by reacting the compound of the present invention (1-J2) or the compound of the present invention (1-J3-$N(CH_3)OCH_3$) with compound (M25).

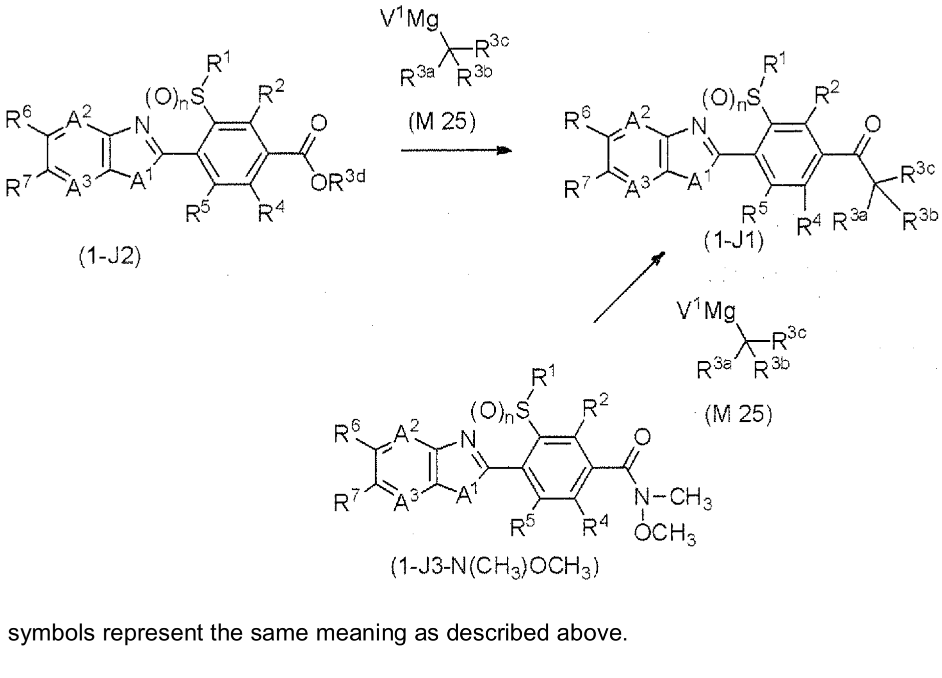


(1-J2) (1-J1) (1-J3-$N(CH_3)OCH_3$)

In the formula, symbols represent the same meaning as described above.

(Production Method 17-1)

The compound of the present invention (1-J1) can be produced by reacting the compound of the present invention (1-J2) with the compound (M25).

The reaction is usually carried out in a solvent. Examples of the solvent include ethers such as THF, ethylene

glycol dimethyl ether, tert-butyl methyl ether and 1,4-dioxane, aromatic hydrocarbons such as toluene and xylene, nitriles such as acetonitrile, aprotic polar solvents such as DMF, NMP and DMSO, and mixtures thereof.

In the reaction, the compound (M25) is usually used in a ratio of 1 to 1.5 mol, based on 1 mol of the compound of the present invention (1-J2).

The reaction temperature is usually within the range of -80 to 100°C. The reaction time is usually within the range of 0.1 to 24 hours.

After completion of the reaction, the reaction mixture is subjected to post-treatment operations, for example, the reaction mixture is extracted with an organic solvent, and the organic layer is subjected to drying and concentration, whereby the compound of the present invention (1-J1) can be isolated. The isolated compound of the present invention (1-J1) also can be further purified by chromatography, recrystallization, or the like.

(Production Method 17-2)

The compound of the present invention (1-J1) can be produced by reacting the compound of the present invention (1-J3-N($CH_3$)$OCH_3$) with the compound (M25).

The compound of the present invention (1-J1) can be produced, using the compound of the present invention (1-J3-N($CH_3$)$OCH_3$) in place of the compound of the present invention (1-J2), in accordance with the method of Production Method 17-1.

(Production Method 18)

Intermediate compound (M2-J1) wherein $R^3$ is group J1 in the intermediate compound (M2) can be produced by hydrolyzing intermediate compound (M26-J1).

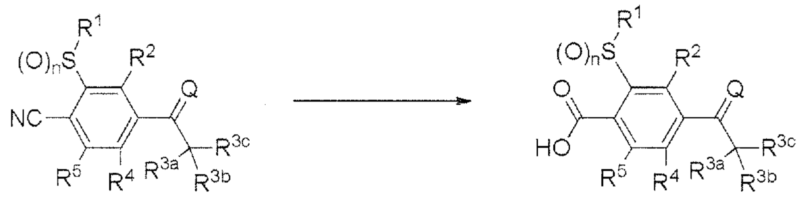

(M 26-J1) (M 2-J1)

In the formula, symbols represent the same meaning as in the formula (1).

When the hydrolysis reaction is carried out with an acid, an aqueous solution of the acid is usually used as a solvent. Examples of the acid include mineral acids such as hydrochloric acid, nitric acid, phosphoric acid and sulfuric acid, and carboxylic acids such as acetic acid and trifluoroacetic acid.

In the hydrolysis reaction, the acid is usually used in a ratio of 1 mol or more, based on 1 mol of the intermediate compound (M26-J1).

The reaction temperature is usually within the range of 0 to 100°C, and the reaction time is usually within the range of 0.1 to 24 hours.

After completion of the reaction, the reaction mixture is subjected to post-treatment operations, for example, the reaction mixture is extracted with an organic solvent, and the organic layer is subjected to drying and concentration, whereby the intermediate compound (M2-J1) can be isolated. The isolated intermediate compound (M2-J1) also can be further purified by chromatography, recrystallization, or the like.

When the hydrolysis reaction is carried out with a base, the reaction is usually carried out in a solvent. Examples of the solvent include ethers such as THF, ethylene glycol dimethyl ether, tert-butyl methyl ether and 1,4-dioxane, alcohols such as methanol and ethanol, water, and mixtures thereof. Examples of the base include alkali metal hydroxides such as sodium hydroxide and potassium hydroxide.

In the hydrolysis reaction, the base is usually used in a ratio of 1 to 10 mol, based on 1 mol of the intermediate compound (M26-J1).

The reaction temperature is usually within the range of 0 to 120°C, and the reaction time is usually within the range of 0.1 to 24 hours.

After completion of the reaction, the reaction mixture is subjected to post-treatment operations, for example, the reaction liquid is acidified, then the reaction mixture is extracted with an organic solvent, and the organic layer is subjected to drying and concentration, whereby the intermediate compound (M2-J1) can be isolated. The isolated intermediate compound (M2-J1) also can be further purified by chromatography, recrystallization, or the like.

(Production Method 19)

The intermediate compound (M4) can be produced by reacting the intermediate compound (M2) with a chlorinating agent.

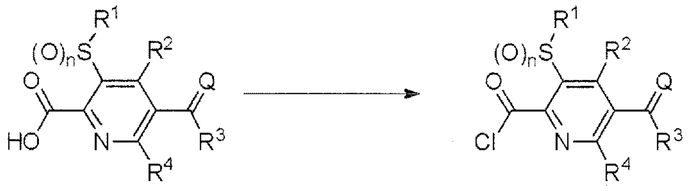

(M 2) (M 4)

In the formula, symbols represent the same meaning as in the formula (1).
The reaction is usually carried out in a solvent. Examples of the solvent include ethers such as THF, ethylene glycol dimethyl ether, tert-butyl methyl ether and 1, 4-dioxane, aromatic hydrocarbons such as toluene and xylene, aliphatic halogenated hydrocarbons such as dichloromethane and chloroform, and mixtures thereof.
Examples of the chlorinating agent include thionyl chloride, oxalyl dichloride and phosphorus oxychloride.
In the reaction, the chlorinating agent is usually used in a ratio of 1 to 5 mol, based on 1 mol of the intermediate compound (M2).
The reaction temperature is usually within the range of 0 to 100°C, and the reaction time is usually within the range of 0.1 to 24 hours.
After completion of the reaction, the intermediate compound (M4) can be isolated by distilling the solvent.

(Production Method 20)

In the present invention, the intermediate compound (M1) can be synthesized according to the method described in WO2012/086848.
Next, specific examples of the compound of the present invention are shown below.
In formula (1-10),

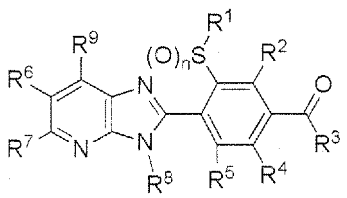

(1-1O)

the compounds of the present invention wherein $R^8$ is a methyl group, $R^3$ is a methyl group, $R^2$, $R^4$, $R^5$ and $R^9$ are a hydrogen atom, and $R^1$, $R^6$, $R^7$ and n are as in the combinations shown in [Table 1] to [Table 24];

[Table 1]

| $R^1$ | $R^6$ | $R^7$ | n |
|---|---|---|---|
| Me | $CF_3$ | H | 0 |
| Et | $CF_3$ | H | 0 |
| Pr | $CF_3$ | H | 0 |
| *i*Pr | $CF_3$ | H | 0 |
| t-Bu | $CF_3$ | H | 0 |
| $CF_3$ | $CF_3$ | H | 0 |
| $CF_2CF_3$ | $CF_3$ | H | 0 |
| $CH=CH_2$ | $CF_3$ | H | 0 |

(continued)

| $R^1$ | $R^6$ | $R^7$ | n |
|---|---|---|---|
| $CH_2CH=CH_2$ | $CF_3$ | H | 0 |
| $C\equiv CH$ | $CF_3$ | H | 0 |
| $CH_2C\equiv CH$ | $CF_3$ | H | 0 |
| CycPr | $CF_3$ | H | 0 |
| $CH_2$CycPr | $CF_3$ | H | 0 |
| Me | $CF_2CF_3$ | H | 0 |
| Et | $CF_2CF_3$ | H | 0 |
| Pr | $CF_2CF_3$ | H | 0 |
| *i*Pr | $CF_2CF_3$ | H | 0 |
| t-Bu | $CF_2CF_3$ | H | 0 |
| $CF_3$ | $CF_2CF_3$ | H | 0 |
| $CF_2CF_3$ | $CF_2CF_3$ | H | 0 |
| $CH=CH_2$ | $CF_2CF_3$ | H | 0 |
| $CH_2CH=CH_2$ | $CF_2CF_3$ | H | 0 |
| $C\equiv CH$ | $CF_2CF_3$ | H | 0 |
| $CH_2C\equiv CH$ | $CF_2CF_3$ | H | 0 |
| CycPr | $CF_2CF_3$ | H | 0 |
| $CH_2$CycPr | $CF_2CF_3$ | H | 0 |

[Table 2]

| $R^1$ | $R^6$ | $R^7$ | n |
|---|---|---|---|
| Me | $CF(CF_3)_2$ | H | 0 |
| Et | $CF(CF_3)_2$ | H | 0 |
| Pr | $CF(CF_3)_2$ | H | 0 |
| *i*Pr | $CF(CF_3)_2$ | H | 0 |
| t-Bu | $CF(CF_3)_2$ | H | 0 |
| $CF_3$ | $CF(CF_3)_2$ | H | 0 |
| $CF_2CF_3$ | $CF(CF_3)_2$ | H | 0 |
| $CH=CH_2$ | $CF(CF_3)_2$ | H | 0 |
| $CH_2CH=CH_2$ | $CF(CF_3)_2$ | H | 0 |
| $C=CH$ | $CF(CF_3)_2$ | H | 0 |
| $CH_2C\equiv CH$ | $CF(CF_3)_2$ | H | 0 |
| CycPr | $CF(CF_3)_2$ | H | 0 |
| $CH_2$CycPr | $CF(CF_3)_2$ | H | 0 |
| Me | $OCF_3$ | H | 0 |
| Et | $OCF_3$ | H | 0 |
| Pr | $OCF_3$ | H | 0 |

(continued)

| $R^1$ | $R^6$ | $R^7$ | n |
|---|---|---|---|
| *i*Pr | $OCF_3$ | H | 0 |
| t-Bu | $OCF_3$ | H | 0 |
| $CF_3$ | $OCF_3$ | H | 0 |
| $CF_2CF_3$ | $OCF_3$ | H | 0 |
| $CH=CH_2$ | $OCF_3$ | H | 0 |
| $CH_2CH=CH_2$ | $OCF_3$ | H | 0 |
| $C\equiv CH$ | $OCF_3$ | H | 0 |
| $CH_2C\equiv CH$ | $OCF_3$ | H | 0 |
| CycPr | $OCF_3$ | H | 0 |
| $CH_2$CycPr | $OCF_3$ | H | 0 |

[Table 3]

| $R^1$ | $R^6$ | $R^7$ | n |
|---|---|---|---|
| Me | $SCF_3$ | H | 0 |
| Et | $SCF_3$ | H | 0 |
| Pr | $SCF_3$ | H | 0 |
| *i*Pr | $SCF_3$ | H | 0 |
| t-Bu | $SCF_3$ | H | 0 |
| $CF_3$ | $SCF_3$ | H | 0 |
| $CF_2CF_3$ | $SCF_3$ | H | 0 |
| $CH=CH_2$ | $SCF_3$ | H | 0 |
| $CH_2CH=CH_2$ | $SCF_3$ | H | 0 |
| $C\equiv CH$ | $SCF_3$ | H | 0 |
| $CH_2C\equiv CH$ | $SCF_3$ | H | 0 |
| CycPr | $SCF_3$ | H | 0 |
| $CH_2$CycPr | $SCF_3$ | H | 0 |
| Me | $S(O)CF_3$ | H | 0 |
| Et | $S(O)CF_3$ | H | 0 |
| Pr | $S(O)CF_3$ | H | 0 |
| *i*Pr | $S(O)CF_3$ | H | 0 |
| t-Bu | $S(O)CF_3$ | H | 0 |
| $CF_3$ | $S(O)CF_3$ | H | 0 |
| $CF_2CF_3$ | $S(O)CF_3$ | H | 0 |
| $CH=CH_2$ | $S(O)CF_3$ | H | 0 |
| $CH_2CH=CH_2$ | $S(O)CF_3$ | H | 0 |
| $C\equiv CH$ | $S(O)CF_3$ | H | 0 |
| $CH_2C\equiv CH$ | $S(O)CF_3$ | H | 0 |

(continued)

| $R^1$ | $R^6$ | $R^7$ | n |
|---|---|---|---|
| CycPr | $S(O)CF_3$ | H | 0 |
| $CH_2CycPr$ | $S(O)CF_3$ | H | 0 |

[Table 4]

| $R^1$ | $R^6$ | $R^7$ | n |
|---|---|---|---|
| Me | $S(O)_2CF_3$ | H | 0 |
| Et | $S(O)_2CF_3$ | H | 0 |
| Pr | $S(O)_2CF_3$ | H | 0 |
| *i*Pr | $S(O)_2CF_3$ | H | 0 |
| t-Bu | $S(O)_2CF_3$ | H | 0 |
| $CF_3$ | $S(O)_2CF_3$ | H | 0 |
| $CF_2CF_3$ | $S(O)_2CF_3$ | H | 0 |
| $CH=CH_2$ | $S(O)_2CF_3$ | H | 0 |
| $CH_2CH=CH_2$ | $S(O)_2CF_3$ | H | 0 |
| $C\equiv CH$ | $S(O)_2CF_3$ | H | 0 |
| $CH_2C\equiv CH$ | $S(O)2CF_3$ | H | 0 |
| CycPr | $S(O)_2CF_3$ | H | 0 |
| $CH_2CycPr$ | $S(O)_2CF_3$ | H | 0 |
| Me | $CF_3$ | H | 1 |
| Et | $CF_3$ | H | 1 |
| Pr | $CF_3$ | H | 1 |
| *i*Pr | $CF_3$ | H | 1 |
| t-Bu | $CF_3$ | H | 1 |
| $CF_3$ | $CF_3$ | H | 1 |
| $CF_2CF_3$ | $CF_3$ | H | 1 |
| $CH=CH_2$ | $CF_3$ | H | 1 |
| $CH_2CH=CH_2$ | $CF_3$ | H | 1 |
| $C=CH$ | $CF_3$ | H | 1 |
| $CH_2C\equiv CH$ | $CF_3$ | H | 1 |
| CycPr | $CF_3$ | H | 1 |
| $CH_2CycPr$ | $CF_3$ | H | 1 |

[Table 5]

| $R^1$ | $R^6$ | $R^7$ | n |
|---|---|---|---|
| Me | $CF_2CF_3$ | H | 1 |
| Et | $CF_2CF_3$ | H | 1 |
| Pr | $CF_2CF_3$ | H | 1 |

(continued)

| $R^1$ | $R^6$ | $R^7$ | n |
|---|---|---|---|
| *i*Pr | $CF_2CF_3$ | H | 1 |
| t-Bu | $CF_2CF_3$ | H | 1 |
| $CF_3$ | $CF_2CF_3$ | H | 1 |
| $CF_2CF_3$ | $CF_2CF_3$ | H | 1 |
| $CH{=}CH_2$ | $CF_2CF_3$ | H | 1 |
| $CH_2CH{=}CH_2$ | $CF_2CF_3$ | H | 1 |
| $C{\equiv}CH$ | $CF_2CF_3$ | H | 1 |
| $CH_2C{\equiv}CH$ | $CF_2CF_3$ | H | 1 |
| CycPr | $CF_2CF_3$ | H | 1 |
| $CH_2$CycPr | $CF_2CF_3$ | H | 1 |
| Me | $CF(CF_3)_2$ | H | 1 |
| Et | $CF(CF_3)_2$ | H | 1 |
| Pr | $CF(CF_3)_2$ | H | 1 |
| *i*Pr | $CF(CF_3)_2$ | H | 1 |
| t-Bu | $CF(CF_3)_2$ | H | 1 |
| $CF_3$ | $CF(CF_3)_2$ | H | 1 |
| $CF_2CF_3$ | $CF(CF_3)_2$ | H | 1 |
| $CH{=}CH_2$ | $CF(CF_3)_2$ | H | 1 |
| $CH_2CH{=}CH_2$ | $CF(CF_3)_2$ | H | 1 |
| $C{\equiv}CH$ | $CF(CF_3)_2$ | H | 1 |
| $CH_2C{\equiv}CH$ | $CF(CF_3)_2$ | H | 1 |
| CycPr | $CF(CF_3)_2$ | H | 1 |
| $CH_2$CycPr | $CF(CF_3)_2$ | H | 1 |

[Table 6]

| $R^1$ | $R^6$ | $R^7$ | n |
|---|---|---|---|
| Me | $OCF_3$ | H | 1 |
| Et | $OCF_3$ | H | 1 |
| Pr | $OCF_3$ | H | 1 |
| *i*Pr | $OCF_3$ | H | 1 |
| t-Bu | $OCF_3$ | H | 1 |
| $CF_3$ | $OCF_3$ | H | 1 |
| $CF_2CF_3$ | $OCF_3$ | H | 1 |
| $CH{=}CH_2$ | $OCF_3$ | H | 1 |
| $CH_2CH{=}CH_2$ | $OCF_3$ | H | 1 |
| $C{\equiv}CH$ | $OCF_3$ | H | 1 |
| $CH_2C{\equiv}CH$ | $OCF_3$ | H | 1 |

(continued)

| $R^1$ | $R^6$ | $R^7$ | n |
|---|---|---|---|
| CycPr | $OCF_3$ | H | 1 |
| $CH_2$CycPr | $OCF_3$ | H | 1 |
| Me | $SCF_3$ | H | 1 |
| Et | $SCF_3$ | H | 1 |
| Pr | $SCF_3$ | H | 1 |
| *i*Pr | $SCF_3$ | H | 1 |
| t-Bu | $SCF_3$ | H | 1 |
| $CF_3$ | $SCF_3$ | H | 1 |
| $CF_2CF_3$ | $SCF_3$ | H | 1 |
| $CH=CH_2$ | $SCF_3$ | H | 1 |
| $CH_2CH=CH_2$ | $SCF_3$ | H | 1 |
| $C=CH$ | $SCF_3$ | H | 1 |
| $CH_2C\equiv CH$ | $SCF_3$ | H | 1 |
| CycPr | $SCF_3$ | H | 1 |
| $CH_2$CycPr | $SCF_3$ | H | 1 |

[Table 7]

| $R^1$ | $R^6$ | $R^7$ | n |
|---|---|---|---|
| Me | $S(O)CF_3$ | H | 1 |
| Et | $S(O)CF_3$ | H | 1 |
| Pr | $S(O)CF_3$ | H | 1 |
| *i*Pr | $S(O)CF_3$ | H | 1 |
| t-Bu | $S(O)CF_3$ | H | 1 |
| $CF_3$ | $S(O)CF_3$ | H | 1 |
| $CF_2CF_3$ | $S(O)CF_3$ | H | 1 |
| $CH=CH_2$ | $S(O)CF_3$ | H | 1 |
| $CH_2CH=CH_2$ | $S(O)CF_3$ | H | 1 |
| $C\equiv CH$ | $S(O)CF_3$ | H | 1 |
| $CH_2C\equiv CH$ | $S(O)CF_3$ | H | 1 |
| CycPr | $S(O)CF_3$ | H | 1 |
| $CH_2$CycPr | $S(O)CF_3$ | H | 1 |
| Me | $S(O)_2CF_3$ | H | 1 |
| Et | $S(O)_2CF_3$ | H | 1 |
| Pr | $S(O)_2CF_3$ | H | 1 |
| *i*Pr | $S(O)_2CF_3$ | H | 1 |
| t-Bu | $S(O)_2CF_3$ | H | 1 |
| $CF_3$ | $S(O)_2CF_3$ | H | 1 |

(continued)

| $R^1$ | $R^6$ | $R^7$ | n |
|---|---|---|---|
| $CF_2CF_3$ | $S(O)_2CF_3$ | H | 1 |
| $CH=CH_2$ | $S(O)_2CF_3$ | H | 1 |
| $CH_2CH=CH_2$ | $S(O)_2CF_3$ | H | 1 |
| $C\equiv CH$ | $S(O)_2CF_3$ | H | 1 |
| $CH_2C\equiv CH$ | $S(O)_2CF_3$ | H | 1 |
| CycPr | $S(O)_2CF_3$ | H | 1 |
| $CH_2$CycPr | $S(O)_2CF_3$ | H | 1 |

[Table 8]

| $R^1$ | $R^6$ | $R^7$ | n |
|---|---|---|---|
| Me | $CF_3$ | H | 2 |
| Et | $CF_3$ | H | 2 |
| Pr | $CF_3$ | H | 2 |
| *i*Pr | $CF_3$ | H | 2 |
| t-Bu | $CF_3$ | H | 2 |
| $CF_3$ | $CF_3$ | H | 2 |
| $CF_2CF_3$ | $CF_3$ | H | 2 |
| $CH=CH_2$ | $CF_3$ | H | 2 |
| $CH_2CH=CH_2$ | $CF_3$ | H | 2 |
| $C=CH$ | $CF_3$ | H | 2 |
| $CH_2C\equiv CH$ | $CF_3$ | H | 2 |
| CycPr | $CF_3$ | H | 2 |
| $CH_2$CycPr | $CF_3$ | H | 2 |
| Me | $CF_2CF_3$ | H | 2 |
| Et | $CF_2CF_3$ | H | 2 |
| Pr | $CF_2CF_3$ | H | 2 |
| *i*Pr | $CF_2CF_3$ | H | 2 |
| t-Bu | $CF_2CF_3$ | H | 2 |
| $CF_3$ | $CF_2CF_3$ | H | 2 |
| $CF_2CF_3$ | $CF_2CF_3$ | H | 2 |
| $CH=CH_2$ | $CF_2CF_3$ | H | 2 |
| $CH_2CH=CH_2$ | $CF_2CF_3$ | H | 2 |
| $C\equiv CH$ | $CF_2CF_3$ | H | 2 |
| $CH_2C\equiv CH$ | $CF_2CF_3$ | H | 2 |
| CycPr | $CF_2CF_3$ | H | 2 |
| $CH_2$CycPr | $CF_2CF_3$ | H | 2 |

[Table 9]

| $R^1$ | $R^6$ | $R^7$ | n |
|---|---|---|---|
| Me | $CF(CF_3)_2$ | H | 2 |
| Et | $CF(CF_3)_2$ | H | 2 |
| Pr | $CF(CF_3)_2$ | H | 2 |
| *i*Pr | $CF(CF_3)_2$ | H | 2 |
| t-Bu | $CF(CF_3)_2$ | H | 2 |
| $CF_3$ | $CF(CF_3)_2$ | H | 2 |
| $CF_2CF_3$ | $CF(CF_3)_2$ | H | 2 |
| $CH=CH_2$ | $CF(CF_3)_2$ | H | 2 |
| $CH_2CH=CH_2$ | $CF(CF_3)_2$ | H | 2 |
| C=CH | $CF(CF_3)_2$ | H | 2 |
| $CH_2C\equiv CH$ | $CF(CF_3)_2$ | H | 2 |
| CycPr | $CF(CF_3)_2$ | H | 2 |
| $CH_2$CycPr | $CF(CF_3)_2$ | H | 2 |
| Me | $OCF_3$ | H | 2 |
| Et | $OCF_3$ | H | 2 |
| Pr | $OCF_3$ | H | 2 |
| *i*Pr | $OCF_3$ | H | 2 |
| t-Bu | $OCF_3$ | H | 2 |
| $CF_3$ | $OCF_3$ | H | 2 |
| $CF_2CF_3$ | $OCF_3$ | H | 2 |
| $CH=CH_2$ | $OCF_3$ | H | 2 |
| $CH_2CH=CH_2$ | $OCF_3$ | H | 2 |
| $C\equiv CH$ | $OCF_3$ | H | 2 |
| $CH_2C\equiv CH$ | $OCF_3$ | H | 2 |
| CycPr | $OCF_3$ | H | 2 |
| $CH_2$CycPr | $OCF_3$ | H | 2 |

[Table 10]

| $R^1$ | $R^6$ | $R^7$ | n |
|---|---|---|---|
| Me | $SCF_3$ | H | 2 |
| Et | $SCF_3$ | H | 2 |
| Pr | $SCF_3$ | H | 2 |
| *i*Pr | $SCF_3$ | H | 2 |
| t-Bu | $SCF_3$ | H | 2 |
| $CF_3$ | $SCF_3$ | H | 2 |
| $CF_2CF_3$ | $SCF_3$ | H | 2 |
| $CH=CH_2$ | $SCF_3$ | H | 2 |

(continued)

| $R^1$ | $R^6$ | $R^7$ | n |
|---|---|---|---|
| $CH_2CH=CH_2$ | $SCF_3$ | H | 2 |
| C≡CH | $SCF_3$ | H | 2 |
| $CH_2C≡CH$ | $SCF_3$ | H | 2 |
| CycPr | $SCF_3$ | H | 2 |
| $CH_2$CycPr | $SCF_3$ | H | 2 |
| Me | $S(O)CF_3$ | H | 2 |
| Et | $S(O)CF_3$ | H | 2 |
| Pr | $S(O)CF_3$ | H | 2 |
| *i*Pr | $S(O)CF_3$ | H | 2 |
| t-Bu | $S(O)CF_3$ | H | 2 |
| $CF_3$ | $S(O)CF_3$ | H | 2 |
| $CF_2CF_3$ | $S(O)CF_3$ | H | 2 |
| $CH=CH_2$ | $S(O)CF_3$ | H | 2 |
| $CH_2CH=CH_2$ | $S(O)CF_3$ | H | 2 |
| C≡CH | $S(O)CF_3$ | H | 2 |
| $CH_2C≡CH$ | $S(O)CF_3$ | H | 2 |
| CycPr | $S(O)CF_3$ | H | 2 |
| $CH_2$CycPr | $S(O)CF_3$ | H | 2 |

[Table 11]

| $R^1$ | $R^6$ | $R^7$ | n |
|---|---|---|---|
| Me | $S(O)_2CF_3$ | H | 2 |
| Et | $S(O)_2CF_3$ | H | 2 |
| Pr | $S(O)_2CF_3$ | H | 2 |
| *i*Pr | $S(O)_2CF_3$ | H | 2 |
| t-Bu | $S(O)_2CF_3$ | H | 2 |
| $CF_3$ | $S(O)_2CF_3$ | H | 2 |
| $CF_2CF_3$ | $S(O)_2CF_3$ | H | 2 |
| $CH=CH_2$ | $S(O)_2CF_3$ | H | 2 |
| $CH_2CH=CH_2$ | $S(O)_2CF_3$ | H | 2 |
| C≡CH | $S(O)_2CF_3$ | H | 2 |
| $CH_2C≡CH$ | $S(O)_2CF_3$ | H | 2 |
| CycPr | $S(O)_2CF_3$ | H | 2 |
| $CH_2$CycPr | $S(O)_2CF_3$ | H | 2 |

[Table 12]

| $R^1$ | $R^6$ | $R^7$ | n |
|---|---|---|---|
| Me | H | $CF_3$ | 0 |
| Et | H | $CF_3$ | 0 |
| Pr | H | $CF_3$ | 0 |
| *i*Pr | H | $CF_3$ | 0 |
| t-Bu | H | $CF_3$ | 0 |
| $CF_3$ | H | $CF_3$ | 0 |
| $CF_2CF_3$ | H | $CF_3$ | 0 |
| $CH=CH_2$ | H | $CF_3$ | 0 |
| $CH_2CH=CH_2$ | H | $CF_3$ | 0 |
| C=CH | H | $CF_3$ | 0 |
| $CH_2C\equiv CH$ | H | $CF_3$ | 0 |
| CycPr | H | $CF_3$ | 0 |
| $CH_2$CycPr | H | $CF_3$ | 0 |
| Me | H | $CF_2CF_3$ | 0 |
| Et | H | $CF_2CF_3$ | 0 |
| Pr | H | $CF_2CF_3$ | 0 |
| *i*Pr | H | $CF_2CF_3$ | 0 |
| t-Bu | H | $CF_2CF_3$ | 0 |
| $CF_3$ | H | $CF_2CF_3$ | 0 |
| $CF_2CF_3$ | H | $CF_2CF_3$ | 0 |
| $CH=CH_2$ | H | $CF_2CF_3$ | 0 |
| $CH_2CH=CH_2$ | H | $CF_2CF_3$ | 0 |
| $C\equiv CH$ | H | $CF_2CF_3$ | 0 |
| $CH_2C\equiv CH$ | H | $CF_2CF_3$ | 0 |
| CycPr | H | $CF_2CF_3$ | 0 |
| $CH_2$CycPr | H | $CF_2CF_3$ | 0 |

[Table 13]

| $R^1$ | $R^6$ | $R^7$ | n |
|---|---|---|---|
| Me | H | $CF(CF_3)_2$ | 0 |
| Et | H | $CF(CF_3)_2$ | 0 |
| Pr | H | $CF(CF_3)_2$ | 0 |
| *i*Pr | H | $CF(CF_3)_2$ | 0 |
| t-Bu | H | $CF(CF_3)_2$ | 0 |
| $CF_3$ | H | $CF(CF_3)_2$ | 0 |
| $CF_2CF_3$ | H | $CF(CF_3)_2$ | 0 |
| $CH=CH_2$ | H | $CF(CF_3)_2$ | 0 |
| $CH_2CH=CH_2$ | H | $CF(CF_3)_2$ | 0 |

(continued)

| $R^1$ | $R^6$ | $R^7$ | n |
|---|---|---|---|
| C≡CH | H | $CF(CF_3)_2$ | 0 |
| $CH_2C≡CH$ | H | $CF(CF_3)_2$ | 0 |
| CycPr | H | $CF(CF_3)_2$ | 0 |
| $CH_2CycPr$ | H | $CF(CF_3)_2$ | 0 |
| Me | H | $OCF_3$ | 0 |
| Et | H | $OCF_3$ | 0 |
| Pr | H | $OCF_3$ | 0 |
| *i*Pr | H | $OCF_3$ | 0 |
| t-Bu | H | $OCF_3$ | 0 |
| $CF_3$ | H | $OCF_3$ | 0 |
| $CF_2CF_3$ | H | $OCF_3$ | 0 |
| $CH=CH_2$ | H | $OCF_3$ | 0 |
| $CH_2CH=CH_2$ | H | $OCF_3$ | 0 |
| C≡CH | H | $OCF_3$ | 0 |
| $CH_2C≡CH$ | H | $OCF_3$ | 0 |
| CycPr | H | $OCF_3$ | 0 |
| $CH_2CycPr$ | H | $OCF_3$ | 0 |

[Table 14]

| $R^1$ | $R^6$ | $R^7$ | n |
|---|---|---|---|
| Me | H | $SCF_3$ | 0 |
| Et | H | $SCF_3$ | 0 |
| Pr | H | $SCF_3$ | 0 |
| *i*Pr | H | $SCF_3$ | 0 |
| t-Bu | H | $SCF_3$ | 0 |
| $CF_3$ | H | $SCF_3$ | 0 |
| $CF_2CF_3$ | H | $SCF_3$ | 0 |
| $CH=CH_2$ | H | $SCF_3$ | 0 |
| $CH_2CH=CH_2$ | H | $SCF_3$ | 0 |
| C≡CH | H | $SCF_3$ | 0 |
| $CH_2C≡CH$ | H | $SCF_3$ | 0 |
| CycPr | H | $SCF_3$ | 0 |
| $CH_2CycPr$ | H | $SCF_3$ | 0 |
| Me | H | $S(O)CF_3$ | 0 |
| Et | H | $S(O)CF_3$ | 0 |
| Pr | H | $S(O)CF_3$ | 0 |
| *i*Pr | H | $S(O)CF_3$ | 0 |

(continued)

| $R^1$ | $R^6$ | $R^7$ | n |
|---|---|---|---|
| t-Bu | H | $S(O)CF_3$ | 0 |
| $CF_3$ | H | $S(O)CF_3$ | 0 |
| $CF_2CF_3$ | H | $S(O)CF_3$ | 0 |
| $CH=CH_2$ | H | $S(O)CF_3$ | 0 |
| $CH_2CH=CH_2$ | H | $S(O)CF_3$ | 0 |
| $C\equiv CH$ | H | $S(O)CF_3$ | 0 |
| $CH_2C\equiv CH$ | H | $S(O)CF_3$ | 0 |
| CycPr | H | $S(O)CF_3$ | 0 |
| $CH_2$CycPr | H | $S(O)CF_3$ | 0 |

[Table 15]

| $R^1$ | $R^6$ | $R^7$ | n |
|---|---|---|---|
| Me | H | $S(O)_2CF_3$ | 0 |
| Et | H | $S(O)_2CF_3$ | 0 |
| Pr | H | $S(O)_2CF_3$ | 0 |
| *i*Pr | H | $S(O)_2CF_3$ | 0 |
| t-Bu | H | $S(O)_2CF_3$ | 0 |
| $CF_3$ | H | $S(O)_2CF_3$ | 0 |
| $CF_2CF_3$ | H | $S(O)_2CF_3$ | 0 |
| $CH=CH_2$ | H | $S(O)_2CF_3$ | 0 |
| $CH_2CH=CH_2$ | H | $S(O)_2CF_3$ | 0 |
| $C=CH$ | H | $S(O)_2CF_3$ | 0 |
| $CH_2C\equiv CH$ | H | $S(O)_2CF_3$ | 0 |
| CycPr | H | $S(O)_2CF_3$ | 0 |
| $CH_2$CycPr | H | $S(O)_2CF_3$ | 0 |
| Me | H | $CF_3$ | 1 |
| Et | H | $CF_3$ | 1 |
| Pr | H | $CF_3$ | 1 |
| *i*Pr | H | $CF_3$ | 1 |
| t-Bu | H | $CF_3$ | 1 |
| $CF_3$ | H | $CF_3$ | 1 |
| $CF_2CF_3$ | H | $CF_3$ | 1 |
| $CH=CH_2$ | H | $CF_3$ | 1 |
| $CH_2CH=CH_2$ | H | $CF_3$ | 1 |
| $C\equiv CH$ | H | $CF_3$ | 1 |
| $CH_2C\equiv CH$ | H | $CF_3$ | 1 |
| CycPr | H | $CF_3$ | 1 |

(continued)

| | | | |
|---|---|---|---|
| $CH_2CycPr$ | H | $S(O)_2CF_3$ | 0 |
| $CH_2CycPr$ | H | $CF_3$ | 1 |

[Table 16]

| $R^1$ | $R^6$ | $R^7$ | n |
|---|---|---|---|
| Me | H | $CF_2CF_3$ | 1 |
| Et | H | $CF_2CF_3$ | 1 |
| Pr | H | $CF_2CF_3$ | 1 |
| *i*Pr | H | $CF_2CF_3$ | 1 |
| t-Bu | H | $CF_2CF_3$ | 1 |
| $CF_3$ | H | $CF_2CF_3$ | 1 |
| $CF_2CF_3$ | H | $CF_2CF_3$ | 1 |
| $CH=CH_2$ | H | $CF_2CF_3$ | 1 |
| $CH_2CH=CH_2$ | H | $CF_2CF_3$ | 1 |
| $C\equiv CH$ | H | $CF_2CF_3$ | 1 |
| $CH_2C\equiv CH$ | H | $CF_2CF_3$ | 1 |
| CycPr | H | $CF_2CF_3$ | 1 |
| $CH_2CycPr$ | H | $CF_2CF_3$ | 1 |
| Me | H | $CF(CF_3)_2$ | 1 |
| Et | H | $CF(CF_3)_2$ | 1 |
| Pr | H | $CF(CF_3)_2$ | 1 |
| *i*Pr | H | $CF(CF_3)_2$ | 1 |
| t-Bu | H | $CF(CF_3)_2$ | 1 |
| $CF_3$ | H | $CF(CF_3)_2$ | 1 |
| $CF_2CF_3$ | H | $CF(CF_3)_2$ | 1 |
| $CH=CH_2$ | H | $CF(CF_3)_2$ | 1 |
| $CH_2CH=CH_2$ | H | $CF(CF_3)_2$ | 1 |
| $C\equiv CH$ | H | $CF(CF_3)_2$ | 1 |
| $CH_2C\equiv CH$ | H | $CF(CF_3)_2$ | 1 |
| CycPr | H | $CF(CF_3)_2$ | 1 |
| $CH_2CycPr$ | H | $CF(CF_3)_2$ | 1 |

[Table 17]

| $R^1$ | $R^6$ | $R^7$ | n |
|---|---|---|---|
| Me | H | $OCF_3$ | 1 |
| Et | H | $OCF_3$ | 1 |
| Pr | H | $OCF_3$ | 1 |
| *i*Pr | H | $OCF_3$ | 1 |

(continued)

| $R^1$ | $R^6$ | $R^7$ | n |
|---|---|---|---|
| t-Bu | H | $OCF_3$ | 1 |
| $CF_3$ | H | $OCF_3$ | 1 |
| $CF_2CF_3$ | H | $OCF_3$ | 1 |
| $CH=CH_2$ | H | $OCF_3$ | 1 |
| $CH_2CH=CH_2$ | H | $OCF_3$ | 1 |
| $C\equiv CH$ | H | $OCF_3$ | 1 |
| $CH_2C\equiv CH$ | H | $OCF_3$ | 1 |
| CycPr | H | $OCF_3$ | 1 |
| $CH_2$CycPr | H | $OCF_3$ | 1 |
| Me | H | $SCF_3$ | 1 |
| Et | H | $SCF_3$ | 1 |
| Pr | H | $SCF_3$ | 1 |
| *i*Pr | H | $SCF_3$ | 1 |
| t-Bu | H | $SCF_3$ | 1 |
| $CF_3$ | H | $SCF_3$ | 1 |
| $CF_2CF_3$ | H | $SCF_3$ | 1 |
| $CH=CH_2$ | H | $SCF_3$ | 1 |
| $CH_2CH=CH_2$ | H | $SCF_3$ | 1 |
| $C\equiv CH$ | H | $SCF_3$ | 1 |
| $CH_2C\equiv CH$ | H | $SCF_3$ | 1 |
| CycPr | H | $SCF_3$ | 1 |
| $CH_2$CycPr | H | $SCF_3$ | 1 |

[Table 18]

| $R^1$ | $R^6$ | $R^7$ | n |
|---|---|---|---|
| Me | H | $S(O)CF_3$ | 1 |
| Et | H | $S(O)CF_3$ | 1 |
| Pr | H | $S(O)CF_3$ | 1 |
| *i*Pr | H | $S(O)CF_3$ | 1 |
| t-Bu | H | $S(O)CF_3$ | 1 |
| $CF_3$ | H | $S(O)CF_3$ | 1 |
| $CF_2CF_3$ | H | $S(O)CF_3$ | 1 |
| $CH=CH_2$ | H | $S(O)CF_3$ | 1 |
| $CH_2CH=CH_2$ | H | $S(O)CF_3$ | 1 |
| $C\equiv CH$ | H | $S(O)CF_3$ | 1 |
| $CH_2C\equiv CH$ | H | $S(O)CF_3$ | 1 |
| CycPr | H | $S(O)CF_3$ | 1 |

(continued)

| $R^1$ | $R^6$ | $R^7$ | n |
|---|---|---|---|
| $CH_2$CycPr | H | $S(O)CF_3$ | 1 |
| Me | H | $S(O)_2CF_3$ | 1 |
| Et | H | $S(O)_2CF_3$ | 1 |
| Pr | H | $S(O)_2CF_3$ | 1 |
| *i*Pr | H | $S(O)_2CF_3$ | 1 |
| t-Bu | H | $S(O)_2CF_3$ | 1 |
| $CF_3$ | H | $S(O)_2CF_3$ | 1 |
| $CF_2CF_3$ | H | $S(O)_2CF_3$ | 1 |
| $CH=CH_2$ | H | $S(O)_2CF_3$ | 1 |
| $CH_2CH=CH_2$ | H | $S(O)_2CF_3$ | 1 |
| $C\equiv CH$ | H | $S(O)_2CF_3$ | 1 |
| $CH_2C\equiv CH$ | H | $S(O)_2CF_3$ | 1 |
| CycPr | H | $S(O)_2CF_3$ | 1 |
| $CH_2$CycPr | H | $S(O)_2CF_3$ | 1 |

[Table 19]

| $R^1$ | $R^6$ | $R^7$ | n |
|---|---|---|---|
| Me | H | $CF_3$ | 2 |
| Et | H | $CF_3$ | 2 |
| Pr | H | $CF_3$ | 2 |
| *i*Pr | H | $CF_3$ | 2 |
| t-Bu | H | $CF_3$ | 2 |
| $CF_3$ | H | $CF_3$ | 2 |
| $CF_2CF_3$ | H | $CF_3$ | 2 |
| $CH=CH_2$ | H | $CF_3$ | 2 |
| $CH_2CH=CH_2$ | H | $CF_3$ | 2 |
| $C\equiv CH$ | H | $CF_3$ | 2 |
| $CH_2C\equiv CH$ | H | $CF_3$ | 2 |
| CycPr | H | $CF_3$ | 2 |
| $CH_2$CycPr | H | $CF_3$ | 2 |
| Me | H | $CF_2CF_3$ | 2 |
| Et | H | $CF_2CF_3$ | 2 |
| Pr | H | $CF_2CF_3$ | 2 |
| *i*Pr | H | $CF_2CF_3$ | 2 |
| t-Bu | H | $CF_2CF_3$ | 2 |
| $CF_3$ | H | $CF_2CF_3$ | 2 |
| $CF_2CF_3$ | H | $CF_2CF_3$ | 2 |

(continued)

| $R^1$ | $R^6$ | $R^7$ | n |
|---|---|---|---|
| $CH{=}CH_2$ | H | $CF_2CF_3$ | 2 |
| $CH_2CH{=}CH_2$ | H | $CF_2CF_3$ | 2 |
| $C{\equiv}CH$ | H | $CF_2CF_3$ | 2 |
| $CH_2C{\equiv}CH$ | H | $CF_2CF_3$ | 2 |
| CycPr | H | $CF_2CF_3$ | 2 |
| $CH_2$CycPr | H | $CF_2CF_3$ | 2 |

[Table 20]

| $R^1$ | $R^6$ | $R^7$ | n |
|---|---|---|---|
| Me | H | $CF(CF_3)_2$ | 2 |
| Et | H | $CF(CF_3)_2$ | 2 |
| Pr | H | $CF(CF_3)_2$ | 2 |
| *i*Pr | H | $CF(CF_3)_2$ | 2 |
| t-Bu | H | $CF(CF_3)_2$ | 2 |
| $CF_3$ | H | $CF(CF_3)_2$ | 2 |
| $CF_2CF_3$ | H | $CF(CF_3)_2$ | 2 |
| $CH{=}CH_2$ | H | $CF(CF_3)_2$ | 2 |
| $CH_2CH{=}CH_2$ | H | $CF(CF_3)_2$ | 2 |
| $C{\equiv}CH$ | H | $CF(CF_3)_2$ | 2 |
| $CH_2C{\equiv}CH$ | H | $CF(CF_3)_2$ | 2 |
| CycPr | H | $CF(CF_3)_2$ | 2 |
| $CH_2$CycPr | H | $CF(CF_3)_2$ | 2 |
| Me | H | $OCF_3$ | 2 |
| Et | H | $OCF_3$ | 2 |
| Pr | H | $OCF_3$ | 2 |
| *i*Pr | H | $OCF_3$ | 2 |
| t-Bu | H | $OCF_3$ | 2 |
| $CF_3$ | H | $OCF_3$ | 2 |
| $CF_2CF_3$ | H | $OCF_3$ | 2 |
| $CH{=}CH_2$ | H | $OCF_3$ | 2 |
| $CH_2CH{=}CH_2$ | H | $OCF_3$ | 2 |
| $C{\equiv}CH$ | H | $OCF_3$ | 2 |
| $CH_2C{\equiv}CH$ | H | $OCF_3$ | 2 |
| CycPr | H | $OCF_3$ | 2 |
| $CH_2$CycPr | H | $OCF_3$ | 2 |

[Table 21]

| $R^1$ | $R^6$ | $R^7$ | n |
|---|---|---|---|
| Me | H | $SCF_3$ | 2 |
| Et | H | $SCF_3$ | 2 |
| Pr | H | $SCF_3$ | 2 |
| *i*Pr | H | $SCF_3$ | 2 |
| t-Bu | H | $SCF_3$ | 2 |
| $CF_3$ | H | $SCF_3$ | 2 |
| $CF_2CF_3$ | H | $SCF_3$ | 2 |
| $CH=CH_2$ | H | $SCF_3$ | 2 |
| $CH_2CH=CH_2$ | H | $SCF_3$ | 2 |
| C≡CH | H | $SCF_3$ | 2 |
| $CH_2C≡CH$ | H | $SCF_3$ | 2 |
| CycPr | H | $SCF_3$ | 2. |
| $CH_2$CycPr | H | $SCF_3$ | 2 |
| Me | H | $S(O)CF_3$ | 2 |
| Et | H | $S(O)CF_3$ | 2 |
| Pr | H | $S(O)CF_3$ | 2 |
| *i*Pr | H | $S(O)CF_3$ | 2 |
| t-Bu | H | $S(O)CF_3$ | 2 |
| $CF_3$ | H | $S(O)CF_3$ | 2 |
| $CF_2CF_3$ | H | $S(O)CF_3$ | 2 |
| $CH=CH_2$ | H | $S(O)CF_3$ | 2 |
| $CH_2CH=CH_2$ | H | $S(O)CF_3$ | 2 |
| C≡CH | H | $S(O)CF_3$ | 2 |
| $CH_2C≡CH$ | H | $S(O)CF_3$ | 2 |
| CycPr | H | $S(O)CF_3$ | 2 |
| $CH_2$CycPr | H | $S(O)CF_3$ | 2 |

[Table 22]

| $R^1$ | $R^6$ | $R^7$ | n |
|---|---|---|---|
| Me | H | $S(O)_2CF_3$ | 2 |
| Et | H | $S(O)_2CF_3$ | 2 |
| Pr | H | $S(O)_2CF_3$ | 2 |
| *i*Pr | H | $S(O)_2CF_3$ | 2 |
| t-Bu | H | $S(O)_2CF_3$ | 2 |
| $CF_3$ | H | $S(O)_2CF_3$ | 2 |
| $CF_2CF_3$ | H | $S(O)_2CF_3$ | 2 |
| $CH=CH_2$ | H | $S(O)_2CF_3$ | 2 |

(continued)

| $R^1$ | $R^6$ | $R^7$ | n |
|---|---|---|---|
| $CH_2CH=CH_2$ | H | $S(O)_2CF_3$ | 2 |
| C≡CH | H | $S(O)_2CF_3$ | 2 |
| $CH_2C≡CH$ | H | $S(O)_2CF_3$ | 2 |
| CycPr | H | $S(O)_2CF_3$ | 2 |
| $CH_2$CycPr | H | $S(O)_2CF_3$ | 2 |

[Table 23]

| $R^1$ | $R^6$ | $R^7$ | n |
|---|---|---|---|
| Me | $CF_3$ | Cl | 0 |
| Et | $CF_3$ | Cl | 0 |
| Pr | $CF_3$ | Cl | 0 |
| *i*Pr | $CF_3$ | Cl | 0 |
| t-Bu | $CF_3$ | Cl | 0 |
| $CF_3$ | $CF_3$ | Cl | 0 |
| $CF_2CF_3$ | $CF_3$ | Cl | 0 |
| $CH=CH_2$ | $CF_3$ | Cl | 0 |
| $CH_2CH=CH_2$ | $CF_3$ | Cl | 0 |
| C≡CH | $CF_3$ | Cl | 0 |
| $CH_2C≡CH$ | $CF_3$ | Cl | 0 |
| CycPr | $CF_3$ | Cl | 0 |
| $CH_2$CycPr | $CF_3$ | Cl | 0 |
| Me | $CF_3$ | Cl | 2 |
| Et | $CF_3$ | Cl | 2 |
| Pr | $CF_3$ | Cl | 2 |
| *i*Pr | $CF_3$ | Cl | 2 |
| t-Bu | $CF_3$ | Cl | 2 |
| $CF_3$ | $CF_3$ | Cl | 2 |
| $CF_2CF_3$ | $CF_3$ | Cl | 2 |
| $CH=CH_2$ | $CF_3$ | Cl | 2 .. |
| $CH_2CH=CH_2$ | $CF_3$ | Cl | 2 |
| C≡CH | $CF_3$ | Cl | 2 |
| $CH_2C≡CH$ | $CF_3$ | Cl | 2 |
| CycPr | $CF_3$ | Cl | 2 |
| $CH_2$CycPr | $CF_3$ | Cl | 2 |

[Table 24]

| $R^1$ | $R^6$ | $R^7$ | n |
|---|---|---|---|
| Me | Cl | $CF_3$ | 0 |
| Et | Cl | $CF_3$ | 0 |
| Pr | Cl | $CF_3$ | 0 |
| *i*Pr | Cl | $CF_3$ | 0 |
| t-Bu | Cl | $CF_3$ | 0 |
| $CF_3$ | Cl | $CF_3$ | 0 |
| $CF_2CF_3$ | Cl | $CF_3$ | 0 |
| $CH{=}CH_2$ | Cl | $CF_3$ | 0 |
| $CH_2CH{=}CH_2$ | Cl | $CF_3$ | 0 |
| $C{\equiv}CH$ | Cl | $CF_3$ | 0 |
| $CH_2C{\equiv}CH$ | Cl | $CF_3$ | 0 |
| CycPr | Cl | $CF_3$ | 0 |
| $CH_2$CycPr | Cl | $CF_3$ | 0 |
| Me | Cl | $CF_3$ | 2 |
| Et | Cl | $CF_3$ | 2 |
| Pr | Cl | $CF_3$ | 2 |
| *i*Pr | Cl | $CF_3$ | 2 |
| t-Bu | Cl | $CF_3$ | 2 |
| $CF_3$ | Cl | $CF_3$ | 2 |
| $CF_2CF_3$ | Cl | $CF_3$ | 2 |
| $CH{=}CH_2$ | Cl | $CF_3$ | 2 |
| $CH_2CH{=}CH_2$ | Cl | $CF_3$ | 2 |
| $C{\equiv}CH$ | Cl | $CF_3$ | 2 |
| $CH_2C{\equiv}CH$ | Cl | $CF_3$ | 2 |
| CycPr | Cl | $CF_3$ | 2 |
| $CH_2$CycPr | Cl | $CF_3$ | 2 |

In [Table 1] to [Table 24] above, Me represents a methyl group, Et represents an ethyl group, Pr represents a propyl group, *i*Pr represents an iso-propyl group, t-Bu represents a tert-butyl group, and CycPr represents a cyclopropyl group.

In the formula (1-10), the compounds of the present invention wherein $R^8$ is a methyl group, $R^3$ is an ethyl group, $R^2$, $R^4$, $R^5$ and $R^9$ are a hydrogen atom, and $R^1$, $R^6$, $R^7$ and n are as in the combinations shown in [Table 1] to [Table 24];

In the formula (1-10), the compounds of the present invention wherein $R^8$ is a methyl group, $R^3$ is a propyl group, $R^2$, $R^4$, $R^5$ and $R^9$ are a hydrogen atom, and $R^1$, $R^6$, $R^7$ and n are as in the combinations shown in [Table 1] to [Table 24];

In the formula (1-10), the compounds of the present invention wherein $R^8$ is a methyl group, $R^3$ is an isopropyl group, $R^2$, $R^4$, $R^5$ and $R^9$ are a hydrogen atom, and $R^1$, $R^6$, $R^7$ and n are as in the combinations shown in [Table 1] to [Table 24];

In the formula (1-10), the compounds of the present invention wherein $R^8$ is a methyl group, $R^3$ is a methoxy group, $R^2$, $R^4$, $R^5$ and $R^9$ are a hydrogen atom, and $R^1$, $R^6$, $R^7$ and n are as in the combinations shown in [Table 1] to [Table 24];

In the formula (1-10), the compounds of the present invention wherein $R^8$ is a methyl group, $R^3$ is an ethoxy group, $R^2$, $R^4$, $R^5$ and $R^9$ are a hydrogen atom, and $R^1$, $R^6$, $R^7$ and n are as in the combinations shown in [Table 1] to [Table 24];

In the formula (1-10), the compounds of the present invention wherein $R^8$ is a methyl group, $R^3$ is a propoxy group, $R^2$, $R^4$, $R^5$ and $R^9$ are a hydrogen atom, and $R^1$, $R^6$, $R^7$ and n are as in the combinations shown in [Table 1] to [Table 24];

In the formula (1-10), the compounds of the present invention wherein $R^8$ is a methyl group, $R^3$ is an amino group, $R^2$, $R^4$, $R^5$ and $R^9$ are a hydrogen atom, and $R^1$, $R^6$, $R^7$ and n are as in the combinations shown in [Table 1] to [Table 24];

In the formula (1-10), the compounds of the present invention wherein $R^8$ is a methyl group, $R^3$ is a methylamino group, $R^2$, $R^4$, $R^5$ and $R^9$ are a hydrogen atom, and $R^1$, $R^6$, $R^7$ and n are as in the combinations shown in [Table 1] to [Table 24];
In the formula (1-10), the compounds of the present invention wherein $R^8$ is a methyl group, $R^3$ is an ethylamino group, $R^2$, $R^4$, $R^5$ and $R^9$ are a hydrogen atom, and $R^1$, $R^6$, $R^7$ and n are as in the combinations shown in [Table 1] to [Table 24];
In the formula (1-10), the compounds of the present invention wherein $R^8$ is a methyl group, $R^3$ is a propylamino group, $R^2$, $R^4$, $R^5$ and $R^9$ are a hydrogen atom, and $R^1$, $R^6$, $R^7$ and n are as in the combinations shown in [Table 1] to [Table 24];
In the formula (1-10), the compounds of the present invention wherein $R^8$ is a methyl group, $R^3$ is a dimethylamino group, $R^2$, $R^4$, $R^5$ and $R^9$ are a hydrogen atom, and $R^1$, $R^6$, $R^7$ and n are as in the combinations shown in [Table 1] to [Table 24];
In the formula (1-10), the compounds of the present invention wherein $R^8$ is a methyl group, $R^3$ is a diethylamino group, $R^2$, $R^4$, $R^5$ and $R^9$ are a hydrogen atom, and $R^1$, $R^6$, $R^7$ and n are as in the combinations shown in [Table 1] to [Table 24];
In the formula (1-10), the compounds of the present invention wherein $R^8$ is a methyl group, $R^3$ is a dipropylamino group, $R^2$, $R^4$, $R^5$ and $R^9$ are a hydrogen atom, and $R^1$, $R^6$, $R^7$ and n are as in the combinations shown in [Table 1] to [Table 24];
In formula (1-1S),

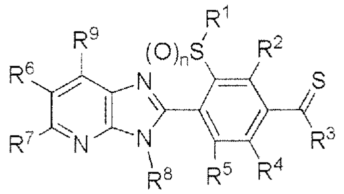
(1-1S)

the compounds of the present invention wherein $R^8$ is a methyl group, $R^3$ is a methyl group, $R^2$, $R^4$, $R^5$ and $R^9$ are a hydrogen atom, and $R^1$, $R^6$, $R^7$ and n are as in the combinations shown in [Table 1] to [Table 24];
In the formula (1-1S), the compounds of the present invention wherein $R^8$ is a methyl group, $R^3$ is an ethyl group, $R^2$, $R^4$, $R^5$ and $R^9$ are a hydrogen atom, and $R^1$, $R^6$, $R^7$ and n are as in the combinations shown in [Table 1] to [Table 24];
In the formula (1-1S), the compounds of the present invention wherein $R^8$ is a methyl group, $R^3$ is a propyl group, $R^2$, $R^4$, $R^5$ and $R^9$ are a hydrogen atom, and $R^1$, $R^6$, $R^7$ and n are as in the combinations shown in [Table 1] to [Table 24];
In the formula (1-1S), the compounds of the present invention wherein $R^8$ is a methyl group, $R^3$ is an isopropyl group, $R^2$, $R^4$, $R^5$ and $R^9$ are a hydrogen atom, and $R^1$, $R^6$, $R^7$ and n are as in the combinations shown in [Table 1] to [Table 24];
In the formula (1-1S), the compounds of the present invention wherein $R^8$ is a methyl group, $R^3$ is a methoxy group, $R^2$, $R^4$, $R^5$ and $R^9$ are a hydrogen atom, and $R^1$, $R^5$, $R^7$ and n are as in the combinations shown in [Table 1] to [Table 24];
In the formula (1-1S), the compounds of the present invention wherein $R^8$ is a methyl group, $R^3$ is an ethoxy group, $R^2$, $R^4$, $R^5$ and $R^9$ are a hydrogen atom, and $R^1$, $R^6$, $R^7$ and n are as in the combinations shown in [Table 1] to [Table 24];
In the formula (1-1S), the compounds of the present invention wherein $R^8$ is a methyl group, $R^3$ is a propoxy group, $R^2$, $R^4$, $R^5$ and $R^9$ are a hydrogen atom, and $R^1$, $R^6$, $R^7$ and n are as in the combinations shown in [Table 1] to [Table 24];
In the formula (1-1S), the compounds of the present invention wherein $R^8$ is a methyl group, $R^3$ is an amino group, $R^2$, $R^4$, $R^5$ and $R^9$ are a hydrogen atom, and $R^1$, $R^6$, $R^7$ and n are as in the combinations shown in [Table 1] to [Table 24];
In the formula (1-1S), the compounds of the present invention wherein $R^8$ is a methyl group, $R^3$ is a methylamino group, $R^2$, $R^4$, $R^5$ and $R^9$ are a hydrogen atom, and $R^1$, $R^6$, $R^7$ and n are as in the combinations shown in [Table 1] to [Table 24];
In the formula (1-1S), the compounds of the present invention wherein $R^8$ is a methyl group, $R^3$ is an ethylamino group, $R^2$, $R^4$, $R^5$ and $R^9$ are a hydrogen atom, and $R^1$, $R^6$, $R^7$ and n are as in the combinations shown in [Table 1] to [Table 24];
In the formula (1-1S), the compounds of the present invention wherein $R^8$ is a methyl group, $R^3$ is a propylamino group, $R^2$, $R^4$, $R^5$ and $R^9$ are a hydrogen atom, and $R^1$, $R^6$, $R^7$ and n are as in the combinations shown in [Table 1] to [Table 24];
In the formula (1-1S), the compounds of the present invention wherein $R^8$ is a methyl group, $R^3$ is a dimethylamino group, $R^2$, $R^4$, $R^5$ and $R^9$ are a hydrogen atom, and $R^1$, $R^6$, $R^7$ and n are as in the combinations shown in [Table 1] to [Table 24];
In the formula (1-1S), the compounds of the present invention wherein $R^8$ is a methyl group, $R^3$ is a diethylamino group, $R^2$, $R^4$, $R^5$ and $R^9$ are a hydrogen atom, and $R^1$, $R^6$, $R^7$ and n are as in the combinations shown in [Table 1] to [Table 24];
In the formula (1-1S), the compounds of the present invention wherein $R^8$ is a methyl group, $R^3$ is a dipropylamino group, $R^2$, $R^4$, $R^3$ and $R^9$ are a hydrogen atom, and $R^1$, $R^6$, $R^7$ and n are as in the combinations shown in [Table 1] to [Table 24];
In formula (1-20),

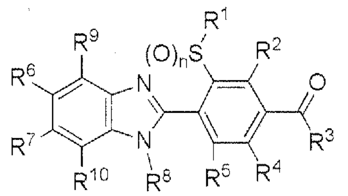 (1-2O)

the compounds of the present invention wherein $R^8$ is a methyl group, $R^3$ is a methyl group, $R^2$, $R^4$, $R^5$, $R^9$ and $R^{10}$ are a hydrogen atom, and $R^1$, $R^6$, $R^7$ and n are as in the combinations shown in [Table 1] to [Table 24];
In the formula (1-20), the.compounds of the present invention wherein $R^8$ is a methyl group, $R^3$ is an ethyl group, $R^2$, $R^4$, $R^5$, $R^9$ and $R^{10}$ are a hydrogen atom, and $R^1$, $R^6$, $R^7$ and n are as in the combinations shown in [Table 1] to [Table 24];
In the formula (1-20), the compounds of the present invention wherein $R^8$ is a methyl group, $R^3$ is a propyl group, $R^2$, $R^4$, $R^5$, $R^9$ and $R^{10}$ are a hydrogen atom, and $R^1$, $R^6$, $R^7$ and n are as in the combinations shown in [Table 1] to [Table 24];
In the formula (1-20), the compounds of the present invention wherein $R^8$ is a methyl group, $R^3$ is an isopropyl group, $R^2$, $R^4$, $R^5$, $R^9$ and $R^{10}$ are a hydrogen atom, and $R^1$, $R^6$, $R^7$ and n are as in the combinations shown in [Table 1] to [Table 24];
In the formula (1-20), the compounds of the present invention wherein $R^8$ is a methyl group, $R^3$ is a methoxy group, $R^2$, $R^4$, $R^5$, $R^9$ and $R^{10}$ are a hydrogen atom, and $R^1$, $R^6$, $R^7$ and n are as in the combinations shown in [Table 1] to [Table 24];
In the formula (1-20), the compounds of the present invention wherein $R^8$ is a methyl group, $R^3$ is an ethoxy group, $R^2$, $R^4$, $R^5$, $R^9$ and $R^{10}$ are a hydrogen atom, and $R^1$, $R^6$, $R^7$ and n are as in the combinations shown in [Table 1] to [Table 24];
In the formula (1-20), the compounds of the present invention wherein $R^8$ is a methyl group, $R^3$ is a propoxy group, $R^2$, $R^4$, $R^5$, $R^9$ and $R^{10}$ are a hydrogen atom, and $R^1$, $R^6$, $R^7$ and n are as in the combinations shown in [Table 1] to [Table 24];
In the formula (1-20), the compounds of the present invention wherein $R^8$ is a methyl group, $R^3$ is an amino group, $R^2$, $R^4$, $R^5$, $R^9$ and $R^{10}$ are a hydrogen atom, and $R^1$, $R^6$, $R^7$ and n are as in the combinations shown in [Table 1] to [Table 24];
In the formula (1-20), the compounds of the present invention wherein $R^8$ is a methyl group, $R^3$ is a methylamino group, $R^2$, $R^4$, $R^5$, $R^9$ and $R^{10}$ are a hydrogen atom, and $R^1$, $R^6$, $R^7$ and n are as in the combinations shown in [Table 1] to [Table 24];
In the formula (1-20), the compounds of the present invention wherein $R^8$ is a methyl group, $R^3$ is an ethylamino group, $R^2$, $R^4$, $R^5$, $R^9$ and $R^{10}$ are a hydrogen atom, and $R^1$, $R^6$, $R^7$ and n are as in the combinations shown in [Table 1] to [Table 24];
In the formula (1-20), the compounds of the present invention wherein $R^8$ is a methyl group, $R^3$ is a propylamino group, $R^2$, $R^4$, $R^5$, $R^9$ and $R^{10}$ are a hydrogen atom, and $R^1$, $R^6$, $R^7$ and n are as in the combinations shown in [Table 1] to [Table 24];
In the formula (1-20), the compounds of the present invention wherein $R^8$ is a methyl group, $R^3$ is a dimethylamino group, $R^2$, $R^4$, $R^5$, $R^9$ and $R^{10}$ are a hydrogen atom, and $R^1$, $R^6$, $R^7$ and n are as in the combinations shown in [Table 1] to [Table 24];
In the formula (1-20), the compounds of the present invention wherein $R^8$ is a methyl group, $R^3$ is a diethylamino group, $R^2$, $R^4$, $R^5$, $R^9$ and $R^{10}$ are a hydrogen atom, and $R^1$, $R^6$, $R^7$ and n are as in the combinations shown in [Table 1] to [Table 24];
In the formula (1-20), the compounds of the present invention wherein $R^8$ is a methyl group, $R^3$ is a dipropylamino group, $R^2$, $R^4$, $R^5$, $R^9$ and $R^{10}$ are a hydrogen atom, and $R^1$, $R^6$, $R^7$ and n are as in the combinations shown in [Table 1] to [Table 24];
In formula (1-2S),

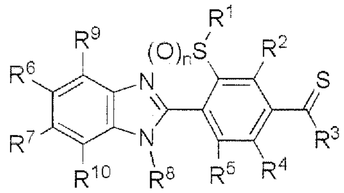 (1-2S)

the compounds of the present invention wherein $R^8$ is a methyl group, $R^3$ is a methyl group, $R^2$, $R^4$, $R^5$, $R^9$ and $R^{10}$ are a hydrogen atom, and $R^1$, $R^6$, $R^7$ and n are as in the combinations shown in [Table 1] to [Table 24];
In the formula (1-2S), the compounds of the present invention wherein $R^8$ is a methyl group, $R^3$ is an ethyl group, $R^2$, $R^4$, $R^5$, $R^9$ and $R^{10}$ are a hydrogen atom, and $R^1$, $R^6$, $R^7$ and n are as in the combinations shown in [Table 1] to [Table 24];
In the formula (1-2S), the compounds of the present invention wherein $R^8$ is a methyl group, $R^3$ is a propyl group, $R^2$,

$R^4$, $R^5$, $R^9$ and $R^{10}$ are a hydrogen atom, and $R^1$, $R^6$, $R^7$ and n are as in the combinations shown in [Table 1] to [Table 24];
In the formula (1-2S), the compounds of the present invention wherein $R^8$ is a methyl group, $R^3$ is an isopropyl group, $R^2$, $R^4$, $R^5$, $R^9$ and $R^{10}$ are a hydrogen atom, and $R^1$, $R^6$, $R^7$ and n are as in the combinations shown in [Table 1] to [Table 24];
In the formula (1-2S), the compounds of the present invention wherein $R^8$ is a methyl group, $R^3$ is a methoxy group, $R^2$, $R^4$, $R^5$, $R^9$ and $R^{10}$ are a hydrogen atom, and $R^1$, $R^6$, $R^7$ and n are as in the combinations shown in [Table 1] to [Table 24];
In the formula (1-2S), the compounds of the present invention wherein $R^8$ is a methyl group, $R^3$ is an ethoxy group, $R^2$, $R^4$, $R^5$, $R^9$ and $R^{10}$ are a hydrogen atom, and $R^1$, $R^6$, $R^7$ and n are as in the combinations shown in [Table 1] to [Table 24];
In the formula (1-2S), the compounds of the present invention wherein $R^8$ is a methyl group, $R^3$ is a propoxy group, $R^2$, $R^4$, $R^5$, $R^9$ and $R^{10}$ are a hydrogen atom, and $R^1$, $R^6$, $R^7$ and n are as in the combinations shown in [Table 1] to [Table 24];
In the formula (1-2S), the compounds of the present invention wherein $R^8$ is a methyl group, $R^3$ is an amino group, $R^2$, $R^4$, $R^5$, $R^9$ and $R^{10}$ are a hydrogen atom, and $R^1$, $R^6$, $R^7$ and n are as in the combinations shown in [Table 1] to [Table 24];
In the formula (1-2S), the compounds of the present invention wherein $R^8$ is a methyl group, $R^3$ is a methylamino group, $R^2$, $R^4$, $R^5$, $R^9$ and $R^{10}$ are a hydrogen atom, and $R^1$, $R^6$, $R^7$ and n are as in the combinations shown in [Table 1] to [Table 24];
In the formula (1-2S), the compounds of the present invention wherein $R^8$ is a methyl group, $R^3$ is an ethylamino group, $R^2$, $R^4$, $R^5$, $R^9$ and $R^{10}$ are a hydrogen atom, and $R^1$, $R^6$, $R^7$ and n are as in the combinations shown in [Table 1] to [Table 24];
In the formula (1-2S), the compounds of the present invention wherein $R^8$ is a methyl group, $R^3$ is a propylamino group, $R^2$, $R^4$, $R^5$, $R^9$ and $R^{10}$ are a hydrogen atom, and $R^1$, $R^6$, $R^7$ and n are as in the combinations shown in [Table 1] to [Table 24]; In the formula (1-2S), the compounds of the present invention wherein $R^8$ is a methyl group, $R^3$ is a dimethylamino group, $R^2$, $R^4$, $R^5$, $R^9$ and $R^{10}$ are a hydrogen atom, and $R^1$, $R^6$, $R^7$ and n are as in the combinations shown in [Table 1] to [Table 24];
In the formula (1-2S), the compounds of the present invention wherein $R^8$ is a methyl group, $R^3$ is a diethylamino group, $R^2$, $R^4$, $R^5$, $R^9$ and $R^{10}$ are a hydrogen atom, and $R^1$, $R^6$, $R^7$ and n are as in the combinations shown in [Table 1] to [Table 24];
In the formula (1-2S), the compounds of the present invention wherein $R^8$ is a methyl group, $R^3$ is a dipropylamino group, $R^2$, $R^4$, $R^5$, $R^9$ and $R^{10}$ are a hydrogen atom, and $R^1$, $R^6$, $R^7$ and n are as in the combinations shown in [Table 1] to [Table 24];
In formula (1-30),

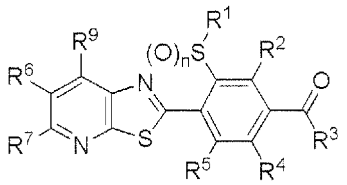

(1-3O)

the compounds of the present invention wherein $R^3$ is a methyl group, $R^2$, $R^4$, $R^5$ and $R^9$ are a hydrogen atom, and $R^1$, $R^6$, $R^7$ and in are as in the combinations shown in [Table 1] to [Table 24];
In the formula (1-30), the compounds of the present invention wherein $R^3$ is an ethyl group, $R^2$, $R^4$, $R^5$ and $R^9$ are a hydrogen atom, and $R^1$, $R^6$, $R^7$ and n are as in the combinations shown in [Table 1] to [Table 24];
In the formula (1-30), the compounds of the present invention wherein $R^3$ is a propyl group, $R^2$, $R^4$, $R^5$ and $R^9$ are a hydrogen atom, and $R^1$, $R^6$, $R^7$ and n are as in the combinations shown in [Table 1] to [Table 24];
In the formula (1-30), the compounds of the present invention wherein $R^3$ is an isopropyl group, $R^2$, $R^4$, $R^5$ and $R^9$ are a hydrogen atom, and $R^1$, $R^6$, $R^7$ and n are as in the combinations shown in [Table 1] to [Table 24];
In the formula (1-30), the compounds of the present invention wherein $R^3$ is a methoxy group, $R^2$, $R^4$, $R^5$ and $R^9$ are a hydrogen atom, and $R^1$, $R^6$, $R^7$ and n are as in the combinations shown in [Table 1] to [Table 24];
In the formula (1-30), the compounds of the present invention wherein $R^3$ is an ethoxy group, $R^2$, $R^4$, $R^5$ and $R^9$ are a hydrogen atom, and $R^1$, $R^6$, $R^7$ and n are as in the combinations shown in [Table 1] to [Table 24];
In the formula (1-30), the compounds of the present invention wherein $R^3$ is a propoxy group, $R^2$, $R^4$, $R^5$ and $R^9$ are a hydrogen atom, and $R^1$, $R^6$, $R^7$ and n are as in the combinations shown in [Table 1] to [Table 24];
In the formula (1-30), the compounds of the present invention wherein $R^3$ is an amino group, $R^2$, $R^4$, $R^5$ and $R^9$ are a hydrogen atom, and $R^1$, $R^6$, $R^7$ and n are as in the combinations shown in [Table 1] to [Table 24];
In the formula (1-30), the compounds of the present invention wherein $R^3$ is a methylamino group, $R^2$, $R^4$, $R^5$ and $R^9$ are a hydrogen atom, and $R^1$, $R^6$, $R^7$ and n are as in the combinations shown in [Table 1] to [Table 24];
In the formula (1-30), the compounds of the present invention wherein $R^3$ is an ethylamino group, $R^2$, $R^4$, $R^5$ and $R^9$ are a hydrogen atom, and $R^1$, $R^6$, $R^7$ and n are as in the combinations shown in [Table 1] to [Table 24];

In the formula (1-30), the compounds of the present invention wherein $R^3$ is a propylamino group, $R^2$, $R^4$, $R^5$ and $R^9$ are a hydrogen atom, and $R^1$, $R^6$, $R^7$ and n are as in the combinations shown in [Table 1] to [Table 24];
In the formula (1-30), the compounds of the present invention wherein $R^3$ is a dimethylamino group, $RR^2$, $R^4$, $R^5$ and $R^9$ are a hydrogen atom, and $R^1$, $R^6$, $R^7$ and n are as in the combinations shown in [Table 1] to [Table 24];
In the formula (1-30), the compounds of the present invention wherein $R^3$ is a diethylamino group, $R^2$, $R^4$, $R^5$ and $R^9$ are a hydrogen atom, and $R^1$, $R^6$, $R^7$ and n are as in the combinations shown in [Table 1] to [Table 24];
In the formula (1-30), the compounds of the present invention wherein $R^3$ is a dipropylamino group, $R^2$, $R^4$, $R^5$ and $R^9$ are a hydrogen atom, and $R^1$, $R^6$, $R^7$ and n are as in the combinations shown in [Table 1] to [Table 24];
In formula (1-3S),

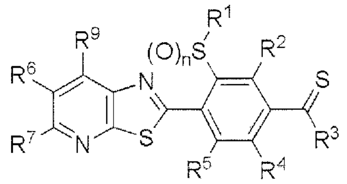
(1-3S)

the compounds of the present invention wherein $R^3$ is a methyl group, $R^2$, $R^4$, $R^5$ and $R^9$ are a hydrogen atom, and $R^1$, $R^6$, $R^7$ and n are as in the combinations shown in [Table 1] to [Table 24]; In the formula (1-3S), the compounds of the present invention wherein $R^3$ is an ethyl group, $R^2$, $R^4$, $R^6$ and $R^9$ are a hydrogen atom, and $R^1$, $R^6$, $R^7$ and n are as in the combinations shown in [Table 1] to [Table 24];
In the formula (1-3S), the compounds of the present invention wherein $R^3$ is a propyl group, $R^2$, $R^4$, $R^5$ and $R^9$ are a hydrogen atom, and $R^1$, $R^6$, $R^7$ and n are as in the combinations shown in [Table 1] to [Table 24];
In the formula (1-3S), the compounds of the present invention wherein $R^3$ is an isopropyl group, $R^2$, $R^4$, $R^5$ and $R^9$ are a hydrogen atom, and $R^1$, $R^6$, $R^7$ and n are as in the combinations shown in [Table 1] to [Table 24];
In the formula (1-3S), the compounds of the present invention wherein $R^3$ is a methoxy group, $R^2$, $R^4$, $R^5$ and $R^9$ are a hydrogen atom, and $R^1$, $R^6$, $R^7$ and n are as in the combinations shown in [Table 1] to [Table 24];
In the formula (1-3S), the compounds of the present invention wherein $R^3$ is an ethoxy group, $R^2$, $R^4$, $R^5$ and $R^9$ are a hydrogen atom, and $R^1$, $R^6$, $R^7$ and n are as in the combinations shown in [Table 1] to [Table 24];
In the formula (1-3S), the compounds of the present invention wherein $R^3$ is a propoxy group, $R^2$, $R^4$, $R^5$ and $R^9$ are a hydrogen .. atom, and $R^1$, $R^6$, $R^7$ and n are as in the combinations shown in [Table 1] to [Table 24];
In the formula (1-3S), the compounds of the present invention wherein $R^3$ is an amino group, $R^2$, $R^4$, $R^5$ and $R^9$ are a hydrogen atom, and $R^1$, $R^6$, $R^7$ and n are as in the combinations shown in [Table 1] to [Table 24];
In the formula (1-3S), the compounds of the present invention wherein $R^3$ is a methyl amino group, $R^2$, $R^4$, $R^5$ and $R^9$ are a hydrogen atom, and $R^1$, $R^6$, $R^7$ and n are as in the combinations shown in [Table 1] to [Table 24];
In the formula (1-3S), the compounds of the present invention wherein $R^3$ is an ethylamino group, $R^2$, $R^4$, $R^5$ and $R^9$ are a hydrogen atom, and $R^1$, $R^6$, $R^7$ and n are as in the combinations shown in [Table 1] to [Table 24];
In the formula (1-3S), the compounds of the present invention wherein $R^3$ is a propylamino group, $R^2$, $R^4$, $R^5$ and $R^9$ are a hydrogen atom, and $R^1$, $R^6$, $R^7$ and n are as in the combinations shown in [Table 1] to [Table 24];
In the formula (1-3S), the compounds of the present invention wherein $R^3$ is a dimethylamino group, $R^2$, $R^4$, $R^5$ and $R^9$ are a hydrogen atom, and $R^1$, $R^6$, $R^7$ and n are as in the combinations shown in [Table 1] to [Table 24];
In the formula (1-3S), the compounds of the present invention wherein $R^3$ is a diethylamino group, $R^2$, $R^4$, $R^5$ and $R^9$ are a hydrogen atom, and $R^1$, $R^6$, $R^7$ and n are as in the combinations shown in [Table 1] to [Table 24];
In the formula (1-3S), the compounds of the present invention wherein $R^3$ is a dipropylamino group, $R^2$, $R^4$, $R^5$ and $R^9$ are a hydrogen atom, and $R^1$, $R^6$, $R^7$ and n are as in the combinations shown in [Table 1] to [Table 24];
In formula (1-40),

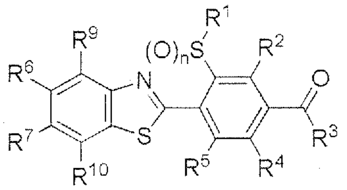
(1-4O)

the compounds of the present invention wherein $R^3$ is a methyl group, $R^2$, $R^4$, $R^5$, $R^9$ and $R^{10}$ are a hydrogen atom, and $R^1$, $R^6$, $R^7$ and n are as in the combinations shown in [Table 1] to [Table 24];
In the formula (1-40), the compounds of the present invention wherein $R^3$ is an ethyl group, $R^2$, $R^4$, $R^5$, $R^9$ and $R^{10}$ are

a hydrogen atom, and $R^1$, $R^6$, $R^7$ and n are as in the combinations shown in [Table 1] to [Table 24];
In the formula (1-40), the compounds of the present invention wherein $R^3$ is a propyl group, $R^2$, $R^4$, $R^5$, $R^9$ and $R^{10}$ are a hydrogen atom, and $R^1$, $R^6$, $R^7$ and n are as in the combinations shown in [Table 1] to [Table 24];
In the formula (1-40), the compounds of the present invention wherein $R^3$ is an isopropyl group, $R^2$, $R^4$, $R^5$, $R^9$ and $R^{10}$ are a hydrogen atom, and $R^1$, $R^6$, $R^7$ and n are as in the combinations shown in [Table 1] to [Table 24];
In the formula (1-40), the compounds of the present invention wherein $R^3$ is a methoxy group, $R^2$, $R^4$, $R^5$, $R^9$ and $R^{10}$ are a hydrogen atom, and $R^1$, $R^6$, $R^7$ and n are as in the combinations shown in [Table 1] to [Table 24];
In the formula (1-40), the compounds of the present invention wherein $R^3$ is an ethoxy group, $R^2$, $R^4$, $R^5$, $R^9$ and $R^{10}$ are a hydrogen atom, and $R^1$, $R^6$, $R^7$ and n are as in the combinations shown in [Table 1] to [Table 24];
In the formula (1-40), the compounds of the present invention wherein $R^3$ is a propoxy group, $R^2$, $R^4$, $R^5$, $R^9$ and $R^{10}$ are a hydrogen atom, and $R^1$, $R^6$, $R^7$ and n are as in the combinations shown in [Table 1] to [Table 24];
In the formula (1-40), the compounds of the present invention wherein $R^3$ is an amino group, $R^2$, $R^4$, $R^5$, $R^9$ and $R^{10}$ are a hydrogen atom, and $R^1$, $R^6$, $R^7$ and n are as in the combinations shown in [Table 1] to [Table 24];
In the formula (1-40), the compounds of the present invention wherein $R^3$ is a methylamino group, $R^2$, $R^4$, $R^5$, $R^9$ and $R^{10}$ are a hydrogen atom, and $R^1$, $R^6$, $R^7$ and n are as in the combinations shown in [Table 1] to [Table 24];
In the formula (1-40), the compounds of the present invention wherein $R^3$ is an ethylamino group, $R^2$, $R^4$, $R^5$, $R^9$ and $R^{10}$ are a hydrogen atom, and $R^1$, $R^6$, $R^7$ and n are as in the combinations shown in [Table 1] to [Table 24];
In the formula (1-40), the compounds of the present invention wherein $R^3$ is a propylamino group, $R^2$, $R^4$, $R^5$, $R^9$ and $R^{10}$ are a hydrogen atom, and $R^1$, $R^6$, $R^7$ and n are as in the combinations shown in [Table 1] to [Table 24];
In the formula (1-40), the compounds of the present invention wherein $R^3$ is a dimethylamino group, $R^2$, $R^4$, $R^5$, $R^9$ and $R^{10}$ are a hydrogen atom, and $R^1$, $R^6$, $R^7$ and n are as in the combinations shown in [Table 1] to [Table 24];
In the formula (1-40), the compounds of the present invention wherein $R^3$ is a diethylamino group, $R^2$, $R^4$, $R^5$, $R^9$ and $R^{10}$ are a hydrogen atom, and $R^1$, $R^6$, $R^7$ and n are as in the combinations shown in [Table 1] to [Table 24];
In the formula (1-40), the compounds of the present invention wherein $R^3$ is a dipropylamino group, $R^2$, $R^4$, $R^5$, $R^9$ and $R^{10}$ are a hydrogen atom, and $R^1$, $R^6$, $R^7$ and n are as in the combinations shown in [Table 1] to [Table 24];
In formula (1-4S),

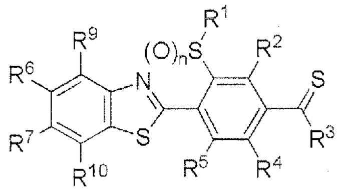

(1-4S)

the compounds of the present invention wherein $R^3$ is a methyl group, $R^2$, $R^4$, $R^5$, $R^9$ and $R^{10}$ are a hydrogen atom, and $R^1$, $R^6$, $R^7$ and n are as in the combinations shown in [Table 1] to [Table 24];
In the formula (1-4S), the compounds of the present invention wherein $R^3$ is an ethyl group, $R^2$, $R^4$, $R^5$, $R^9$ and $R^{10}$ are a hydrogen atom, and $R^1$, $R^6$, $R^7$ and n are as in the combinations shown in [Table 1] to [Table 24];
In the formula (1-4S), the compounds of the present invention wherein $R^3$ is a propyl group, $R^2$, $R^4$, $R^5$, $R^9$ and $R^{10}$ are a hydrogen atom, and $R^1$, $R^6$, $R^7$ and n are as in the combinations shown in [Table 1] to [Table 24];
In the formula (1-4S), the compounds of the present invention wherein $R^3$ is an isopropyl group, $R^2$, $R^4$, $R^5$, $R^9$ and $R^{10}$ are a hydrogen atom, and $R^1$, $R^6$, $R^7$ and n are as in the combinations shown in [Table 1] to [Table 24];
In the formula (1-4S), the compounds of the present invention wherein $R^3$ is a methoxy group, $R^2$, $R^4$, $R^5$, $R^9$ and $R^{10}$ are a hydrogen atom, and $R^1$, $R^6$, $R^7$ and n are as in the combinations shown in [Table 1] to [Table 24];
In the formula (1-4S), the compounds of the present invention wherein $R^3$ is an ethoxy group, $R^2$, $R^4$, $R^5$, $R^9$ and $R^{10}$ are a hydrogen atom, and $R^1$, $R^6$, $R^7$ and n are as in the combinations shown in [Table 1] to [Table 24];
In the formula (1-4S), the compounds of the present invention wherein $R^3$ is a propoxy group, $R^2$, $R^4$, $R^5$, $R^9$ and $R^{10}$ are a hydrogen atom, and $R^1$, $R^6$, $R^7$ and n are as in the combinations shown in [Table 1] to [Table 24];
In the formula (1-4S), the compounds of the present invention wherein $R^3$ is an amino group, $R^2$, $R^4$, $R^5$, $R^9$ and $R^{10}$ are a hydrogen atom, and $R^1$, $R^6$, $R^7$ and n are as in the combinations shown in [Table 1] to [Table 24];
In the formula (1-4S), the compounds of the present invention wherein $R^3$ is a methylamino group, $RR^2$, $R^4$, $R^5$, $R^9$ and $R^{10}$ are a hydrogen atom, and $R^1$, $R^6$, $R^7$ and n are as in the combinations shown in [Table 1] to [Table 24];
In the formula (1-4S), the compounds of the present invention wherein $R^3$ is an ethylamino group, $R^2$, $R^4$, $R^5$, $R^9$ and $R^{10}$ are a hydrogen atom, and $R^1$, $R^6$, $R^7$ and n are as in the combinations shown in [Table 1] to [Table 24];
In the formula (1-4S), the compounds of the present invention wherein $R^3$ is a propylamino group, $R^2$, $R^4$, $R^5$, $R^9$ and $R^{10}$ are a hydrogen atom, and $R^1$, $R^6$, $R^7$ and n are as in the combinations shown in [Table 1] to [Table 24];
In the formula (1-4S), the compounds of the present invention wherein $R^3$ is a dimethylamino group, $R^2$, $R^4$, $R^5$, $R^9$ and

$R^{10}$ are a hydrogen atom, and $R^1$, $R^6$, $R^7$ and n are as in the combinations shown in [Table 1] to [Table 24];
In the formula (1-4S), the compounds of the present invention wherein $R^3$ is a diethylamino group, $R^2$, $R^4$, $R^5$, $R^9$ and $R^{10}$ are a hydrogen atom, and $R^1$, $R^6$, $R^7$ and n are as in the combinations shown in [Table 1] to [Table 24];
In the formula (1-4S), the compounds of the present invention wherein $R^3$ is a dipropylamino group, $R^2$, $R^4$, $R^5$, $R^9$ and $R^{10}$ are a hydrogen atom, and $R^1$, $R^6$, $R^7$ and n are as in the combinations shown in [Table 1] to [Table 24];
In formula (1-5O),

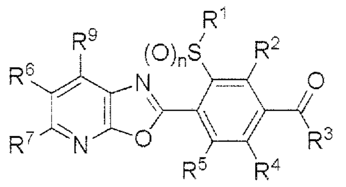 (1-5O)

the compounds of the present invention wherein $R^3$ is a methyl group, $R^2$, $R^4$, $R^5$ and $R^9$ are a hydrogen atom, and $R^1$, $R^6$, $R^7$ and n are as in the combinations shown in [Table 1] to [Table 24]; In the formula (1-5O), the compounds of the present invention wherein $R^3$ is an ethyl group, $R^2$, $R^4$, $R^5$ and $R^9$ are a hydrogen atom, and $R^1$, $R^6$, $R^7$ and n are as in the combinations shown in [Table 1] to [Table 24];
In the formula (1-50), the compounds of the present invention wherein $R^3$ is a propyl group, $R^2$, $R^4$, $R^5$ and $R^9$ are a hydrogen atom, and $R^1$, $R^6$, $R^7$ and n are as in the combinations shown in [Table 1] to [Table 24];
In the formula (1-50), the compounds of the present invention wherein $R^3$ is an isopropyl group, $R^2$, $R^4$, $R^5$ and $R^9$ are a hydrogen atom, and $R^1$, $R^6$, $R^7$ and n are as in the combinations shown in [Table 1] to [Table 24];
In the formula (1-5O), the compounds of the present invention wherein $R^3$ is a methoxy group, $R^2$, $R^4$, $R^5$ and $R^9$ are a hydrogen atom, and $R^1$, $R^6$, $R^7$ and n are as in the combinations shown in [Table 1] to [Table 24];
In the formula (1-50), the compounds of the present invention wherein $R^3$ is an ethoxy group, $R^2$, $R^4$, $R^5$ and $R^9$ are a hydrogen atom, and $R^1$, $R^6$, $R^7$ and n are as in the combinations shown in [Table 1] to [Table 24];
In the formula (1-5O), the compounds of the present invention wherein $R^3$ is a propoxy group, $R^2$, $R^4$, $R^5$ and $R^9$ are a hydrogen atom, and $R^1$, $R^6$, $R^7$ and n are as in the combinations shown in [Table 1] to [Table 24];
In the formula (1-5O), the compounds of the present invention wherein $R^3$ is an amino group, $R^2$, $R^4$, $R^5$ and $R^9$ are a hydrogen atom, and $R^1$, $R^6$, $R^7$ and n are as in the combinations shown in [Table 1] to [Table 24];
In the formula (1-50), the compounds of the present invention wherein $R^3$ is a methylamino group, $R^2$, $R^4$, $R^5$ and $R^9$ are a hydrogen atom, and $R^1$, $R^6$, $R^7$ and n are as in the combinations shown in [Table 1] to [Table 24];
In the formula (1-50), the compounds of the present invention wherein $R^3$ is an ethylamino group, $R^2$, $R^4$, $R^5$ and $R^9$ are a hydrogen atom, and $R^1$, $R^6$, $R^7$ and n are as in the combinations shown in [Table 1] to [Table 24];
In the formula (1-50), the compounds of the present invention wherein $R^3$ is a propylamino group, $R^2$, $R^4$, $R^5$ and $R^9$ are a hydrogen atom, and $R^1$, $R^6$, $R^7$ and n are as in the combinations shown in [Table 1] to [Table 24];
In the formula (1-5O), the compounds of the present invention wherein $R^3$ is a dimethylamino group, $R^2$, $R^4$, $R^5$ and $R^9$ are a hydrogen atom, and $R^1$, $R^6$, $R^7$ and n are as in the combinations shown in [Table 1] to [Table 24];
In the formula (1-5O), the compounds of the present invention wherein $R^3$ is a diethylamino group, $R^2$, $R^4$, $R^5$ and $R^9$ are a hydrogen atom, and $R^1$, $R^6$, $R^7$ and n are as in the combinations shown in [Table 1] to [Table 24];
In the formula (1-50), the compounds of the present invention wherein $R^3$ is a dipropylamino group, $R^2$, $R^4$, $R^5$ and $R^9$ are a hydrogen atom, and $R^1$, $R^6$, $R^7$ and n are as in the combinations shown in [Table 1] to [Table 24];
In formula (1-5S),

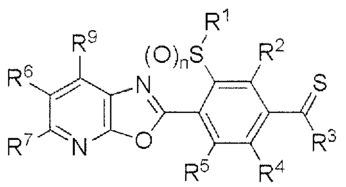 (1-5S)

the compounds of the present invention wherein $R^3$ is a methyl group, $R^2$, $R^4$, $R^5$ and $R^9$ are a hydrogen atom, and $R^1$, $R^6$, $R^7$ and n are as in the combinations shown in [Table 1] to [Table 24]; In the formula (1-5S), the compounds of the present invention wherein $R^3$ is an ethyl group, $R^2$, $R^4$, $R^5$ and $R^9$ are a hydrogen atom, and $R^1$, $R^6$, $R^7$ and n are as in the combinations shown in [Table 1] to [Table 24];
In the formula (1-5S), the compounds of the present invention wherein $R^3$ is a propyl group, $R^2$, $R^4$, $R^5$ and $R^9$ are a hydrogen atom, and $R^1$, $R^6$, $R^7$ and n are as in the combinations shown in [Table 1] to [Table 24];
In the formula (1-5S), the compounds of the present invention wherein $R^3$ is an isopropyl group, $R^2$, $R^4$, $R^5$ and $R^9$ are

a hydrogen atom, and $R^1$, $R^6$, $R^7$ and n are as in the combinations shown in [Table 1] to [Table 24];
In the formula (1-5S), the compounds of the present invention wherein $R^3$ is a methoxy group, $R^2$, $R^4$, $R^5$ and $R^9$ are. a hydrogen atom, and $R^1$, $R^6$, $R^7$ and n are as in the combinations shown in [Table 1] to [Table 24];
In the formula (1-5S), the compounds of the present invention wherein $R^3$ is an ethoxy group, $R^2$, $R^4$, $R^5$ and $R^9$ are a hydrogen atom, and $R^1$, $R^6$, $R^7$ and n are as in the combinations shown in [Table 1] to [Table 24];
In the formula (1-5S), the compounds of the present invention wherein $R^3$ is a propoxy group, $R^2$, $R^4$, $R^5$ and $R^9$ are a hydrogen atom, and $R^1$, $R^6$, $R^7$ and n are as in the combinations shown in [Table 1] to [Table 24];
In the formula (1-5S), the compounds of the present invention wherein $R^3$ is an amino group, $R^2$, $R^4$, $R^5$ and $R^9$ are a hydrogen atom, and $R^1$, $R^6$, $R^7$ and n are as in the combinations shown in [Table 1] to [Table 24];
In the formula (1-5S), the compounds of the present invention wherein $R^3$ is a methylamino group, $R^2$, $R^4$, $R^5$ and $R^9$ are a hydrogen atom, and $R^1$, $R^6$, $R^7$ and n are as in the combinations shown in [Table 1] to [Table 24];
In the formula (1-5S), the compounds of the present invention wherein $R^3$ is an ethylamino group, $R^2$, $R^4$, $R^5$ and $R^9$ are a hydrogen atom, and $R^1$, $R^6$, $R^7$ and n are as in the combinations shown in [Table 1] to [Table 24];
In the formula (1-5S), the compounds of the present invention wherein $R^3$ is a propylamino group, $R^2$, $R^4$, $R^5$ and $R^9$ are a hydrogen atom, and $R^1$, $R^6$, $R^7$ and n are as in the combinations shown in [Table 1] to [Table 24];
In the formula (1-5S), the compounds of the present invention wherein $R^3$ is a dimethylamino group, $R^2$, $R^4$, $R^5$ and $R^9$ are a hydrogen atom, and $R^1$, $R^6$, $R^7$ and n are as in the combinations shown in [Table 1] to [Table 24];
In the formula (1-5S), the compounds of the present invention wherein $R^3$ is a diethylamino group, $R^2$, $R^4$, $R^5$ and $R^9$ are a hydrogen atom, and $R^1$, $R^6$, $R^7$ and n are as in the combinations shown in [Table 1] to [Table 24];
In the formula. (1-5S), the compounds of the present invention wherein $R^3$ is a dipropylamino group, $R^2$, $R^4$, $R^5$ and $R^9$ are a hydrogen atom, and $R^1$, $R^6$, $R^7$ and n are as in the combinations shown in [Table 1] to [Table 24];
In formula (1-60),

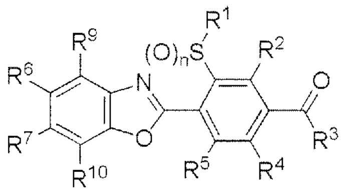

(1-6O)

the compounds of the present invention wherein $R^3$ is a methyl group, $R^2$, $R^4$, $R^5$, $R^9$ and $R^{10}$ are a hydrogen atom, and $R^1$, $R^6$, $R^7$ and n are as in the combinations shown in [Table 1] to [Table 24];
In the formula (1-60), the compounds of the present invention wherein $R^3$ is an ethyl group, $R^2$, $R^4$, $R^5$, $R^9$ and $R^{10}$ are a hydrogen atom, and $R^1$, $R^6$, $R^7$ and n are as in the combinations shown in [Table 1] to [Table 24];
In the formula (1-60), the compounds of the present invention wherein $R^3$ is a propyl group, $R^2$, $R^4$, $R^5$, $R^9$ and $R^{10}$ are a hydrogen atom, and $R^1$, $R^6$, $R^7$ and n are as in the combinations shown in [Table 1] to [Table 24];
In the formula (1-60), the compounds of the present invention wherein $R^3$ is an isopropyl group, $R^2$, $R^4$, $R^5$, $R^9$ and $R^{10}$ are a hydrogen atom, and $R^1$, $R^6$, $R^7$ and n are as in the combinations shown in [Table 1] to [Table 24];
In the formula (1-60), the compounds of the present invention wherein $R^3$ is a methoxy group, $R^2$, $R^4$, $R^5$, $R^9$ and $R^{10}$ are a hydrogen atom, and $R^1$, $R^6$, $R^7$ and n are as in the combinations shown in [Table 1] to [Table 24];
In the formula (1-60), the compounds of the present invention wherein $R^3$ is an ethoxy group, $R^2$, $R^4$, $R^5$, $R^9$ and $R^{10}$ are a hydrogen atom, and $R^1$, $R^6$, $R^7$ and n are as in the combinations shown in [Table 1] to [Table 24];
In the formula (1-60), the compounds of the present invention wherein $R^3$ is a propoxy group, $R^2$, $R^4$, $R^5$, $R^9$ and $R^{10}$ are a hydrogen atom, and $R^1$, $R^6$, $R^7$ and n are as in the combinations shown in [Table 1] to [Table 24];
In the formula (1-60), the compounds of the present invention wherein $R^3$ is an amino group, $R^2$, $R^4$, $R^5$, $R^9$ and $R^{10}$ are a hydrogen atom, and $R^1$, $R^6$, $R^7$ and n are as in the combinations shown in [Table 1] to [Table 24];
In the formula (1-60), the compounds of the present invention wherein $R^3$ is a methylamino group, $R^2$, $R^4$, $R^5$, $R^9$ and $R^{10}$ are a hydrogen atom, and $R^1$, $R^6$, $R^7$ and n are as in the combinations shown in [Table 1] to [Table 24];
In the formula (1-60), the compounds of the present invention wherein $R^3$ is an ethylamino group, $R^2$, $R^4$, $R^5$, $R^9$ and $R^{10}$ are a hydrogen atom, and $R^1$, $R^6$, $R^7$ and n are as in the combinations shown in [Table 1] to [Table 24];
In the formula (1-60), the compounds of the present invention wherein $R^3$ is a propylamino group, $R^2$, $R^4$, $R^5$, $R^9$ and $R^{10}$ are a hydrogen atom, and $R^1$, $R^6$, $R^7$ and n are as in the combinations shown in [Table 1] to [Table 24];
In the formula (1-6O), the compounds of the present invention wherein $R^3$ is a dimethylamino group, $R^2$, $R^4$, $R^5$, $R^9$ and $R^{10}$ are a hydrogen atom, and $R^1$, $R^6$, $R^7$ and n are as in the combinations shown in [Table 1] to [Table 24];
In the formula (1-60), the compounds of the present invention wherein $R^3$ is a diethylamino group, $R^2$, $R^4$, $R^5$, $R^9$ and $R^{10}$ are a hydrogen atom, and $R^1$, $R^6$, $R^7$ and n are as in the combinations shown in [Table 1] to [Table 24];.
In the formula (1-60), the compounds of the present invention wherein $R^3$ is a dipropylamino group, $R^2$, $R^4$, $R^5$, $R^9$ and

$R^{10}$ are a hydrogen atom, and $R^1$, $R^6$, $R^7$ and n are as in the combinations shown in [Table 1] to [Table 24];
In formula (1-6S),

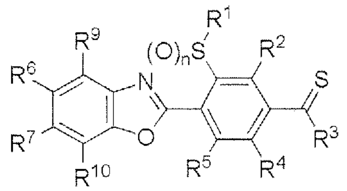
(1-6S)

the compounds of the present invention wherein $R^3$ is a methyl group, $R^2$, $R^4$, $R^5$, $R^9$ and $R^{10}$ are a hydrogen atom, and $R^1$, $R^6$, $R^7$ and n are as in the combinations shown in [Table 1] to [Table 24];
In the formula (1-6S), the compounds of the present invention wherein $R^3$ is an ethyl group, $R^2$, $R^4$, $R^3$, $R^9$ and $R^{1C}$ are a hydrogen atom, and $R^1$, $R^6$, $R^7$ and n are as in the combinations shown in [Table 1] to [Table 24];
In the formula (1-6S), the compounds of the present invention wherein $R^3$ is a propyl group, $R^2$, $R^4$, $R^5$, $R^9$ and $R^{10}$ are a hydrogen atom, and $R^1$, $R^6$, $R^7$ and n are as in the combinations shown in [Table 1] to [Table 24];
In the formula (1-6S), the compounds of the present invention wherein $R^3$ is an isopropyl group, $R^2$, $R^4$, $R^5$, $R^9$ and $R^{10}$ are a hydrogen atom, and $R^1$, $R^6$, $R^7$ and n are as in the combinations shown in [Table 1] to [Table 24];
In the formula (1-6S), the compounds of the present invention wherein $R^3$ is a methoxy group, $R^2$, $R^4$, $R^5$, $R^9$ and $R^{10}$ are a hydrogen atom, and $R^1$, $R^6$, $R^7$ and n are as in the combinations shown in [Table 1] to [Table 24];
In the formula (1-6S), the compounds of the present invention wherein $R^3$ is an ethoxy group, $R^2$, $R^4$, $R^5$, $R^9$ and $R^{10}$ are a hydrogen atom, and $R^1$, $R^6$, $R^7$ and n are as in the combinations shown in [Table 1] to [Table 24];
In the formula (1-6S), the compounds of the present invention wherein $R^3$ is a propoxy group, $R^2$, $R^4$, $R^5$, $R^9$ and $R^{10}$ are a hydrogen atom, and $R^1$, $R^6$, $R^7$ and n are as in the combinations shown in [Table 1] to [Table 24];
In the formula (1-6S), the compounds of the present invention wherein $R^3$ is an amino group, $R^2$, $R^4$, $R^5$, $R^9$ and $R^{10}$ are a hydrogen atom, and $R^1$, $R^6$, $R^7$ and.n are as in the combinations shown in [Table 1] to [Table 24];
In the formula (1-6S), the compounds of the present invention wherein $R^3$ is a methylamino group, $R^2$, $R^4$, $R^5$, $R^9$ and $R^{10}$ are a hydrogen atom, and $R^1$, $R^6$, $R^7$ and n are as in the combinations shown in [Table 1] to [Table 24];
In the formula (1-6S), the compounds of the present invention wherein $R^3$ is an ethylamino group, $R^2$, $R^4$, $R^5$, $R^9$ and $R^{10}$ are a hydrogen atom, and $R^1$, $R^6$, $R^7$ and n are as in the combinations shown in [Table 1] to [Table 24];
In the formula (1-6S), the compounds of the present invention wherein $R^3$ is a propylamino group, $R^2$, $R^4$, $R^5$, $R^9$ and $R^{10}$ are a hydrogen atom, and $R^1$, $R^6$, $R^7$ and n are as in the combinations shown in [Table 1] to [Table 24];
In the formula (1-6S), the compounds of the present invention wherein $R^3$ is a dimethylamino group, $R^2$, $R^4$, $R^5$, $R^9$ and $R^{10}$ are a hydrogen atom, and $R^1$, $R^6$, $R^7$ and n are as in the combinations shown in [Table 1] to [Table 24];
In the formula (1-6S), the compounds of the present invention wherein $R^3$ is a diethylamino group, $R^2$, $R^4$, $R^5$, $R^9$ and $R^{10}$ are a hydrogen atom, and $R^1$, $R^6$, $R^7$ and n are as in the combinations shown in [Table 1] to [Table 24]; and
In the formula (1-6S), the compounds of the present invention wherein $R^3$ is a dipropylamino group, $R^2$, $R^4$, $R^5$, $R^9$ and $R^{10}$ are a hydrogen atom, and $R^1$, $R^6$, $R^7$ and n are as in the combinations shown in [Table 1] to [Table 24].

Examples of the pest on which the compound of the present invention has an effect include arthropod pests such as pest insects and pest mites and nematoda. Specifically, examples of the pests include those shown below.

Hemiptera: Delphacidae such as Laodelphax striatellus, Nilaparvata lugens, and Sogatella furcifera, Deltocephalidae such as Nephotettix cincticeps, Nephotettix virescens, and Empoasca onukii, Aphididae such as Aphis gossypii, Myzus persicae, Brevicoryne brassicae, Aphis spiraecola, Macrosiphum euphorbiae, Aulacorthum solani, Rhopalosiphum padi, Toxoptera citricidus, and Hyalopterus pruni, Pentatomidae such as Nezara antennata, Riptortus clavetus, Leptocorisa chinensis, Eysarcoris parvus, and Halyomorpha mista, Aleyrodidae such as Trialeurodes vaporariorum, Bemisia tabaci, Dialeurodes citri, and Aleurocanthus spiniferus, Coccidae such as Aonidiella aurantii, Comstockaspis perniciosa, Unaspis citri, Ceroplastes rubens, Icerya purchasi, Planococcus kraunhiae, Pseudococcus longispinis, and Pseudaulacaspis pentagona, Tingidae, Cimicoidea such as Cimex lectularius, and Psyliidae.

Lepidoptera: Pyralidae such as Chilo suppressalis, Tryporyza incertulas, Cnaphalocrocis medinalis, Notarcha derogata, Plodia interpunctella, Ostrinia furnacalis, Hellula undalis, and Pediasia teterrellus, Noctuidae such as Spodoptera litura, Spodoptera exigua, Pseudaletia separata, Mamestra brassicae, Agrotis ipsilon, Plusia nigrisigna, Thoricoplusia spp., Heliothis spp., and Helicoverpa spp., Pieridae such as Pieris rapae, Adoxophyes spp., Tortricidae such as Grapholita molesta, Leguminivora glycinivorella, Matsumuraeses azukivora, Adoxophyes orana fasciata, Adoxophyes honmai., Homona magnanima, Archips fuscocupreanus, and Cydia pomonella, Gracillariidae such as Caloptilia theivora and Phyllonorycter ringoneella, Carposinidae such as Carposina niponensis, Lyonetiidae such as Lyonetia spp., Lymantriidae such as Lymantria spp. and Euproctis spp., Yponomeutidae such as Plutella xylostella, Gelechiidae such as Pectinophora gossypiella and Phthorimaea operculella, Arctiidae such as Hyphantria cunea, and Tineidae such as Tinea

translucens and Tineola bisselliella.

Thysanoptera: Thripidae such as Frankliniella occidentalis, Thrips parmi, Scirtothrips dorsalis, Thrips tabaci, and Frankliniella intonsa.

Diptera: Culex such as Culex pipiens pallens, Culex tritaeniorhynchus, and Culex quinquefasciatus, Aedes spp. such as Aedes aegypti and Aedes albopictus, Anopheles spp. such as Anopheles sinensis, Chironomidae, Muscidae such as Musca domestica and Muscina stabulans, Calliphoridae, Sarcophagidae, Fanniidae, Anthomyiidae such as Delia platura and Delia antiqua, Agromyzidae such as Agromyza oryzae, Hydrellia griseola, Liriomyza sativae, Liriomyza trifolii, and Chromatomyia horticola, Chloropidae such as Chlorops oryzae, Tephritidae such as Dacus cucurbitae and Ceratitis capitata, Drosophilidae, Phoridae such as Megaselia spiracularis, Psychodidae such as Clogmia albipunctata, Sciaridae, Simuliidae, Tabanidae such as Tabanus trigonus, Hippoboscidae, and Stomoxys.

Coleoptera: Diabrotica such as Diabrotica virgifera virgifera and Diabrotica undecimpunctata howardi, Scarabaeidae such as Anomala cuprea, Anomala rufocuprea, and Popillia japonica, Curculionidae such as Sitophilus zeamais, Lissorhoptrus oryzophilus, Callosobruchuys chienensis, Echinocnemus squameus, Anthonomus grandis, and Sphenophorus venatus, Tenebrionidae such as Tenebrio molitor and Tribolium castaneum, Chrysomelidae such as Oulema oryzae, Aulacophora femoralis, Phyllotreta striolata, and Leptinotarsa decemlineata, Dermestidae such as Anthrenus verbasci and Dermestes maculates, Anobiidae such as Lasioderma serricorne, Epilachna such as Epilachna vigintioctopunctata, Lyctus brunneus, Scolytidae such as Tomicus piniperda, Bostrychidae, Ptinidae, Cerambycidae such as Anoplophora malasiaca, Agriotes spp., and Paederus fuscipes.

Orthoptera: Locusta migratoria, Gryllotalpa africana, Oxya yezoensis, Oxya japonica, and Grylloidea.

Aphaniptera: Ctenocephalides felis, Ctenocephalides canis, Pulex irritans, and Xenopsylla cheopis.

Anoplura: Pediculus humanus corporis, Pediculus humanus humanus, Phthirus pubis, Haematopinus eurysternus, Dalmalinia ovis, Haematopinus suis, and Linognathus setosus.

Mallophaga: Dalmalinia ovis, Dalmalinia bovis, Menopon gallinae, Trichodectes canis, and Felicola subrostrata.

Hymenoptera: Formicidae such as Monomorium pharaosis, Formica fusca japonica, Ochetellus glaber, Pristomyrmex pungens, Pheidole noda, Acromyrmex spp., Solenopsis spp., and Linepithema humile, Vespidae, Bethylidae, and Tenthredinidae such as Athalia rosae and Athalia japonica.

Nematoda: Aphelenchoides besseyi, Nothotylenchus acris, Meloidogyne incognita, Meloidogyne hapla, Meloidogyne javanica, Heterodera glycines, Globodera rostochiensis, Pratylenchus coffeae, and Pratylenchus neglectus.

Blattodea: Blattella germanica, Periplaneta fuliginosa, Periplaneta americana, Periplaneta brunnea, and Blatta orientalis.

Isoptera: Reticulitermes speratus, Coptotermes formosanus, Incisitermes minor, Cryptotermes domesticus, Odontotermes formosanus, Neotermes koshunensis, Glyptotermes satsumensis, Glyptotermes nakajimai, Glyptotermes fuscus, Glyptotermes kodamai, Glyptotermes kushimensis, Hodotermopsis japonica, Coptotermes guangzhoensis, Reticulitermes miyatakei, Reticulitermes flaviceps amamianus, Reticulitermes sp., Nasutitermes takasagoensis, Pericapritermes nitobei, and Sinocapritermes mushae.

Acarina: Tetranychidae such as Tetranychus urticae, Tetranychus kanzawai, Panonychus citri, Panonychus ulmi, and Oligonychus spp., Eriophyidae such as Aculops pelekassi, Phyllocoptruta citri, Aculops lycopersici, Calacarus carinatus, Acaphylla theavagrans, Eriophyes chibaensis, and Aculus schlechtendali, Tarsonemidae such as Polyphagotarsonemus latus, Tenuipalpidae such as Brevipalpus phoenicis, Tuckerellidae, Metastigmata such as Haemaphysalis longicornis, Haemaphysalis flava, Dermacentor taiwanicus, Dermacentor variabilis, Ixodes ovatus, Ixodes persulcatus, Ixodes scapularis, Amblyomma americanum, Boophilus microplus, and Rhipicephalus sanguineus, Acaridae such as Tyrophagus putrescentiae and Tyrophagus similis, Pyroglyphidae such as Dermatophagoides farinae and Dermatophagoides ptrenyssnus, Cheyletidae such as Cheyletus eruditus, Cheyletus malaccensis, Cheyletus moorei, and Cheyletiella yasguri, Sarcoptidae such as Octodectes cynotis and Sacroptes scabiei, Demodicidae such as Demodex canis, Listrophoridae, Cryptostigmata, Dermanyssidae such as Ornithonyssus bacoti, Ornithonyssus sylvairum, and Dermanyssus gallinae, Trombiculidae such as Leptotrombidium akamushi, and Arachnida such as Chiracanthium japonicum and Latrodectus hasseltii.

Chilopoda: Thereuonema hilgendorfi and Scolopendra subspinipes.

Diplopoda: Oxidus gracilis and Nedyopus tambanus.

Isopoda: Armadillidium vulgare.

Gastropoda: Limax marginatus and Limax flavus.

The pest control agent of the present invention contains the compound of the present invention and an inert carrier. The pest control agent of the present invention is usually obtained by mixing the compound of the present invention and an inert carrier such as a solid carrier, a liquid carrier or a gaseous carrier, and adding a surfactant or other auxiliaries for formulation as necessary, to be formulated into emulsifiable concentrates, oil formulations, dust formulations, granules, wettable powders, flowables, microcapsule formulations, aerosols, smoking agents, poisonous bait formulations, resin formulations, shampoo agents, paste formulations, foam agents, carbon dioxide preparations, tablets, and the like. These formulations may be processed into mosquito repellent coil, electric mosquito repellent mat,

mosquito repellent liquid formulation, smoking agent, fumigant, sheet formulation, spot-on agent, or oral treatment agent, and used.

The pest control agent of the present invention usually contains the compound of the present invention in an amount of 0.01 to 95% by weight.

Examples of the solid carrier which is used in the formulation include fine powder and granules of clays (kaolin clay, diatomaceous earth, bentonite, Fubasami clay, acid clay, etc.), synthetic hydrated silicon oxide, talc, ceramics, other inorganic minerals (sericite, quartz, sulfur, activated carbon, calcium carbonate, hydrated silica, etc.), fine powder and granulated substances of chemical fertilizers (ammonium sulfate, ammonium phosphate, ammonium nitrate, urea, ammonium chloride, etc.) and the like, synthetic resins (polyester resins such as polypropylene, polyacrylonitrile, polymethylmethacrylate and polyethylene terephtalate, nylon resins such as nylon-6, nylon-11 and nylon-66, polyamide resin, polyvinyl chloride, polyvinylidene chloride, vinyl chloride-propylene copolymer, etc.).

Examples of the liquid carrier include water, alcohols (methanol, ethanol, isopropyl alcohol, butanol, hexanol, benzyl alcohol, ethylene glycol, propylene glycol, phenoxyethanol, etc.), ketones (acetone, methyl ethyl ketone, cyclohexanone, etc.), aromatic hydrocarbons (toluene, xylene, ethylbenzene, dodecylbenzene, phenylxylylethane, methylnaphthalene, etc.), aliphatic hydrocarbons (hexane, cyclohexane, kerosene, light oil, etc.), esters (ethyl acetate, butyl acetate, isopropyl myristate, ethyl oleate, diisopropyl adipate, diisobutyl adipate, propylene glycol monomethyl ether acetate, etc.), nitriles (acetonitrile, isobutyronitrile, etc.), ethers (diisopropyl ether, 1,4-dioxane, ethylene glycol dimethyl ether, diethylene glycol dimethyl ether, diethylene glycol monomethyl ether, propylene glycol monomethyl ether, dipropylene glycol monomethyl ether, 3-methoxy-3-methyl-1-butanol, etc.), acid amides (N,N-dimethylformamide, N,N-dimethylacetamide, etc.), halogenated hydrocarbons (dichloromethane, trichloroethane, carbon tetrachloride, etc.), sulfoxides (dimethyl sulfoxide, etc.), and propylene carbonate and vegetable oils (soybean oil, cottonseed oil, etc.).

Examples of the gaseous carrier include fluorocarbon, butane gas, LPG (liquefied petroleum gas), dimethyl ether, and carbon dioxide.

Examples of the surfactant include nonionic surfactants such as polyoxyethylene alkyl ether, polyoxyethylene alkylaryl ether and polyethylene glycol fatty acid ester, and anionic surfactants such as alkyl sulfonates, alkylbenzene sulfonates and alkylsulfates.

The other auxiliaries for formulation include such as fixing agents, dispersants, colorants and stabilizers, specifically, for example, casein, gelatin, polysaccharides (starch, arabic gum, cellulose derivatives, alginic acid, etc.), lignin derivatives, bentonite, synthetic water-soluble polymers (polyvinyl alcohol, polyvinyl pyrrolidone, polyacrylic acid, etc.), PAP (isopropyl acid phosphate), BHT (2,6-di-tert-butyl-4-methylphenol) and BHA (mixtures of 2-tert-butyl-4-methoxyphenol and 3-tert-butyl-4-methoxyphenol).

Examples of a base material of the resin formulation include vinyl chloride polymer, polyurethane and the like, and a plasticizer such as phthalate esters (dimethyl phthalate, dioctyl phthalate, etc.), adipate esters or stearic acid may be added to these base materials as necessary. The resin formulation is obtained by kneading a compound into the base material using an ordinary kneading apparatus, and then molding it by injection molding, extrusion molding, press molding or the like, and the resin formulation obtained can be further subjected to molding or cutting step as necessary to be processed into a plate, film, taped, reticular or string resin formulation. These resin formulations are processed into, for example, a collar for animal, an ear tag for animal, a sheet formulation, an induction cord, and a gardening pole.

Examples of a base material of the poisonous bait include grain powder, vegetable oil, sugar, crystalline cellulose and the like, and further, an antioxidant such as dibutylhydroxytoluene and nordihydroguaiaretic acid, a preservative such as dehydroacetic acid, a substance for preventing accidental ingestion by children and.pets such as red pepper powder, a pest attractant flavor such as cheese flavor, onion flavor and peanut oil or the like are added as necessary.

The method for controlling pests of the present invention is carried out by applying an effective amount of the compound of the present invention to a pest directly and/or a pest-infested area (plants, soil, in-house, animal body, etc.). In the method for controlling pests of the present invention, the compound is usually used in the form of the pest control agent of the present invention.

When the pest control agent of the present invention is used in pest controlling in the agricultural field, the application amount is usually 1 to 10000 g in the amount of the compound of the present invention per 10000 $m^2$. When the pest control agent of the present invention is formulated into an emulsifiable concentrate, a wettable powder, a flowable or the like, the pest control agent is usually diluted with water for an application so as to have a concentration of the active ingredient of 0.01 to 10000 ppm, and dust formulations, granules and the like are usually applied as they are.

These formulations and formulation solutions diluted with water may be directly applied by being sprayed on a pest or a plant such as crops which should be protected from pests, and also may be applied to a soil in order to control a pest that infests in the soil of cultivated land.

Also, the resin formulation processed into a sheet or string can be also applied by a method such as winding it around crops, spreading it in the vicinity of crops, or spreading it to the soil around crop roots.

When the pest control agent of the present invention is used in controlling the pest that inhabits in the house, the application amount is usually 0.01 to 1000 mg in an amount of the compound of the present invention per 1 $m^2$ of

an area to be treated, in the case of using it on a planar area, and is usually 0.01 to 500 mg in an amount of the compound of the present invention per 1 $m^3$ of a space to be treated, in the case of using it in a space. When the pest control agent of the present invention is formulated into an emulsifiable concentrate, a wettable powder, a flowable or the like, the pest control agent is usually diluted with water for an application so as to have a concentration of the active ingredient of 0.1 to 10000 ppm, and oil formulations, aerosols, smoking agents, poisonous bait formulations and the like are applied as they are.

When the arthropod pest control agent of the present invention is used in the control of external parasites on livestock such as cows, horses, pigs, sheep, goats and chickens, and small animals such as dogs, cats, rats and mice, veterinary known methods can be applied to the animals. As specific methods, the formulation is administered, for example, by way of a tablet, mixing in feed, a suppository and injection (intramuscular, subcutaneous, intravenous, intraperitoneal injections, etc.), when systemic control is intended, and the formulation is used, for example, by way of spraying an oil solution or aqueous solution, pour-on or spot-on treatment, washing an animal with a shampoo formulation, or putting a collar or ear tag made of a resin formulation on to an animal, when non-systemic control is intended. The amount of the compound of the present invention when administered to an animal body is usually in the range from 0.1 to 1000 mg per 1 kg of the weight of an animal.

The pest control agent of the present invention can be used in the farmlands where the following crops are grown.

Crops: corn, rice, wheat, barley, rye, oat, sorghum, cotton, soybean, peanut, sarrazin, sugar beet, rapeseed, sunflower, sugar cane, tobacco, etc.

Vegetables: Solanaceae vegetables (eggplant, tomato, green pepper, hot pepper, potato, etc.), Cucurbitaceae vegetables (cucumber, pumpkin, zucchini, watermelon, melon, etc.), Cruciferae vegetables (Japanese radish, turnip, horseradish, kohlrabi, Chinese cabbage, cabbage, brown mustard, broccoli, cauliflower, etc.), Compositae vegetables (burdock, garland chrysanthemum, artichoke, lettuce, etc.), Liliaceae vegetables (Welsh onion, onion, garlic, asparagus, etc.), Umbelliferae vegetables (carrot, parsley, celery, parsnip, etc.), Chenopodiaceae vegetables (spinach, Swiss chard, etc.), Labiatae vegetables (Japanese mint, mint, basil, etc.), strawberry, sweat potato, yarn, aroid, etc.

Fruit trees: pomaceous fruits (apple, common pear, Japanese pear, Chinese quince, quince, etc.), stone fleshy fruits (peach, plum, nectarine, Japanese plum, cherry, apricot, prune, etc.), citrus plants (Satsuma mandarin, orange, lemon, lime, grapefruits, etc.), nuts (chestnut, walnut, hazel nut, almond, pistachio, cashew nut, macadamia nut, etc.), berry fruits (blueberry, cranberry, blackberry, raspberry, etc.), grape, persimmon, olive, loquat, banana, coffee, date, coconut, oil palm, etc.

Trees other than fruit trees: tea, mulberry, flowering trees and shrubs (azalea, camellia, hydrangea, sasanqua, Illicium religiosum, cherry tree, tulip tree, crape myrtle, fragrant olive, etc.), street trees (ash tree, birch, dogwood, eucalyptus, ginkgo, lilac, maple tree, oak, poplar, cercis, ..Chinese sweet gum, plane tree, zelkova, Japanese arborvitae, fir tree, Japanese hemlock, needle juniper, pine, spruce, yew, elm, horse-chestnut, etc.), sweet viburnum, Podocarpus macrophyllus, Japanese cedar, Japanese cypress, croton, spindle tree, Japanese photinia, etc.

Grass: zoysia (Japanese lawn grass, mascarene grass, etc.), Bermuda grass (Cynodon dactylon, etc.), bent grass (creeping bent grass, Agrostis stolonifera, Agrostis tenuis, etc.), bluegrass (Kentucky bluegrass, rough bluegrass, etc.), fescue (tall fescue, chewing fescue, creeping fescue, etc.), ryegrass (darnel, perennial ryegrass, etc.), cocksfoot, timothy grass, etc.

Others: flowers (rose, carnation, chrysanthemum, Eustoma grandiflorum Shinners (prairie gentian), gypsophila, gerbera, pot marigold, salvia, petunia, verbena, tulip, aster, gentian, lily, pansy, cyclamen, orchid, lily of the valley, lavender, stock, ornamental kale, primula, poinsttia, gladiolus, cattleya, daisy, cymbidium, begonia, etc.), bio-fuel plants (Jatropha, curcas, safflower, Camelina alyssum, switchgrass, miscanthus, reed canary grass, Arundo donax, kenaf, cassava, willow, algae, etc.), foliage plants, etc.

The above crops also contain genetically modified crops.

The pest control agent of the present invention can be used as a mixture with or in combination with other insecticide, miticide, nematicide, fungicide, plant growth regulator, herbicide or synergist. Examples of the active ingredient of said insecticide, miticide, nematicide, fungicide, herbicide and synergist are shown below.

Active Ingredients of Insecticide

(1) Organic phosphorus compounds

acephate, aluminium phosphide, butathiofos, cadusafos, chlorethoxyfos, chlorfenvinphos, chlorpyrifos, chlorpyrifos-methyl, cyanophos: CYAP, diazinon, DCIP (dichlorodiisopropyl ether), dichlofenthion: ECP, dichlorvos: DDVP, dimethoate, dimethylvinphos, disulfoton, EPN, ethion, ethoprophos, etrimfos, fenthion: MPP, fenitrothion: MEP, fosthiazate, formothion, hydrogen phosphide, isofenphos, isoxathion, malathion, mesulfenfos, methidathion: DMTP, monocrotophos, naled: BRP, oxydeprofos: ESP, parathion, phosalone, phosmet: PMP, pirimiphos-methyl, pyridafenthion, quinalphos, phenthoate: PAP, profenofos, propaphos, prothiofos, pyraclorfos, salithion, sulprofos, tebupirimfos, temephos,

tetrachlorvinphos, terbufos, thiometon, trichlorphon: DEP, vamidothion, phorate, and cadusafos.

(2) Carbamate compounds

alanycarb, bendiocarb, benfuracarb, BPMC, carbaryl, carbofuran, carbosulfan, cloethocarb, ethiofencarb, fenobucarb, fenothiocarb, fenoxycarb, furathiocarb, isoprocarb: MIPC, metolcarb, methomyl, methiocarb, NAC, oxamyl, pirimicarb, propoxur: PHC, XMC, thiodicarb, xylylcarb, and aldicarb.

(3) Pyrethroid compounds

acrinathrin, allethrin, benfluthrin, beta-cyfluthrin, bifenthrin, cycloprothrin, cyfluthrin, cyhalothrin, cypermethrin, deltamethrin, esfenvalerate, ethofenprox, fenpropathrin, fenvalerate, flucythrinate, flufenoprox, flumethrin, fluvalinate, halfenprox, imiprothrin, permethrin, prallethrin, pyrethrins, resmethrin, sigma-cypermethrin, silafluofen, tefluthrin, tralomethrin, transfluthrin, tetramethrin, phenothrin, cyphenothrin, alpha-cypermethrin, zeta-cypermethrin, lambda-cyhalothrin, gamma-cyhalothrin, furamethrin, tau-fluvalinate, metofluthrin, profluthrin, dimefluthrin, 2,3,5,6-tetrafluoro-4-(methoxymethyl)benzyl (EZ)-(1RS,3RS;1RS,3SR)-2,2-dimethyl-3-prop-1-enylcyclopropa necarboxylate, 2,3,5,6-tetrafluoro-4-methylbenzyl (EZ)-(1RS,3RS;1RS,3SR)-2,2-dimethyl-3-prop-1-enylcyclopropa necarboxylate, 2,3,5,6-tetrafluoro-4-(methoxymethyl)benzyl (1RS,3RS;1RS,3SR)-2,2-dimethyl-3-(2-methyl-1-propenyl)cyclo propanecarboxylate, and 2,3,5,6-tetrafluoro-4-(methoxymethyl)benzyl(EZ)-(1RS,3RS:1R S,3SR)-2,2-dimethyl-3-(2-cyano-1-propenyl)cyclopropanecarbo xylate.

(4) Nereistoxin compounds

cartap, bensultap, thiocyclam, monosultap, andbisultap.

(5) Neonicotinoid Compounds

imidacloprid, nitenpyram, acetamiprid, thiamethoxam, thiacloprid, dinotefuran, and clothianidin.

(6) Benzoyl urea compounds

chlorfluazuron, bistrifluron, diafenthiuron, diflubenzuron, fluazuron, flucycloxuron, flufenoxuron, hexaflumuron, lufenuron, novaluron, noviflumuron, teflubenzuron, triflumuron, and triazuron.

(7) Phenylpyrazole compounds

acetoprole, ethiprole, fipronil, vaniliprole, pyriprole, and pyrafluprole.

(8) Bt toxins

Living spores derived from Bacillus thuringiensis and produced crystalline toxins and mixtures thereof;

(9) Hydrazine compounds

chromafenozide, halofenozide, methoxyfenozide, and tebufenozide.

(10) Organic chlorine compounds

aldrin, dieldrin, dienochlor, endosulfan, and methoxychlor.

(11) Other active ingredients of insecticide

machine oil, nicotine-sulfate; avermectin-B, bromopropylate, buprofezin, chlorphenapyr, cyantraniliprole, cyromazine, D-D(1, 3-Dichloropropene), emamectin-benzoate, fenazaquin, flupyrazofos, hydroprene, methoprene, indoxacarb, metoxadiazone, milbemycin-A, pymetrozine, pyridalyl, pyriproxyfen, spinosad, sulfluramid, tolfenpyrad, triazamate, flubendiamide, lepimectin, arsenic acid, benclothiaz, calcium cyanamide, calcium polysulfide, chlordane, DDT, DSP, flufenerim, flonicamid, flurimfen, formetanate, metam-ammonium, metam-sodium, methyl bromide, potassium oleate, protrifenbute, spiromesifen, sulfoxaflor, sulfur, metaflumizone, spirotetramat, pyrifluquinazone, spinetoram, chlorantraniliprole, tralopyril, cyantraniliprole, compounds represented by formula (K):

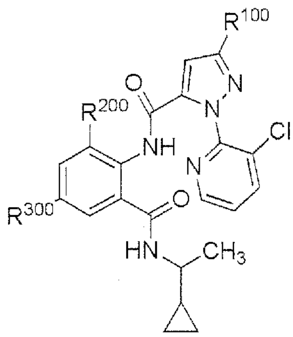

(K)

wherein $R^{100}$ represents chlorine, bromine or a trifluoromethyl group, $R^{200}$ represents chlorine, bromine or a methyl group, and $R^{300}$ represents chlorine, bromine or a cyano group, and compounds represented by formula (L):

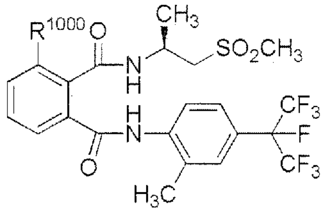

(L)

wherein $R^{1000}$ represents chlorine, bromine or iodine.

Active Ingredients of Miticide

acequinocyl, amitraz, benzoximate, bifenaate, bromopropylate, chinomethionat, chlorobenzilate, CPCBS (chlorfenson), clofentezine, cyflumetofen, kelthane (dicofol), etoxazole, fenbutatin oxide, fenothiocarb, fenpyroximate, fluacrypyrim, fluproxyfen, hexythiazox, propargite: BPPS, polynactins, pyridaben, pyrimidifen, tebufenpyrad, tetradifon, spirodiclofen, spiromesifen, spirotetramat, amidoflumet, and cyenopyrafen.

Active ingredients of Nematicide

DCIP, fosthiazate, levamisol, methyisothiocyanate, morantel tartarate, and imicyafos.

Active Ingredients of Fungicide

azole fungicidal compounds such as propiconazole, prothioconazole, triadimenol, prochloraz, penconazole, tebuconazole, flusilazole, diniconazole, bromuconazole, epoxiconazole, difenoconazole, cyproconazole, metconazole, triflumizole, tetraconazole, myclobutanil, fenbuconazole, hexaconazole, fluquinconazole, triticonazole, bitertanol, imazalil, and flutriafol; cyclic amine fungicidal compounds such as fenpropimorph, tridemorph, and fenpropidin; benzimidazole fungicidal compounds such as carbendezim, benomyl, thiabendazole, and thiophanate-methyl; procymidone; cyprodinil; pyrimethanil; diethofencarb; thiuram; fluazinam; mancozeb; iprodione; vinclozolin; chlorothalonil; captan; mepanipyrim; fenpiclonil; fludioxonil; dichlofluanid; folpet; kresoxim-methyl; azoxystrobin; trifloxystrobin; fluoxastrobin; picoxystrobin; pyraclostrobin; dimoxystrobin; pyribencarb; spiroxamine; quinoxyfen; fenhexamid; famoxadone; fenamidone; zoxamide; ethaboxam; amisulbrom; iprovalicarb; benthiavalicarb; cyazofamid; mandipropamid; boscalid; penthiopyrad; metrafenone; fluopiran; bixafen; cyflufenamid; proquinazid; isotianil and tiadinil.

Active Ingredients of Herbicide

(1) Phenoxy fatty acid herbicidal compounds

2,4-PA, MCP, MCPB, phenothiol, mecoprop, fluroxypyr, triclopyr, clomeprop, and naproanilide.

(2) Benzoate herbicidal compounds

2,3,6-TBA, dicamba, clopyralid, picloram, aminopyralid, quinclorac, and quinmerac.

(3) Urea herbicidal compounds

diuron, linuron, chlortoluron, isoproturon, fluometuron, isouron, tebuthiuron, methabenzthiazuron, cumyluron, daimuron, and methyl-daimuron.

(4) Triazine herbicidal compounds

atrazine, ametoryn, cyanazine, simazine, propazine, simetryn, dimethametryn, prometryn, metribuzin, triaziflam, and indaziflam.

(5) Bipyridinium herbicidal compounds

paraquat and diquat.

(6) Hydroxybenzonitrile herbicidal compounds

bromoxynil and ioxynil.

(7) Dinitroaniline herbicidal compounds

pendimethalin, prodiamine, and trifluralin.

(8) Organophosphorus herbicidal compounds

amiprofos-methyl, butamifos, bensulide, piperophos, anilofos, glyphosate, glufosinate, glufosinate-P, and bialaphos.

(9) Carbamate herbicidal compounds

di-allate, tri-allate, EPTC, butylate, benthiocarb, esprocarb, molinate, dimepiperate, swep, chlorpropham, phenmedipham, phenisopham, pyributicarb, and asulam.

(10) Acid amide herbicidal compounds

propanil, propyzamide, bromobutide, and etobenzanid.

(11) Chloroacetanilide herbicidal compounds

acetochlor, alachlor, butachlor, dimethenamid, propachlor, metazachlor, metolachlor, pretilachlor, thenylchlor, and pethoxamid.

(12) Diphenyl ether herbicidal compounds

acifluorfen-sodium, bifenox, oxyfluorfen, lactofen, fomesafen, chlomethoxynil, and aclonifen.

(13) Cyclic imide herbicidal compounds

oxadiazon, cinidon-ethyl, carfentrazone-ethyl, surfentrazone, flumiclorac-pentyl, flumioxazin, pyraflufen-ethyl, oxadiargyl, pentoxazone, fluthiacet-methyl, butafenacil, benzfendizone, bencarbazone, and saflufenacil.

(14) Pyrazole herbicidal compounds

benzofenap, pyrazolate, pyrazoxyfen, topramezone, and pyrasulfotole.

(15) Triketone herbicidal compounds

isoxaflutole, benzobicyclon, sulcotrione, mesotrione, tembotrione, and tefuryltrione.

(16) Aryloxyphenoxypropionate herbicidal compounds

clodinafop-propargyl, cyhalofop-butyl, diclofop-methyl, fenoxaprop-ethyl, fluazifop-butyl, haloxyfop-methyl, and quizalofop-ethyl, metamifop.

(17) Trione oxime herbicidal compounds

alloxydim-sodium, sethoxydim, butroxydim, clethodim, cloproxydim, cycloxydim, tepraloxydim, tralkoxydim, and profoxydim.

(18) Sulfonyl urea herbicidal compounds

chlorsulfuron, sulfometuron-methyl, metsulfuron-methyl, chlorimuron-ethyl, tribenuron-methyl, triasulfuron, bensulfuron-methyl, thifensulfuron-methyl, pyrazosulfuron-ethyl, primisulfuron-methyl, nicosulfuron, amidosulfuron, cinosulfuron, imazosulfuron, rimsulfuron, halosulfuron-methyl, prosulfuron, ethametsulfuron-methyl, triflusulfuron-methyl, flazasulfuron, cyclosulfamuron, flupyrsulfuron, sulfosulfuron, azimsulfuron, ethoxysulfuron, oxasulfuron, iodosulfuron-methyl-sodium, foramsulfuron, mesosulfuron-methyl, trifloxysulfuron, tritosulfuron, orthosulfamuron, flucetosulfuron, and propyrisulfuron.

(19) Imidazolinone herbicidal compounds

imazamethabenz-methyl, imazamethapyr, imazamox, imazapyr, imazaquin, and imazethapyr.

(20) Sulfonamide herbicidal compounds

flumetsulam, metosulam, diclosulam, florasulam, cloransulam-methyl, penoxsulam, and pyroxsulam.

(21) Pyrimidinyloxybenzoate herbicidal compounds

pyrithiobac-sodium, bispyribac-sodium, pyriminobac-methyl, pyribenzoxim, pyriftalid, and pyrimisulfan.

(22) Other herbicidal compounds

bentazon, bromacil, terbacil, chlorthiamid, isoxaben, dinoseb, amitrole, cinmethylin, tridiphane, dalapon, diflufenzopyr-sodium, dithiopyr, thiazopyr, flucarbazone-sodium, propoxycarbazone-sodium, mefenacet, flufenacet, fentrazamide, cafenstrole, indanofan, oxaziclomefone, benfuresate, ACN, pyridate, chloridazon, norflurazon, flurtamone, diflufenican, picolinafen, beflubutamid, clomazone, amicarbazone, pinoxaden, pyraclonil, pyroxasulfone, thiencarbazone-methyl, aminocyclopyrachlor, ipfencarbazone, and methiozolin.

Active Ingredients of Synergist

piperonyl butoxide, sesamex, sulfoxide, N-(2-ethylhexyl)-8,9,10-trinorborn-5-ene-2,3-dicarboximide (MGK 264), N-declyimidazole), WARF-antiresistant, TBPT, TPP, IBP, PSCP, methyl iodide ($CH_3I$), t-phenylbutenone, diethylmaleate, DMC, FDMC, ETP, and ETN.

EXAMPLES

Hereinbelow, the present invention will be further described in detail by production examples, formulation examples, test examples, and the like. However, the present invention is not limited to these examples.
First, the production examples for the production of the compounds of the present invention are shown below.

Production Example 1 (1)

A mixture of 5.79 g of $N^2$-methyl-5-trifluoromethylpyridine-2,3-diamine synthesized by a method described in

WO2010-125985, 5.0 g of 4-cyano-2-fluorobenzoic acid, 6.10 g of EDAC, 0.40 g of HOBt and 60 ml of pyridine was stirred at 60°C for 4 hours. Water was added to the cooled reaction mixture, and the mixture was extracted with ethyl acetate. The organic layer was washed with water, and then dried over anhydrous magnesium sulfate and concentrated under reduced pressure to obtain 10.46 g of 4-cyano-2-fluoro-N-(2-methylamino-5-trifluoromethylpyridin-3-yl) benzamide.

4-Cyano-2-fluoro-N-(2-methylamino-5-trifluoromethylpyridin-3-yl)benzamide

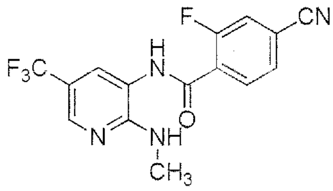

$^1$H-NMR($CDCl_3$)$\delta$:8.40(1H,s),8.29(1H,t),8.04-7.93(1H,m),7.74(1 H,d),7.66(1H,dd),7.56(1H,dd),4.96(1H,brs),3.08(3H,d) Production Example 1 (2)

A mixture of 10.46 g of 4-cyano-2-fluoro-N-(2-methylamino-5-trifluoromethylpyridin-3-yl)benzamide obtained above and 62 ml of acetic acid was heated and stirred at 130°C for 8 hours: The cooled reaction mixture was added to water, and the precipitated solid was obtained by filtration, and dried under reduced pressure to obtain 9.27 g of 2-(4-cyano-2-fluorophenyl)-3-methyl-6-trifluoromethyl-3H-im idazo[4,5-b] pyridine.

2-(4-Cyano-2-fluorophenyl)-3-methyl-6-trifluoromethyl-3H-im idazo[4,5-b] pyridine

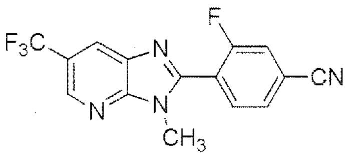

$^1$H-NMR($CDCl_3$)$\delta$:8.76(1H,d), 8.36(1H,d),7.92(1H,dd),7.71(1H,dd ),7.63(1H,dd),3.92(3H,d)

Production Example 1 (3)

1.95 ml of ethyl mercaptan was added to a mixture of 51 ml of DMF and 1.08 g of 60% sodium hydride (oil-based) under ice cooling, and the mixture was stirred at room temperature for 30 minutes. The reaction mixture was ice-cooled, and 8.25 g of 2-(4-cyano-2-fluorophenyl)-3-methyl-6-trifluoromethyl-3H-im idazo[4,5-b] pyridine obtained above was added thereto, and then the mixture was stirred at room temperature for 1 hour. Water was added to the reaction mixture, and the mixture was extracted with ethyl acetate. The organic layer was washed with water and dried over anhydrous magnesium sulfate, and then concentrated under reduced pressure. The resulting crude product was subjected to a silica gel column chromatography to obtain 9.05 g of 2-(2-ethylsulfanyl-4-cyanophenyl)-3-methyl-6-trifluoromethy l-3H-imidazo[4,5-b] pyridine.

2-(2-Ethylsulfanyl-4-cyanophenyl)-3-methyl-6-trifluoromethy l-3H-imidazo[4,5-b] pyridine

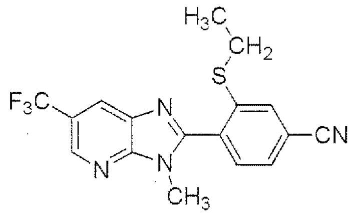

$^1$H-NMR($CDCl_3$)δ:8.75(1H,d),8.35(1H,d),7.70(1H,d),7.62(1H,dd) ,7.56(1H,d),3.79(3H,s),2.96(2H,q),1.31(3H,t)

Production Example 1 (4)

13.5 g of m-chloroperoxybenzoic acid (purity.of 65% or more) was added to a mixture of 9.01 g of 2-(2-ethylsulfanyl-4-cyanophenyl)-3-methyl-6-trifluoromethy l-3H-imidazo[4,5-b] pyridine and 125 ml of chloroform, under ice cooling, and then the mixture was stirred at room temperature for 10 hours. A 10% aqueous sodium thiosulfate solution and a saturated sodium bicarbonate solution were added to the reaction mixture, and the mixture was extracted with chloroform. The organic layer was washed with water and dried over anhydrous magnesium sulfate, and then concentrated under reduced pressure to obtain 9.13 g of 2-(2-ethylsulfonyl-4-cyanophenyl)-3-methyl-6-trifluoromethy l-3H-imidazo[4,5-b] pyridine.

2-(2-Ethylsulfonyl-4-cyanophenyl)-3-methyl-6-trifluoromethy l-3H-imidazo[4,5-b] pyridine

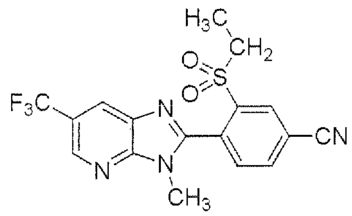

$^1$H-NMR($CDCl_3$)δ:8.77(1H,dd),8.52(1H,d),8.31(1H,d),8.12(1H,dd ),7.73(1H,d),3.74(3H,s),3.50(2H,q),1.29(3H,t)

Production Example 1 (5)

6 ml of concentrated sulfuric acid was added to a mixture of 3 g of 2-(2-ethylsulfonyl-4-cyanophenyl)-3-methyl-6-trifluoromethy l-3H-imidazo[4,5-b] pyridine and 2 ml of water, under ice cooling, and then the mixture was stirred under heat refluxing for 6 hours. Water was added to the reaction mixture cooled to room temperature, and sodium hydroxide was further added under cooling, and then the precipitated solid was obtained by filtration and washed with water. The resulting solid was dried under reduced pressure to obtain 3.21 g of 3-ethylsulfonyl-4-(3-methyl-6-trifluoromethyl-3H-imidazo[4, 5-b] pyridin-2-yl)benzoate (hereinafter, referred to as Compound of Present Invention 1-1).

Compound of Present Invention 1-1

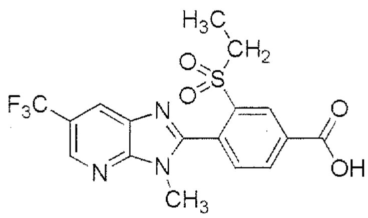

$^1$H-NMR($CDCl_3$)δ:8.90(1H,d),8.79(1H,d),8.54(1H,dd),8.34(1H,d) ,7.72(1H,d),3.75(3H,s),3.46(2H,q),1.30(3H,t)

Production Example 2 (1)

0.37 ml of oxalyl chloride was added to a mixture of Compound of Present Invention 1-1 (1.0 g), 24 ml of chloroform and 0.1 ml of DMF under ice cooling. The reaction mixture was stirred at room temperature for 2 hours to obtain 24 ml of a chloroform solution (0.1 mol/l) of 3-ethylsulfonyl-4-(3-methyl-6-trifluoromethyl-3H-imidazo[4, 5-b] pyridin-2-yl)benzoyl chloride.

3-Ethylsulfonyl-4-(3-methyl-6-trifluoromethyl-3H-imidazo[.4, 5-b] pyridin-2-yl)benzoyl chloride

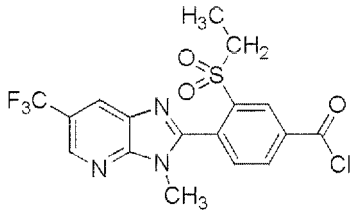

Production Example 2 (2)

2 ml of methanol was added to 8 ml of the chloroform solution (0.1 mol/l) of 3-ethylsulfonyl-4-(3-methyl-6-trifluoromethyl-3H-imidazo[4, 5-b] pyridin-2-yl)benzoyl chloride obtained above, under ice cooling, and the mixture was stirred at room temperature for 1 day. Water and a saturated aqueous sodium bicarbonate solution were added to the reaction mixture, and the mixture was extracted with chloroform. The aqueous layer was extracted twice with ethyl acetate, and combined with the organic layer, and the mixture was dried over anhydrous sodium sulfate and then concentrated under reduced pressure, and the resulting crude product was subjected to a silica gel column chromatography to obtain 0.21 g of methyl 3-ethylsulfonyl-4-(3-methyl-6-trifluoromethyl-3H-imidazo[4, 5-b] pyridin-2-yl)benzoate (hereinafter, referred to as Compound of Present Invention 1-2).

Compound of Present Invention 1-2

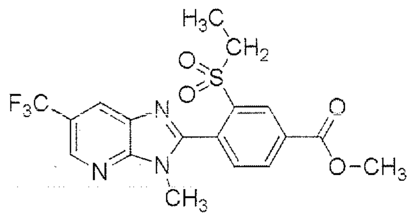

$^1H$-NMR($CDCl_3$)δ:8.85(1H,d),8.76(1H,d),8.49(1H,dd),8.29(1H,d) ,7.67(1H,d),4.04(3H,s),3.73(3H,s),3.46(2H,q),1.28(3H,t)

Production Example 3

2 ml of ethanol was added to 8 ml of the chloroform solution (0.1 mol/l) of 3-ethylsulfonyl-4-(3-methyl-6-trifluoromethyl-3H-imidazo[4, 5-b] pyridin-2-yl)benzoyl chloride, under ice cooling, and the mixture was stirred at room temperature for 1 day. Water and a saturated aqueous sodium bicarbonate solution were added to the reaction mixture, and the mixture was extracted with chloroform. The aqueous layer was extracted twice with ethyl acetate, and combined with the organic layer, and the mixture was dried over anhydrous sodium sulfate and then concentrated under reduced pressure, and the resulting crude product was subjected to a silica gel column chromatography to obtain 0.20 g of ethyl 3-ethylsulfonyl-4-(3-methyl-6-trifluoromethyl-3H-imidazo[4, 5-b] pyridin-2-yl)benzoate (hereinafter, referred to as Compound of Present Invention 1-3).

Compound of Present Invention 1-3

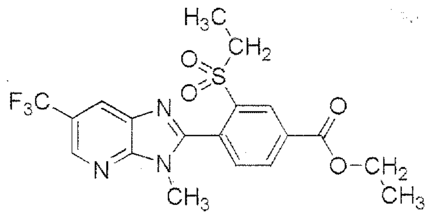

$^1$H-NMR($CDCl_3$)δ:8.84(1H,d),8.76(1H,dd),8.50(1H,dd),8.30(1H,d d),7.67(1H,d),4.50(2H,q),3.72(3H,s),3.47(2H,q),1.47(3H,t),1 .29(3H,t)

Production Example 4

5 ml of a 40% aqueous dimethylamine solution was added to 8 ml of the chloroform solution (0.1 mol/l) of 3-ethylsulfonyl-4-(3-methyl-6-trifluoromethyl-3H-imidazo[4, 5-b] pyridin-2-yl)benzoyl chloride, under ice cooling, and the mixture was stirred at room temperature for 1 day. Water and a saturated aqueous sodium bicarbonate solution were added to the reaction mixture, and the mixture was extracted with chloroform. The aqueous layer was extracted twice with ethyl acetate, and combined with the organic layer, and the mixture was dried over anhydrous sodium sulfate and then concentrated under reduced pressure, and the resulting crude product was subjected to a silica gel column chromatography to obtain 0.23 g of 3-ethylsulfonyl-4-(3-methyl-6-trifluoromethyl-3H-imidazo[4, 5-b] pyridin-2-yl)-N,N-dimethylbenzamide (hereinafter, referred to as Compound of Present Invention 1-4).

Compound of Present Invention 1-4

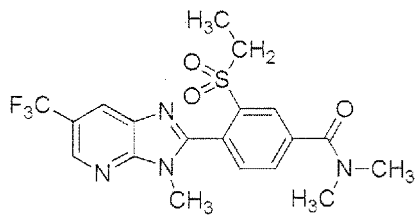

$^1$H-NMR($CDCl_3$)δ:8.75(1H,brs),8.29(1H,d),8.26(1H,d),7.92(1H,d d),7.64(1H,d),3.74(3H,s),3.47(2H,q),3.19(3H,s),3.07(3H,s),1 .27(3H,t)

Production Example 5 (1)

0.73 ml of oxalyl chloride was added to a mixture of Compound of Present Invention 1-1 (1.95 g), 24 ml of chloroform and 0.1 ml of DMF, under ice cooling. The reaction mixture was stirred at room temperature for 5 hours to obtain 24 ml of a chloroform solution (0.2 mol/l) of 3-ethylsulfonyl-4-(3-methyl-6-trifluoromethyl-3H-imidazo[4, 5-b] pyridin-2-yl)benzoyl chloride.

Production Example 5 (2)

0.50 g of N,O-dimethylhydroxylamine hydrochloride and 4.1 ml of diisopropylethylamine were added to 24 ml of the chloroform solution (0.2 mol/l) of 3-ethylsulfonyl-4-(3-methyl-6-trifluoromethyl-3H-imidazo[4, 5-b] pyridin-2-yl)benzoyl chloride obtained above, under ice cooling, and the mixture was stirred at room temperature for 1 day. Water and a saturated aqueous sodium bicarbonate solution were added to the reaction mixture, and the mixture was extracted with chloroform. The aqueous layer was extracted twice with ethyl acetate, and combined with the organic layer, and the mixture was dried over anhydrous sodium sulfate and then concentrated under reduced pressure, and the resulting crude product was subjected to a silica gel column chromatography to obtain 1.54 g of 3-ethylsulfonyl-4-(3-methyl-6-trifluoromethyl-3H-imidazo[4, 5-b] pyridin-2-yl)-N-methoxy-N-methylbenzamide (hereinafter, referred to as Compound of Present Invention 1-5).

Compound of Present Invention 1-5

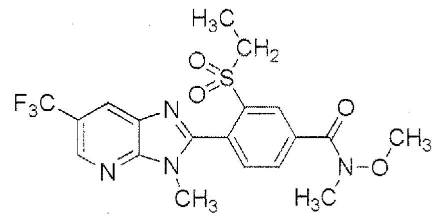

$^1H$-NMR($CDCl_3$)δ:8.76(1H,dd),8.54(1H,d),8.29(1H,d),8.15(1H,dd ),7.63(1H,d),3.75(3H,s),3.63(3H,s),3.50-3.41(5H,m),1.28(3H, t)

The compounds described in the production examples described above and the compounds produced by production methods according to the methods described in the production examples described above are shown in the tables.

The compounds of the present invention represented by the formula (1).

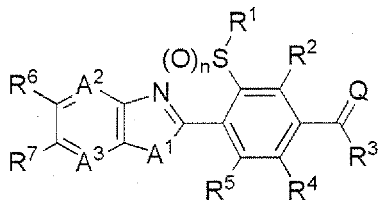
(1)

In the formula, $A^1$, $A^2$, $A^3$, $R^1$, $R^2$, $R^3$, $R^4$, $R^5$, $R^6$, $R^7$, $R^8$, Q and n represent the combinations shown in [Table 25] to [Table 26] shown below.

[Table 25]

| Compound of Present Invention | $A^1$ | $A^2$ | $A^3$ | $R^1$ | $R^2$ | $R^3$ | $R^4$ | $R^5$ | $R^6$ | $R^7$ | Q | n |
|---|---|---|---|---|---|---|---|---|---|---|---|---|
| 1-1 | NMe | CH | N | Et | H | OH | H | H | $CF_3$ | H | O | 2 |
| 1-2 | NMe | CH | N | Et | H | OMe | H | H | $CF_3$ | H | O | 2 |
| 1-3 | NMe | CH | N | Et | H | OEt | H | H | $CF_3$ | H | O | 2 |
| 1-4 | NMe | CH | N | Et | H | NMe2 | H | H | $CF_3$ | H | O | 2 |
| 1-5 | NMe | CH | N | Et | H | N(Me)OMe | H | H | $CF_3$ | H | O | 2 |
| 1-6 | NMe | CH | N | Et | H | Me | H | H | $CF_3$ | H | O | 2 |
| 1-7 | NMe | CH | CH | Et | H | OMe | H | H | $CF_3$ | H | O | 2 |
| 1-8 | NMe | CH | CH | Et | H | OEt | H | H | $CF_3$ | H | O | 2 |
| 1-9 | NMe | CH | CH | Et | H | NMe2 | H | H | $CF_3$ | H | O | 2 |
| 1-10 | NMe | CH | CH | Et | H | Me | H | H | $CF_3$ | H | O | 2 |
| 1-11 | O | CH | N | Et | H | OMe | H | H | $CF_3$ | H | O | 2 |
| 1-12 | O | CH | N | Et | H | OEt | H | H | $CF_3$ | H | O | 2 |
| 1-13 | O | CH | N | Et | H | NMe2 | H | H | $CF_3$ | H | O | 2 |
| 1-14 | O | CH | N | Et | H | Me | H | H | $CF_3$ | H | O | 2 |
| 1-15 | O | CH | CH | Et | H | OMe | H | H | $CF_3$ | H | O | 2 |
| 1-16 | O | CH | CH | Et | H | OEt | H | H | $CF_3$ | H | O | 2 |
| 1-17 | O | CH | CH | Et | H | NMe2 | H | H | $CF_3$ | H | O | 2 |

(continued)

| Compound of Present Invention | $A^1$ | $A^2$ | $A^3$ | $R^1$ | $R^2$ | $R^3$ | $R^4$ | $R^5$ | $R^6$ | $R^7$ | Q | n |
|---|---|---|---|---|---|---|---|---|---|---|---|---|
| 1-18 | O | CH | CH | Et | H | Me | H | H | $CF_3$ | H | O | 2 |
| 1-19 | S | CH | N | Et | H | OMe | H | H | $CF_3$ | H | O | 2 |
| 1-20 | S | CH | N | Et | H | OEt | H | H | $CF_3$ | H | O | 2 |

[Table 26]

| Compound of Present Invention | $A^1$ | $A^2$ | $A^3$ | $R^1$ | $R^2$ | $R^3$ | $R^4$ | $R^5$ | $R^6$ | $R^7$ | Q | n |
|---|---|---|---|---|---|---|---|---|---|---|---|---|
| 1-21 | S | CH | N | Et | H | NMe2 | H | H | $CF_3$ | H | O | 2 |
| 1-22 | S | CH | N | Et | H | Me | H | H | $CF_3$ | H | O | 2 |
| 1-23 | S | CH | CH | Et | H | OMe | H | H | $CF_3$ | H | O | 2 |
| 1-24 | S | CH | CH | Et | H | OEt | H | H | $CF_3$ | H | O | 2 |
| 1-25 | S | CH | CH | Et | H | NMe2 | H | H | CF3 | H | O | 2 |
| 1-26 | S | CH | CH | Et | H | Me | H | H | $CF_3$ | H | O | 2 |

In [Table 25] to [Table 26] above, Me represents a methyl group, and Et represents an ethyl group.

$^1$H-NMR data of the compounds of the present invention shown in [Table 25] to [Table 26] are shown below.

Compound of Present Invention 1-8

$^1$H-NMR($CDCl_3$)δ:8.83(1H,d),8.46(1H,dd),8.07(1H,s),7.69-7.60( 2H,m),7.52(1H,d),4.49(2H,q),3.63(3H,s),3.44(2H,q),1.47 (3H,t ),1.26(3H,t)

Compound of Present Invention 1-9

$^1$H-NMR($CDCl_3$)δ:8.25(1H,d),8.06(1H,s),7.89(1H,dd),7.66-7.60( 2H,m),7.52(1H,d),3.65(3H,s),3.52-3.35(2H,m),3.19(3H,s) ,3.06 (3H,s),1.25(3H,t)

Compound of Present Invention 1-24

$^1$H-NMR($CDCl_3$)δ:8.86(1H,d),8.42(1H,dd),8.36-8.32(1H,m),8.10( 1H,d),7.79(1H,d),7.76-7.70(1H,m),4.49(2H,q),3.78(2H,q ),1.46 (3H,t),1.39(3H,t)

Compound of Present Invention 1-25

$^1$H-NMR($CDCl_3$)δ:8.37-8.32(1H,m),8.27(1H,d),8.09(1H,d),7.85(1 H,dd),7.77(1H,d),7.74-7.68(1H,m),3.79(2H,q),3.18(3H,s),3.05 (3H,s),1.38(3H,t)

Next, formulation examples of the compound of the present invention are shown. The part in the formulation example represents part by weight.

Formulation Example 1

10 parts of any one of Compounds of Present Invention 1-1 to 1-26 is dissolved in a mixture of 35 parts of xylene and 35 parts of DMF, 14 parts of polyoxyethylenestyrylphenyl ether and 6 parts of calcium dodecylbenzenesulfonate are added thereto. The mixture is mixed to obtain each emulsifiable concentrate. Formulation Example 2

4 parts of sodium lauryl sulfate, 2 parts of calcium lignosulfonate, 20 parts of synthetic hydrous silicon oxide fine powder and 54 parts of diatomaceous earth are mixed, and 20 parts of any one of Compounds of Present Invention

1-1 to 1-26 is further added thereto. The mixture is mixed to obtain each wettable powder.

Formulation Example 3

1 part of synthetic hydrous silicon oxide fine powder, 2 parts of calcium lignosulfonate, 30 parts of bentonite and 65 parts of kaolin clay are added to and mixed with 2 parts of any one of Compounds of Present Invention 1-1 to 1-26. Subsequently, an appropriate amount of water is added to this mixture, and the mixture is further stirred, granulated with a granulator, and forced-air dried to obtain each granule. Formulation Example 4

1 part of any one of Compounds of Present Invention 1-1 to 1-26 is dissolved in an appropriate amount of acetone, and 5 parts of synthetic hydrous silicon oxide fine powder, 0.3 parts of PAP and 93.7 parts of Fubasami clay are added thereto. The mixture is sufficiently stirred and mixed to evaporate and eliminate acetone to obtain each dust formulation. Formulation Example 5

35 parts of a mixture of polyoxyethylene alkyl ether sulfate ammonium salt and white carbon (weight ratio 1 : 1), 10 parts of any one of Compounds of Present Invention 1-1 to 1-26 and 55 parts of water are mixed, and finely pulverized by wet grinding method to obtain each flowable.

Formulation Example 6

0.1 parts of any one of Compounds of Present Invention 1-1 to 1-26 is dissolved in 5 parts of xylene and 5 parts of trichloroethane, and the mixture is mixed with 89.9 parts of deodorized kerosene to obtain each oil solution.

Formulation Example 7

10 mg of any one of Compounds of Present Invention 1-1 to 1-26 is dissolved in 0.5 ml of acetone, and this solution is applied to 5 g of solid feed powder for animal (solid feed powder for breeding CE-2, product of CLEA Japan, Inc.), and the mixture is uniformly mixed. Subsequently, acetone is evaporated to dryness to obtain each poisonous bait formulation.

Formulation Example 8

0.1 parts of any one of Compounds of Present Invention 1-1 to 1-26 and 49.9 parts of Neothiozol (Chuo Kasei Co., Ltd.) are filled into an aerosol can, and an aerosol valve is attached, then the container is filled with 25 parts of dimethyl ether and 25 parts of LPG and shaken, and an actuator is attached to obtain an oil-based aerosol.

Formulation Example 9

0.6 parts of any one of Compounds of Present Invention 1-1 to 1-26, 0.01 parts of BHT (2,6-di-tert-butyl-4-methylphenol), 5 parts of xylene, 3.39 parts of deodorized kerosene and 1 part of emulsifier {RHEODOL MO-60 (manufactured by Kao Corporation)} are mixed and dissolved, and the resulting solution and 50 parts of distilled water are filled into an aerosol container. A valve is attached to the container, and then 40 parts of a propellant (LPG) is filled under pressure through the valve to obtain an aqueous aerosol.

Formulation Example 10

0.1 g of any one of Compounds of Present Invention 1-1 to 1-26 is dissolved in 2 ml of propylene glycol, and a porous ceramic plate with a size of 4.0 cm x 4.0 cm and 1.2 cm in thickness is impregnated with the solution to obtain a heating type smoking agent.

Formulation Example 11

5 parts of any one of Compounds of Present Invention 1-1 to 1-26 and 95 parts of an ethylene-methyl methacrylate copolymer (a ratio of methyl methacrylate in the copolymer: 10% by weight, Acryft WD301, manufactured by SUMITOMO CHEMICAL Co., Ltd.) are melt-kneaded with a closed pressurizing kneader (manufactured by Moriyama Works), and the resulting kneaded matter is extruded from a molding machine through a molding die to obtain a rod-shaped molded body with a size of 15 cm in length and 3 mm in diameter.

Formulation Example 12

5 parts of any one of Compounds of Present Invention 1-1 to 1-26 and 95 parts of a soft vinyl chloride resin are melt-kneaded with a closed pressurizing kneader (manufactured by Moriyama Works), and the resulting kneadedmatter is extruded from a molding machine through a molding die to obtain a rod-shaped molded body with a size of 15 cm in length and 3 mm in diameter.

Formulation Example 13

100 mg of any one of Compounds of Present Invention 1-1 to 1-26, 68.75 mg of lactose, 237.5 mg of corn starch, 43.75 mg of microcrystalline cellulose, 18.75 mg of polyvinylpyrrolidone, 28.75 mg of sodium carboxymethyl starch and 2.5 mg of magnesium stearate are mixed, and the resulting mixture was compressed to an appropriate size to obtain a tablet.

Formulation Example 14

25 mg of any one of Compounds of Present Invention 1-1 to 1-26, 60 mg of lactose, 25 mg of corn starch, 6 mg of carmellose calcium and an appropriate amount of 5% hydroxypropyl methylcellulose, and the resulting mixture is filled into a hard shell gelatin capsule or a hydroxypropyl methylcellulose capsule to obtain an encapsulated formulation.

Formulation Example 15

Distilled water is added to 1000 mg of any one of Compounds of Present Invention 1-1 to 1-26, 500 mg of fumaric acid, 2000 mg of sodium chloride, 150 mg of methylparaben, 50 mg of propylparaben, 25000 mg of granulated sugar, 13000 mg of sorbitol (70% solution), 100 mg of Veegum K (Vanderbilt Co.), 35 mg of flavor and 500 mg of colorant, such that the final volume is 100 ml, and the mixture is mixed to obtain a suspension for oral administration.

Formulation Example 16

5 parts of any one of Compounds of Present Invention 1-1 to 1-26 is dissolved in a mixture of 5 parts of polysorbate 85, 3 parts of benzyl alcohol and 30 parts of propylene glycol, and a phosphate buffer is added to this solution so as to have a pH of 6.0 to 6.5, and then water is added to have a total amount of 100 parts to obtain a liquid formulation for oral administration.

Formulation Example 17

5 parts of aluminum distearate is dispersed in a mixture of 57 parts of fractionated palm oil and 3 parts of polysorbate 85 while heating. The dispersion is cooled to room temperature to obtain an oily vehicle, and 25 parts of saccharin is dispersed in the oily vehicle. Further, 10 parts of any one of Compounds of Present Invention 1-1 to 1-26 is added thereto to obtain a paste formulation for oral administration.

Formulation Example 18

5 parts of any one of Compounds of Present Invention 1-1 to 1-26 and 95 parts of limestone filler are mixed, and a granule for oral administration is obtained using wet granulation method.

Formulation Example 19

5 parts of any one of Compounds of Present Invention 1-1 to 1-26 is dissolved in 80 parts of diethylene glycol monoethyl ether, and 15 parts of propylene carbonate is mixed therewith to obtain a spot-on solution.

Formulation Example 20

10 parts of any one of Compounds of Present Invention 1-1 .. to 1-26 is dissolved in 70 parts of diethylene glycol monoethyl ether, and 20 parts of 2-octyl dodecanol is mixed therewith to obtain a pour-on solution.

Formulation Example 21

60 parts of NIKKOL TEALS-42 (Nikko Chemicals Co., Ltd., 42% aqueous solution of triethanolamine lauryl sulfate) and 20 parts of propylene glycol are added to 0.5 parts of any one of Compounds of Present Invention 1-1 to 1-26, and the mixture is sufficiently stirred and mixed until it becomes a uniform solution, and then 19.5 parts of water is added and further sufficiently stirred and mixed to obtain a shampoo agent as a uniform solution.

Formulation Example 22

0.15 parts of any one of Compounds of Present Invention 1-1 to 1-26, 95 parts of an animal feed and 4.85 parts of a mixture of secondary calcium phosphate, diatomaceous earth, Aerosil and carbonate (or chalk) are sufficiently stirred and mixed to obtain a feed premix for animal.

Formulation Example 23

7.2.g of any one of Compounds of Present Invention 1-1 to 1-26 and 92.8 g of VOSCO S-55 (manufactured by.Maruishi Pharmaceutical Co., Ltd.) are dissolved and mixed at 100°C, poured into a suppository mold, and cooled and solidified to obtain a suppository.
Next, the pest control effect of the compound of the present invention is shown as test examples.

Test Example 1

The formulations of Compounds of Present Invention 1-1, 1-2, 1-3, 1-4, 1-5, 1-8 and 1-24 as obtained in Formulation Example 5 were diluted with water, so as to have a concentration of the active ingredient of 500 ppm, to prepare a test drug solution.
On the other hand, on a cucumber seedling (the first true leaf stage) planted in a plastic cup was inoculated with about 30 Aphis gossypii (whole stage), and left for a day. 20 ml of the test drug solution was sprayed on the seedling.
Six days after spraying, the number of surviving Aphis gossypii parasitic on the leaves of the cucumber was examined, and the control value was calculated according to the following equation:

$$\text{Control value (\%)} = \{1 - (Cb \times Tai)/(Cai \times Tb)\} \times 100$$

wherein the symbols represent as follows:

Cb: the number of insects in a non-treated section before treatment
Cai: the number of surviving parasitic insects in a non-treated section on observation
Tb: the number of insects in a treated section before treatment
Tai: the number of surviving parasitic insects in a treated section on observation
wherein the non-treated section refers to a section where the test drug solution prepared by diluting the formulation obtained as in Formulation Example 5 but not containing the compound of the present invention with the same amount of water as in the treated section was sprayed.

As a result, in the treated section where the test drug solution containing each of Compounds of Present Invention 1-1, 1-2, 1-3, 1-4, 1-5, 1-8 and 1-24 was used, the control value was 90% or more.

Test Example 2

The formulations of Compounds of Present Invention 1-1, 1-2 and 1-3 as obtained in Formulation Example 5 were diluted with water, so as to have a concentration of the active ingredient of 500 ppm, to prepare a test drug solution.
On the other hand, a cucumber seedling (the second true leaf stage) planted in a plastic cup was drenched at its foot with 5 ml of the test drug solution, and kept in a greenhouse at 25°C for 7 days. On the cucumber leaf surface was inoculated about 30 Aphis gossypii (whole stage), and further kept in the greenhouse for 6. days, then the number of surviving Aphis gossypii parasitic on the leaves of the cucumber was examined, and the control value was calculated according to the following equation:

$$\text{Control value (\%)} = \{1 - (Cb \times Tai)/(Cai \times Tb)\} \times 100$$

wherein the symbols represent as follows:

Cb: the number of insects in a non-treated section before treatment
Cai: the number of surviving parasitic insects in a non-treated section on observation
Tb: the number of insects in a treated section before treatment
Tai: the number of surviving parasitic insects in a treated section on observation
wherein the non-treated section refers to a section where the test drug solution prepared by diluting the formulation obtained as in Formulation Example 5 but not containing the compound of the present invention with the same amount of water as in the treated section was sprayed.

As a result, in the treated section where the test drug solution containing each of Compounds of Present Invention 1-1, 1-2 and 1-3 was used, the control value was 90% or more.

Test Example 3

The formulation of the compound of the present invention as obtained in Formulation Example 5 is diluted with water, so as to have a concentration of the active ingredient of 500 ppm, to prepare a test drug solution.

On a rice seedling in the second leaf stage planted in a polyethylene cup is sprayed 10 ml of each test drug solution. After air-drying, 20 third-fourth instar larvae of Nilaparvata lugens are released, and kept in the greenhouse at 25°C. After 6 days, the number of surviving Nilaparvata lugens parasitic on the rice is examined, and the control value is calculated according to the following equation, and then the sufficient control value is obtained.

$$\text{Control value (\%)} = \{1 - (Cb \times Tai)/(Cai \times Tb)\} \times 100$$

wherein the symbols represent as follows.

Cb: the number of insects in a non-treated section before treatment
Cai: the number of surviving parasitic insects in a non-treated section on observation
Tb: the number of insects in a treated section before treatment
Tai: the number of surviving parasitic insects in a treated section on observation

Here, the non-treated section refers to a section where the test drug solution prepared by diluting the formulation obtained as in Formulation Example 5 but not containing the compound of the present invention with the same amount of water as in the treated section was sprayed.

Test Example 4

The formulation of the compound of the present invention as obtained in Formulation Example 5 is diluted with water, so as to have a concentration of the active ingredient of 500 ppm, to prepare a test drug solution.

On the other hand, a rice seedling (2 weeks after sowing, the second leaf stage) planted in a plastic cup is drenched at its foot with 5 ml of each test drug solution, and kept in a greenhouse at 25°C for 7 days. 20 third-fourth instar larvae of Nilaparvata lugens are released, and further kept in the greenhouse for 6 days, and then the number of surviving Nilaparvata lugens parasitic on the rice leaves is examined. The control value is calculated according to the following equation, and then the sufficient control value is obtained.

$$\text{Control value (\%)} = \{1 - (Cb \times Tai)/(Cai \times Tb)\} \times 100$$

wherein the symbols represent as follows.

Cb: the number of insects in a non-treated section before treatment
Cai: the number of surviving parasitic insects in a non-treated section on observation
Tb: the number of insects in a treated section before treatment

Tai: the number of surviving parasitic insects in a treated section on observation

Here, the non-treated section refers to a section where the test drug solution prepared by diluting the formulation obtained as in Formulation Example 5 but not containing the compound of the present invention with the same amount of water as in the treated section was sprayed.

Test Example 5

The formulation of the compound of the present invention as obtained in Formulation Example 5 is diluted with water, so as to have a concentration of the active ingredient of 500 ppm, to prepare a test drug solution.

On the other hand, Bemisia tabaci adult is released on a tomato seedling (the third true leaf stage) planted in a polyethylene cup, and made to lay eggs for about 72 hours. The tomato seedling is kept in a greenhouse for 8 days, and when instar larvae hatch from the eggs, the above test drug solution is sprayed at a rate of 20 ml/cup, and the cup is kept in a greenhouse at 25°C. After 7 days, the number of surviving instar larvae on the tomato leaves is examined, and the control value is calculated according to the following equation. The sufficient control value is obtained.

$$\text{Control value } (\%) = \{1 - (Cb \times Tai)/(Cai \times Tb)\} \times 100$$

wherein the symbols represent as follows.

Cb: the number of instar larvae in a non-treated section before treatment
Cai: the number of surviving instar larvae in a non-treated section on observation
Tb: the number of instar larvae in a treated section before treatment
Tai: the number of surviving instar larvae in a treated section on observation

Here, the non-treated section refers to a section where the test drug solution prepared by diluting the formulation obtained as in Formulation Example 5 but not containing the compound of the present invention with the same amount of water as in the treated section was sprayed.

Test Example 6

The formulations of Compounds of Present Invention 1-2, 1-3, 1-4, 1-5 and 1-25 obtained in Formulation Example 5 were diluted with water so as to have a concentration of the active ingredient of 500 ppm to prepare a test drug solution.

On the other hand, on cabbage at the third leaf stage planted in a polyethylene cup was sprayed, at a rate 20 mL/cup, the test drug solution. After the drug solution was dried, the foliage part was cut off, and then placed in a 50 mL volume cup. Five second instar larvae of Plutella xylostella were released into the cup, and the cup was sealed with a lid. After the cup was kept at 25°C for 5 days, the number of dead insects was counted. The death rate was calculated according to the following equation.

$$\text{Death rate } (\%) = (\text{Number of dead insects}/\text{Number of tested insects}) \times 100$$

As a result, in the treated-section where each test drug solution of Compounds of Present Invention 1-2, 1-3, 1-4, 1-5 and 1-25 was used, the death rate was 80% or more.

Test Example 7

The formulations of Compounds of Present Invention 1-2 and 1-3 as obtained in Formulation Example 5 were diluted with water, so as to have a concentration of the active ingredient of 500 ppm, to prepare a test spray solution.

On the other hand, an apple tree was planted in a plastic cup, and grown until the seventh-eighth true leaf was spread. To the apple tree was sprayed, at a rate of 20 mL/cup, the test drug solution. After the drug solution was dried, 60 first-instar larvae of Adoxophyes orana fasciata were released, and covered with a plastic cup the bottom of which was cut off and on which a filter paper was put, with the plastic cup cover placed upside-down. After 7 days, the number

of dead insects was counted, and the death rate was calculated according to the following equation.

Death rate (%) = (Number of dead insects/Number of tested insects) × 100

As a result, in the treated-section where each test drug solution of Compounds of Present Invention 1-2 and 1-3 was used, the death rate was 90% or more.

Test Example 8

The formulation of Compound of Present Invention 1-2 as obtained in Formulation Example 5 was diluted with water, so as to have a concentration of the active ingredient of 500 ppm, to prepare a test drug solution.
A filter paper having a diameter of 5.5 cm was spread on the bottom of a polyethylene cup having the same diameter and 0.7 ml of the test drug solution was added dropwise onto the filter paper, and 30 mg of sucrose was uniformly placed as bait. Into the polyethylene cup, 10 female imagoes of Musca domestica were released, and the cup was sealed with a lid. After 24 hours, the life and death of Musca domestica was examined, the number of dead insects was counted, and the death rate was calculated according to the following equation.

Death rate (%) = (Number of dead insects/Number of tested insects) × 100

As a result, in the treated section where the test drug solution containing Compound of Present Invention 1-2 was used, the death rate was 100%.

Test Example 9

The formulation of the compound of the present invention as obtained in Formulation Example 5 is diluted with water, so as to have a concentration of the active ingredient of 500 ppm, to prepare a test drug solution.
A filter paper having a diameter of 5.5 cm is spread on the bottom of a polyethylene cup having the same diameter and 0.7 ml of the test drug solution is added dropwise onto the filter paper, and 30 mg of sucrose is uniformly placed as bait. Into the polyethylene cup, 2 male imagoes of Blattella germanica are released, and the cup is sealed with a lid. After 6 days, the life and death of Blattella germanica is examined, and the number of dead insects is counted. The death rate is calculated according to the following equation, and then the sufficient death rate is obtained.

Death rate (%) = (Number of dead insects/Number of tested insects) × 100

Test Example 10

The formulations of Compounds of Present Invention 1-2, 1-3 and 1-8 as obtained in Formulation Example 5 were diluted with water, so as to have a concentration of the active ingredient of 500 ppm, to prepare a test drug solution.
0.7 ml of the test drug solution was added to 100 ml of ion-exchanged water (active ingredient concentration: 3.5 ppm). 20 last-instar larvae of Culex pipiens pallens were released into the solution. One day later, the life and death of the Culex pipiens pallens was examined, and the number of dead insects was counted to calculate the death rate.

Death rate (%) = (Number of dead insects/Number of tested insects) × 100

As a result, in the treated-section where each test drug solution of Compounds of Present Invention 1-2, 1-3 and 1-8 was used, the death rate was 95% or more.

Test Example 11

Compound of Present Invention 1-24 (2 mg) was weighed in a screw tube (Maruemu No. 5; 27 x 55 mm), and 0.2 mL of acetone was added thereto, and the screw tube was sealed with a cap to dissolve the compound. The screw tube was rotated and inverted to uniformly coat the drug solution onto the whole inner wall of the tube. After removing the cap, the solution was air-dried for about 2 hours, then unfed nymphal ticks, Haemaphysalis longicornis (5 ticks/group) were released, and the tube was sealed with the cap. After 2 days, the number of dead insects was counted, and the death rate was calculated according to the following equation.

Death rate (%) = 100 × (Number of dead insects/Number of tested insects)

As a result, in the treated section where the test drug solution containing Compound of Present Invention 1-24 was used, the death rate was 100%.

INDUSTRIAL APPLICABILITY

The compound of the present invention has a control effect on pests and is useful as an active ingredient of a pest control agent.

**Claims**

**1.** A fused heterocyclic compound represented by formula (1):

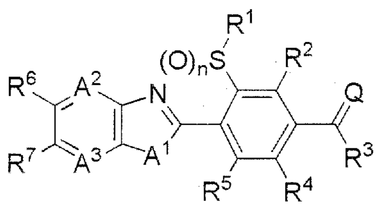

(1)

wherein

$A^1$ represents $NR^8$, S or 0;
$A^2$ represents N or $CR^9$;
$A^3$ represents N or $CR^{10}$;
Q represents O or S;
$R^1$ represents a C1 to C6 chain hydrocarbon group optionally having one or more atoms or groups selected from group $\alpha$ or a C3 to C6 cycloalkyl group optionally having one or more halogen atoms or one or more C1 to C3 alkyl groups;
$R^2$, $R^4$ and $R^5$ are the same or different and represent a C1 to C6 chain hydrocarbon group optionally having one or more atoms or groups selected from group $\alpha$, $NR^{11}R^{12}$, $S(O)_mR^{11}$, $OP^{11}$, a cyano group, a nitro group, a halogen atom or a hydrogen atom;
$R^3$ represents a group represented by group J1, group J2 or group J3 of the following formulae:

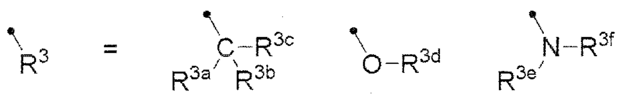

(J1) (J2) (J3)

wherein
$R^{3a}$, $R^{3b}$ and $R^{3c}$ are the same or different and represents a C1 to C6 alkyl group optionally having one or more halogen atoms, a halogen atom or a hydrogen atom,

$R^{3d}$ represents a C1 to C6 chain hydrocarbon group optionally having one or more atoms or groups selected from group β, a C3 to C9 cycloalkyl group optionally having one or more halogen atoms or one or more C1 to C3 alkyl groups, a phenyl group optionally having one or more atoms or groups selected from group γ, a 5- or 6-membered heterocyclic group optionally having one or more atoms or groups selected from group γ or a hydrogen atom, and

$R^{3e}$ represents a C1 to C6 chain hydrocarbon group optionally having one or more halogen atoms or a hydrogen atom and

$R^{3f}$ represents a C1 to C6 chain hydrocarbon group optionally having one or more atoms or groups selected from group β, a C3 to C9 cycloalkyl group optionally having one or more halogen atoms or one or more C1 to C3 alkyl groups, a phenyl group optionally having one or more atoms or groups selected from group γ, a 5- or 6-membered heterocyclic group optionally having one or more atoms or groups selected from group γ, $NR^{11}R^{12}$, $OR^{11}$, $C(O)R^{11}$, $CO_2R^{11}$ or a hydrogen atom, or

$R^{3e}$ and $R^{3f}$, together with the nitrogen atom to which they are bound, may form a 5- or 6-membered heterocyclic ring optionally having one or more atoms or groups selected from group y;

$R^6$ and $R^7$ are the same or different and represent a C1 to C6 chain hydrocarbon group optionally having one or more atoms or groups selected from group α, $OR^{11}$, $S(O)_mR^{11}$, $SF_5$, a halogen atom or a hydrogen atom (wherein $R^6$ and $R^7$ do not represent a hydrogen atom at the same time);

$R^8$ represents a C1 to C6 chain hydrocarbon group optionally having one or more atoms or groups selected from group β, a C3 to C6 cycloalkyl group optionally having one or more halogen atoms or one or more C1 to C3 alkyl groups, $C(O)R^{11}$, $CO_2R^{11}$ or a hydrogen atom;

$R^9$ and $R^{10}$ are the same or different and represent a C1 to C6 alkyl group optionally having one or more halogen atoms, $NR^{11}R^{12}$, $S(O)_mR^{11}$, $OR^{11}$, $C(O)R^{11}$, $CO_2R^{11}$, a cyano group, a halogen atom or a hydrogen atom;

$R^{11}$ and $R^{12}$ are the same or different and represent a C1 to C6 chain hydrocarbon group optionally having one or more atoms or groups selected from group α or a hydrogen atom (wherein, in $S(O)_mR^{11}$, when m is 1 or 2, $R^{11}$ does not represent a hydrogen atom);

n represents 0, 1 or 2; and

m represents 0, 1 or 2;

Group α: a group consisting of C1 to C6 alkoxy groups optionally having one or more halogen atoms, C1 to C6 alkylsulfanyl groups optionally having one or more halogen atoms, C1 to C6 alkylsulfinyl groups optionally having one or more halogen atoms, C1 to C6 alkylsulfonyl groups optionally having one or more halogen atoms, C2 to C6 alkylcarbonyl groups optionally having one or more halogen atoms, C2 to C6 alkoxycarbonyl groups optionally having one or more halogen atoms, C3 to C6 cycloalkyl groups optionally having one or more halogen atoms or one or more C1 to C3 alkyl groups, cyano groups, hydroxy groups, and halogen atoms,

Group β: a group consisting of C1 to C6 alkoxy groups optionally having one or more halogen atoms, C1 to C6 alkylsulfanyl groups optionally having one or more halogen atoms, C1 to C6 alkylsulfinyl groups optionally having one or more halogen atoms, C1 to C6 alkylsulfonyl groups optionally having one or more halogen atoms, C2 to C6 alkylcarbonyl groups optionally having one or more halogen atoms, C2 to C6 alkoxycarbonyl groups optionally having one or more halogen atoms, C3 to C6 cycloalkyl groups optionally having one or more halogen atoms or one or more C1 to C3 alkyl groups, phenyl groups optionally having one or more atoms or groups selected from group γ, 5- or 6-membered heterocyclic groups optionally having one or more atoms or groups selected from group γ, cyano groups, hydroxy groups, and halogen atoms,

Group γ: a group consisting of C1 to C3 alkyl groups optionally having one or more halogen atoms, C1 to C3 alkoxy groups optionally having one or more halogen atoms, C1 to C3 alkylamino groups optionally having one or more halogen atoms, C2 to C6 dialkylamino groups optionally having one or more halogen atoms, C1 to C3 alkylsulfanyl groups optionally having one or more halogen atoms, C1 to C3 alkylsulfinyl groups optionally having one or more halogen atoms, C1 to C3 alkylsulfonyl groups optionally having one or more halogen atoms, C2 to C4 alkylcarbonyl groups optionally having one or more halogen atoms, C2 to C4 alkoxycarbonyl groups optionally having one or more halogen atoms, nitro groups, amino groups, cyano groups, and halogen atoms, or an N-oxide thereof (in the case of the N-oxide, n represents 2, and m represents 2).

**2.** The compound according to claim 1, wherein

$R^1$ is a C1 to C6 alkyl group optionally having one or more halogen atoms or one or more cyclopropyl groups (wherein the cyclopropyl group optionally has one or more halogen atoms or one or more C1 to C3 alkyl groups), a C2 to C6 alkenyl group optionally having one or more halogen atoms, a C2 to C6 alkynyl group optionally having one or more halogen atoms or a C3 to C6 cycloalkyl group optionally having one or more halogen atoms or one or more C1 to C3 alkyl groups;

$R^2$, $R^4$ and $R^5$ are the same or different and are a C1 to C3 alkyl group optionally having one or more halogen

atoms, a halogen atom or a hydrogen atom;
$R^6$ is a C1 to C6 alkyl group optionally having one or more halogen atoms, $OR^{11a}$, $S(O)_mR^{11a}$ or a halogen atom;
$R^7$ is a C1 to C6 alkyl group optionally having one or more halogen atoms, $OR^{11a}$, $S(O)_mR^{11a}$, a halogen atom or a hydrogen atom;
$R^8$ is a C1 to C6 alkyl group optionally having one or more halogen atoms or one or more cyclopropyl groups (wherein the cyclopropyl group optionally has one or more halogen atoms or one or more C1 to C3 alkyl groups), a C2 to C6 alkenyl group optionally having one or more halogen atoms, a C2 to C6 alkynyl group optionally having one or more halogen atoms, a C1 two C3 alkyl group having a phenyl group optionally having one or more atoms or groups selected from group γ, a C1 to C3 alkyl group having a 5- or 6-membered heterocyclic group optionally having one or more atoms or groups selected from group γ, a C3 to C6 cycloalkyl group optionally having one or more halogen atoms or one or more C1 to C3 alkyl groups, $C(O)R^{11a}$, $CO_2R^{11a}$ or a hydrogen atom;
$R^9$ and $R^{10}$ are the same or different and are a C1 to C6 alkyl group optionally having one or more halogen atoms, $OR^{11a}$, $S(O)_mR^{11a}$, a halogen atom or a hydrogen atom; and
$R^{11a}$ is a C1 to C6 alkyl group optionally having one or more halogen atoms or a hydrogen atom (wherein, in $S(O)_mR^{11a}$, when m is 1 or 2, $R^{11a}$ does not represent a hydrogen atom).

**3.** The compound according to claim 1, wherein

$R^1$ is a C1 to C6 alkyl group optionally having one or more halogen atoms or one or more cyclopropyl groups (wherein the cyclopropyl group optionally has one or more halogen atoms or one or more C1 to C3 alkyl groups);
all of $R^2$, $R^4$ and $R^5$ are a hydrogen atom;
$R^6$ is a C1 to C6 perfluoroalkyl group, $OR^{11b}$, $S(O)_mR^{11b}$ or a halogen atom;
$R^7$ is a C1 to C6 perfluoroalkyl group, $OR^{11b}$, $S(O)_mR^{11b}$, a halogen atom or a hydrogen atom;
$R^8$ is a C1 to C6 alkyl group optionally having one or more halogen atoms, a C2 to C6 alkenyl group optionally having one or more halogen atoms, a C2 to C6 alkynyl group optionally having one or more halogen atoms, a C1 to C3 alkyl group having a thiazolyl group optionally having one or more atoms or groups selected from group γ or a C1 to C3 alkyl group having a pyridyl group optionally having one or more atoms or groups selected from group γ;
$R^9$ is a hydrogen atom;
$R^{10}$ is a C1 to C6 alkyl group optionally having one or more halogen atoms, $OR^{11c}$, $S(O)_mR^{11c}$, a halogen atom or a hydrogen atom;
$R^{11b}$ is a C1 to C6 perfluoroalkyl group; and
$R^{11c}$ is a C1 to C6 alkyl group optionally having one or more halogen atoms.

**4.** The compound according to any of claim 1 to claim 3, wherein $R^3$ is group J1.

**5.** The compound according to any of claim 1 to claim 3, wherein $R^3$ is group J2.

**6.** The compound according to any of claim 1 to claim 3, wherein $R^3$ is group J3.

**7.** The compound according to any of claim 1 to claim 6, wherein $A^1$ is $NR^8$.

**8.** The compound according to any of claim 1 to claim 6, wherein $A^1$ is S.

**9.** The compound according to any of claim 1 to claim 6, wherein $A^1$ is C.

**10.** A pest control agent comprising the compound as defined in any of claim 1 to claim 9, and an inert carrier.

**11.** A method for controlling pests comprising applying an effective amount of the compound as defined in any of claim 1 to claim 9 to a pest or a pest-infested area.

## INTERNATIONAL SEARCH REPORT

International application No.
PCT/JP2014/054597

A. CLASSIFICATION OF SUBJECT MATTER
See extra sheet.

According to International Patent Classification (IPC) or to both national classification and IPC

B. FIELDS SEARCHED

Minimum documentation searched (classification system followed by classification symbols)
C07D235/18, A01N43/50, A01N43/78, A01N43/90, A01P7/02, A01P7/04, A61K31/4184, A61K31/423, A61K31/437, A61P33/00, A61P33/14, C07D263/56, C07D277/66, C07D471/04, C07D498/04, C07D513/04

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2014 |
| Kokai Jitsuyo Shinan Koho | 1971-2014 | Toroku Jitsuyo Shinan Koho | 1994-2014 |

Electronic data base consulted during the international search (name of data base and, where practicable, search terms used)
CAplus/REGISTRY(STN)

C. DOCUMENTS CONSIDERED TO BE RELEVANT

| Category* | Citation of document, with indication, where appropriate, of the relevant passages | Relevant to claim No. |
|---|---|---|
| X<br>Y | WO 2012/086848 A1 (SUMITOMO CHEMICAL CO., LTD.),<br>28 June 2012 (28.06.2012),<br>entire text; particularly, claims<br>& JP 2013-136519 A & CA 2822919 A<br>& CN 103261170 A & TW 201234965 A<br>& AR 84588 A | 1-5,7-10<br>6 |
| Y | JP 2005-505524 A (Syngenta Ltd.),<br>24 February 2005 (24.02.2005),<br>entire text; particularly, claims<br>& US 2004/0209776 A1 & EP 1414818 A1<br>& WO 2003/011861 A1 & BR 211424 A<br>& CA 2452751 A & NZ 530431 A<br>& CN 1533389 A & ZA 200400577 A<br>& MX PA04000797 A | 6 |

☒ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 10 April, 2014 (10.04.14) | 22 April, 2014 (22.04.14) |
| Name and mailing address of the ISA/<br>Japanese Patent Office<br>Facsimile No. | Authorized officer<br><br>Telephone No. |

Form PCT/ISA/210 (second sheet) (July 2009)

**INTERNATIONAL SEARCH REPORT**

International application No.
PCT/JP2014/054597

C (Continuation). DOCUMENTS CONSIDERED TO BE RELEVANT

| Category* | Citation of document, with indication, where appropriate, of the relevant passages | Relevant to claim No. |
|---|---|---|
| A | WO 2013/018928 A1 (SUMITOMO CHEMICAL CO., LTD.), 07 February 2013 (07.02.2013), entire text & JP 2014-5263 A & TW 201321363 A | 1-10 |
| P,X | JP 2014-24840 A (Sumitomo Chemical Co., Ltd.), 06 February 2014 (06.02.2014), entire text; particularly, claims (Family: none) | 1-10 |
| P,X | WO 2013/191189 A1 (Sumitomo Chemical Co., Ltd.), 27 December 2013 (27.12.2013), entire text; particularly, claims (Family: none) | 1-5,7-10 |
| P,A | WO 2013/191188 A1 (Sumitomo Chemical Co., Ltd.), 27 December 2013 (27.12.2013), entire text (Family: none) | 1-10 |

Form PCT/ISA/210 (continuation of second sheet) (July 2009)

**INTERNATIONAL SEARCH REPORT**

International application No.
PCT/JP2014/054597

Continuation of A. CLASSIFICATION OF SUBJECT MATTER
(International Patent Classification (IPC))

*C07D235/18*(2006.01)i, *A01N43/50*(2006.01)i, *A01N43/78*(2006.01)i, *A01N43/90*(2006.01)i, *A01P7/02*(2006.01)i, *A01P7/04*(2006.01)i, *A61K31/4184*(2006.01)i, *A61K31/423*(2006.01)i, *A61K31/437*(2006.01)i, *A61P33/00*(2006.01)i, *A61P33/14*(2006.01)i, *C07D263/56*(2006.01)i, *C07D277/66*(2006.01)i, *C07D471/04*(2006.01)i, *C07D498/04*(2006.01)i, *C07D513/04*(2006.01)i

(According to International Patent Classification (IPC) or to both national classification and IPC)

Form PCT/ISA/210 (extra sheet) (July 2009)

**INTERNATIONAL SEARCH REPORT**

International application No.
PCT/JP2014/054597

**Box No. II Observations where certain claims were found unsearchable (Continuation of item 2 of first sheet)**

This international search report has not been established in respect of certain claims under Article 17(2)(a) for the following reasons:

1. ☒ Claims Nos.: 11
because they relate to subject matter not required to be searched by this Authority, namely:
(See extra sheet)

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

**Box No. III Observations where unity of invention is lacking (Continuation of item 3 of first sheet)**

This International Searching Authority found multiple inventions in this international application, as follows:

1. ☐ As all required additional search fees were timely paid by the applicant, this international search report covers all searchable claims.

2. ☐ As all searchable claims could be searched without effort justifying additional fees, this Authority did not invite payment of additional fees.

3. ☐ As only some of the required additional search fees were timely paid by the applicant, this international search report covers only those claims for which fees were paid, specifically claims Nos.:

4. ☐ No required additional search fees were timely paid by the applicant. Consequently, this international search report is restricted to the invention first mentioned in the claims; it is covered by claims Nos.:

**Remark on Protest**

☐ The additional search fees were accompanied by the applicant's protest and, where applicable, the payment of a protest fee.

☐ The additional search fees were accompanied by the applicant's protest but the applicable protest fee was not paid within the time limit specified in the invitation.

☐ No protest accompanied the payment of additional search fees.

Form PCT/ISA/210 (continuation of first sheet (2)) (July 2009)

**INTERNATIONAL SEARCH REPORT**

International application No.
PCT/JP2014/054597

Continuation of Box No.II-1 of continuation of first sheet(2)

Claim 11 pertains to [a method for treatment of the human body or animal body by surgery or therapy] and thus relates to a subject matter on which this International Searching Authority is not required to carry out an international search under the provisions of PCT Article 17(2)(a)(i) and [PCT Rule 39.1(iv)].

Form PCT/ISA/210 (extra sheet) (July 2009)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2012086848 A
- WO 2010125985 A